# EUROPÄISCHE PATENTSCHRIFT

(11) **EP 3 560 924 B1**
(45) Veröffentlichungstag und Bekanntmachung des Hinweises auf die Patenterteilung: **31.03.2021**
(21) Anmeldenummer: 19172903.7
(22) Anmeldetag: 31.03.2016
(51) Int. Cl.: C07D 471/04, A61P 35/00, A61K 31/4375, A61K 45/06

(54) **IMIDAZOLONYLCHINOLINE UND DEREN VERWENDUNG ALS ATM-KINASE-INHIBITOREN**
IMIDAZOLONYL QUINOLINES AND THEIR USE AS ATM KINASE INHIBITORS
IMIDAZOLONYL-QUINOLÉINES ET LEUR UTILISATION COMME INHIBITEURS DU ATM KINASE

(30) Priorität: 02.04.2015 EP 15000968
(43) Veröffentlichungstag der Anmeldung: 30.10.2019
(62) Teilanmeldung aus: 16717560.3
(73) Patentinhaber: Merck Patent GmbH, 64293 Darmstadt (DE)
(72) Erfinder: Fuchss, Thomas, 64625 BENSHEIM-AUERBACH (DE); Schiemann, Kai, 64342 SEEHEIM-JUGENHEIM (DE)

(56) Entgegenhaltungen:
- WO-A1-2010/139731
- WO-A1-2011/054846

## Beschreibung

### HINTERGRUND DER ERFINDUNG

Die vorliegende Erfindung betrifft spezifische Imidazolonylchinolin-Verbindungen und deren Verwendung bei der Hemmung, Regulierung und/oder Modulation der Signaltransduktion von Kinasen, insbesondere der ATM Kinase, ferner pharmazeutische Zusammensetzungen, die diese Verbindungen enthalten, sowie die Verwendung der Verbindungen zur Behandlung von Krankheiten, die einen Bezug zu ATM Kinase aufweisen, insbesondere Krebs.

Die Serin/Threonin Protein-Kinase ATM *(Ataxia* Telangiectasia Mutated Kinase) gehört zur PIKK- Familie von Kinasen mit katalytische Domänen, die Homologie zu den Phosphoinositid-3-Kinasen (PI3-Kinase, PI3K) aufweisen. Diese Kinasen sind an einer Vielzahl von zellulären Schlüsselfunktionen, wie Zellwachstum, Zellproliferation, Migration, Differenzierung, Überleben und Zelladhäsion beteiligt. Insbesondere reagieren diese Kinasen auf DNA-Schädigung durch Aktivierung des Zellzyklus-Arrests und DNA-Reparaturprogrammen (DDR: DNA Damage Response). ATM ist ein Produkt des ATM Gens und spielt eine Schlüsselrolle bei der Reparatur von Schäden am DNA-Doppelstrang (DSB. Double strand breaks) durch homologe Rekombination und nicht homologe End-zu-End-Verbindung (NHEJ: non-homologous end joining). Derartige Doppelstrangschäden sind besonders zytotoxisch.

Eines der durchgängigen Merkmale von Tumoren beim Menschen ist deren genomische Instabilität, wobei die spezifischen Defekte des DNA-Reparaturmechanismus bei den meisten Krebsarten bislang nicht bekannt sind. Diese Instabilität stellt den therapeutischen Ansatzpunkt für die seit langem vorherrschend praktizierte Chemotherapie dar. Es gibt darüber hinaus einige wenige Syndrome, bei denen der zugrundeliegende genetische Einflussfaktor auf eine mit Funktionsverlust einhergehende Mutation eines Gens zurück zu führen ist, welches die Reaktion auf Schäden am DNA-Doppelstrang moduliert. Dazu gehört *Ataxia* Telangiectasia, welches durch ein defektes ATM Gen hervorgerufen wird. Eine Gemeinsamkeit all dieser Syndrome ist, dass sie eine extreme Strahlungsempfindlichkeit verursachen (Lavin & Shiloh (1997) Annu. Rev. Immunol. 15: 177; Rotman & Shiloh (1998) Hum. Mol. Genet. 7: 1555, in ihrer Gesamtheit durch Bezugnahme hierin aufgenommen). ATM-defektive Zellen sind entsprechend empfindlich gegenüber Mitteln bzw. Maßnahmen, die Schäden am DNA-Doppelstrang hervorrufen, was ATM zu einem attraktiven Target für die Chemo- und Strahlungssensibilisierung bei der Krebsbehandlung macht.

Die zunächst vor diesem Hintergrund untersuchten Moleküle Koffein und Wortmannin zeigten zwar die unter anderem auf eine Inhibierung von ATM zurück geführte Radiosensibilisierung, sie sind jedoch *in vivo* zu toxisch für eine mögliche therapeutische Anwendung. Ausgehend von der chemischen Struktur des PI3K-Inhibitors LY294002 wurde von KuDOS Pharmaceuticals der erste potente und selektive ATM Inhibitor entwickelt: KU-55933 (2-Morpholino-6-(thianthren-1-yl)-4H-pyran-4-one). Mit ihm gelang eine Sensibilisierung gegenüber ionisierender Strahlung und DNA-Doppelstrangschädigenden Chemotherapeutika (Hickson, I., et al. (2004).Cancer Res 64, 9152-9159, in seiner Gesamtheit durch Bezugnahme hierin aufgenommen). Allerdings erwies sich KU-55933 mutmaßlich aufgrund hoher Lipophilie als ungeeignet für eine *in vivo* Anwendung. Basierend auf KU-55933 wurden unter leichter Modifizierung des Grundgerüsts KU-60019 (2-((2S,6R)-2,6-Dimethylmorpholino)-N-(5-(6-morpholino-4-oxo-4H-pyran-2-yl)-9H-thioxanthen-2-yl)acetamid) und KU-559403 (2-(4-Methylpiperazin-1-yl)-N-[5-(6-morpholino-4-oxo-pyran-2-yl)thioxanthen-2-yl]acetamid) entwickelt, wobei Löslichkeit und Potenz verbessert werden konnten. Zwischenzeitlich wurde beispielsweise berichtet, dass Glioblastoma-initiierende Zellen durch KU-60019 sicher und effektiv für Bestrahlung sensibilisiert werden konnten, woraus geschlossen wurde, dass KU-60019 für die Strahlungssensibilisierung einer ganzen Reihe von Hirntumoren fungieren kann (Vecchio D. et al. (2015), Int. J. Cancer 136: 1445, in seiner Gesamtheit durch Bezugnahme hierin aufgenommen).

WO 2011/054846 offenbart Bromodomain-Inhibitoren, die auf einem Imidazo-[4,5-c]chinolin-Grundgerüst beruhen und möglicherweise zur Behandlung von Krebs, entzündlichen Erkrankungen, oder Autoimmunerkrankungen verwendet werden können.

Die Bereitstellung von kleinen Molekülen, die die Signaltransduktion von Kinasen, und zwar insbesondere ATM Kinase, wirksam hemmen, regulieren und/oder modulieren, ist daher wünschenswert und Aufgabe der vorliegenden Erfindung.

Weiter ist es wünschenswert, dass derartige Kinase-Inhibitoren selektiv sind, das heißt keine bzw. eine deutlich geringere Aktivität gegenüber anderen Kinasen aufweisen. So können Off-Target-Effekte bzw. damit zusammenhängende Toxizitäten verringert werden.

### BESCHREIBUNG DER ERFINDUNG

Die Aufgabe wurde überraschend gelöst durch Verbindungen der Formel (I) 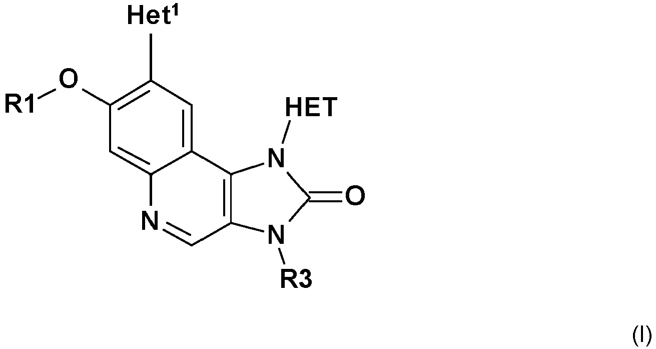 wobei
- R1: Methyl ist,
- R3: unverzweigtes oder verzweigtes Alkyl mit 1, 2, oder 3 C-Atomen bedeutet, wobei unabhängig voneinander 1, 2, 3, 4, oder 5 H-Atome durch Hal ersetzt sein können,
- Het¹: ausgewählt ist aus Pyrazolyl, Triazolyl und Imidazolyl, wobei Het¹ unsubstituiert oder mit einem oder zwei Substituenten substituiert ist, die unabhängig voneinander ausgewählt sind aus Alkyl, welches unsubstituiert oder ein- oder mehrfach durch Halogen substituiert sein kann, -OY, -NYY, Hal, und -Het²,
- HET: einen 5-gliedrigen einkernigen aromatischen Heterozyklus bedeutet, wobei dieser Heterozyklus über ein Ring-C-Atom mit dem N-Atom des Grundgerüsts verknüpft ist, wobei dieser Heterozyklus ausgewählt ist aus Pyrazolyl, Thiazolyl, und Imidazolyl, und unsubstituiert oder durch einen, zwei oder drei Substituenten substituiert sein kann, die unabhängig voneinander ausgewählt sind aus der Gruppe bestehend aus: Hal, A, Het², -(CY₂)ₚ-OY, -(CY₂)ₚ-OZ, -(CY₂)ₚ-O-Het²,-(CY₂)ₚ-O-(CY₂)t-Het², -(CY₂)ₚ-O-(CY₂)ₜ-NYY, -(CY₂)ₚ-O-(CY₂)ₜ-OY, -(CY₂)ₚ-NYY,-(CY₂)ₚ-COOY, -(CY₂)ₚ-CO-NYY, und -(CY₂)ₚ-NY-COY,
- A: jeweils unabhängig unverzweigtes oder verzweigtes Alkyl mit 1, 2, 3, 4, 5, 6, 7, 8, 9 oder 10 C-Atomen bedeutet, wobei unabhängig voneinander 1, 2, 3, 4, 5, 6 oder 7 H-Atome durch Hal ersetzt sein können,
- Het²: einen einkernigen gesättigten Heterozyklus mit 2, 3, 4, 5, 6 oder 7 C-Atomen und 1, 2, 3 oder 4 N-, O- und/oder S-Atomen bedeutet, der unsubstituiert oder einfach durch A substituiert sein kann,
- Y: H oder A bedeutet,
- Z: unverzweigtes oder verzweigtes Alkenyl mit 2, 3, 4, 5, 6, 7, 8, 9 oder 10 C-Atomen bedeutet, wobei unabhängig voneinander 1, 2, 3, 4, 5, 6 oder 7 H-Atome durch Hal ersetzt sein können,
- Hal: F, Cl, Br oder I bedeutet, und
- p: 0, 1, 2, 3, 4, 5 oder 6
- t: 1, 2, 3, 4, 5 oder 6 bedeuten,
und/oder pharmazeutisch verwendbares Salz, Solvat, Tautomer, Stereoisomer davon.

Wie nachfolgend detaillierter ausgeführt wird, sind die Verbindungen dahingehend zu verstehen, dass die Atome, wie etwa H, C, N, jeweils auch die schwereren Isotope dieser Atome umfassen. Das gilt insbesondere für H, wobei Deuterium vorteilhaft eingesetzt werden kann, wie durch die Beispiele aufgezeigt wird.

Erfindungsgemäße Verbindungen haben sich überraschend als potente Inhibitoren von ATM Kinase erwiesen. Noch überraschender ist die Selektivität gegenüber anderen verwandten Kinasen, wie etwa mTOR (mammalian target of rapamycin) Kinase, einer weiteren Protein-Kinase aus der Familie der PIK-Kinasen (auch als Klasse IV PI3K bezeichnet).

Ganz im Gegensatz zur vorliegenden Erfindung wird in der internationalen Patentanmeldung WO 2010/139731 für die dort beschriebenen und beanspruchten Imidazolonylchinolin-Verbindungen offenbart, dass es sich bevorzugt um Inhibitoren von Klasse I PI3 Kinasen und/oder mTOR Kinase handelt. Klasse I PI3 Kinasen sind Lipidkinasen. Entsprechend zeigen experimentellen Daten IC₅₀-Werte für mTOR im Bereich bis hinunter zu weniger als 3 nM, also sehr starke Inhibition.

Die erfindungsgemäßen Verbindungen belegen wieder einmal eindrucksvoll, wie auf den ersten Blick relativ gering erscheinende strukturelle Unterschiede einen entscheidenden Einfluss auf die biologische Aktivität ausüben.

Darüber hinaus zeichnen sich die erfindungsgemäßen Verbindungen auch durch das Fehlen der häufig beobachteten, unerwünschten Hemmung kardialer lonenkanäle, insbesondere des Kv1.11 hERG aus, dessen Blockade zu lebensbedrohlichen Arrhytmien führen kann.

Die erfindungsgemäßen Verbindungen eröffnen damit völlig neue Möglichkeiten bei der Krebstherapie, beispielsweise als Monotherapie bei Tumoren mit defektiver DNA-Doppelstrang-Reparaturfähigkeit oder in Kombination mit Radio- oder Chemotherapie, insbesondere als Radio- und Chemosensibilisierer bei der Behandlung von Krebs, besonders bevorzugt als Radiosensibilisierer.

Die Verbindungen der Formel (I) können mithin zur Hemmung von sowie zur Sensibilisierung von Krebszellen gegenüber Antikrebsmitteln und/oder ionisierender Strahlung verwendet werden. Gegenstand der Erfindung ist auch die Verwendung der Verbindungen der Formel (I) bei der Behandlung von Krebs, Tumoren, und/oder Metastasen, in Kombination mit Radiotherapie und/oder einem Antikrebsmittel, bevorzugt Radiotherapie.

Vor- und nachstehend haben die Radikale R1, R3, Y, A, Z, Het¹, Het², HET und Hal sowie p und t die bei der Formel (I) oben angegebenen Bedeutungen, sofern nicht ausdrücklich etwas anderes angegeben ist. Bei mehrfachem Auftreten einzelner Reste innerhalb einer Verbindung oder eines Radikals nehmen die Reste unabhängig voneinander die angegebenen Bedeutungen an, sofern nicht ausdrücklich etwas anderes angegeben ist. Beispielsweise sind die Reste YY im Radikal -NYY, in dem sie mehrfach vorkommen, identisch oder verschieden, aber vorzugsweise in jedem Fall unabhängig voneinander unter den vorstehend und/oder nachstehend angegebenen Bedeutungen ausgewählt (z.B. Methyl und/oder Ethyl), sofern nicht ausdrücklich etwas anderes angegeben ist. Bei mehrfachem Auftreten von Y kann das Radikal alternativ auch mit Y', Y", Y"' und Y"" bezeichnet werden. Entsprechendes gilt für die anderen Reste bzw. Zahlenvariablen. Entsprechend bedeutet die Formulierung "unsubstituiertes oder ein- oder mehrfach unabhängig voneinander durch Hal, A, ... und/oder -(CY₂)ₚ-NY-COY substituiertes Cycloalkyl", dass der Cycloalkylrest einfach durch einen der genannten Substituenten substituiert sein kann, oder aber mehrere Substituenten aufweisen kann, die unabhängig voneinander ausgewählt sind aus den genannten Substituenten, im Falle des Cycloalkyls Hal, A, CN, -(CY₂)ₚ-OY, -(CY₂)ₚ-NYY, -(CY₂)ₚ-COOY, -(CY₂)ₚ-CO-NYY und/oder -(CY₂)ₚ-NY-COY, also zum Beispiel ein Cyclohexyl mit den drei Substituenten Fluor, Chlor und - CH₂-NH₂.

Den vorliegend verwendeten Bezeichnungen zur Definition der Verbindungen liegen im Allgemeinen die Regeln der IUPAC-Organisation für chemische Verbindungen und insbesondere organische Verbindungen zugrunde. Die Bezeichnungen zur Erläuterung der o.g. Verbindungen der Erfindung haben immer die nachstehenden Bedeutungen, sofern die Beschreibung oder die Ansprüche nicht etwas anderes anzeigen.

Der Begriff "unsubstituiert" bedeutet, dass ein Radikal, eine Gruppe oder ein Rest keine Substituenten trägt. Der Begriff "substituiert" bedeutet, dass ein Radikal, eine Gruppe oder ein Rest einen oder mehrere Substituenten trägt. Wird im Folgenden bezüglich einer bestimmten Gruppe oder eines bestimmten Radikals, dessen Substitution in Zusammenhang mit obiger Formel (I) spezifisch definiert ist, lediglich "substituiert" erwähnt, so versteht sich, dass diese Substitution der oben genannten entspricht, sofern nicht spezifisch etwas anderes angegeben ist. Es versteht sich hierbei im Sinne der Erfindung auch, dass ein Radikal durch Verweis auf "die oben angegebene Bedeutung" ohne nähere Spezifizierung derselben sämtliche im bisherigen Verlauf der Beschreibung genannte Bedeutungen für das entsprechende Radikal annehmen kann.

"A" steht für ein unverzweigtes oder verzweigtes Alkyl mit 1, 2, 3, 4, 5, 6, 7, 8, 9 oder 10 C-Atomen, wobei unabhängig voneinander 1, 2, 3, 4, 5, 6 oder 7 H-Atome durch Hal ersetzt sein können. Besonders bevorzugt als "A" ist unverzweigtes oder verzweigtes Alkyl mit 1, 2, 3, 4, 5 oder 6 C-Atomen, wobei unabhängig voneinander 1, 2, 3, 4 oder 5 H-Atome durch Hal, also F, Cl, Br und/oder I, ersetzt sein können. Es versteht sich, dass Hal in diesem Zusammenhang für verschiedene Halogene F, Cl, Br, I stehen kann, also beispielsweise 1 H durch F ersetzt sein kann, ein anderes H durch Cl. Ganz besonders bevorzugt ist C₁₋₄-Alkyl, wobei unabhängig voneinander 1, 2 oder 3 H-Atome durch Hal ersetzt sein können. Ein solches C₁₋₄-Alkyl ist beispielsweise Methyl, Ethyl, Propyl, Isopropyl, Butyl, Isobutyl, sek.-Butyl, tert.-Butyl, Fluormethyl, Difluormethyl, Trifluormethyl, Pentafluorethyl, 1,1,1-Trifluorethyl oder Brommethyl, höchst bevorzugt Methyl, Ethyl, Fluormethyl, Difluormethyl oder Trifluormethyl. Es versteht sich, dass die jeweiligen Bedeutungen von "A" unabhängig voneinander in den Radikalen einer erfindungsgemäßen Formel sind.

"Alkyl" bezeichnet hierin ein gesättigtes Kohlenwasserstoffradikal, das unverzweigt (linear) oder verzweigt ist und vorzugsweise 1, 2, 3, 4, 5, 6, 7, 8, 9 oder 10 C-Atome besitzt, d.h. C₁₋₁₀-Alkyl. Beispiele für Alkylradikale sind Methyl, Ethyl, Propyl, Isopropyl, 1,1-, 1,2- oder 2,2-Dimethylpropyl, 1-Ethylpropyl, 1-Ethyl-1-methylpropyl, 1-Ethyl-2-methylpropyl, 1,1,2- oder 1,2,2-Trimethylpropyl, Butyl, Isobutyl, sek.-Butyl, tert.-Butyl, 1-, 2- oder 3-Methylbutyl, 1,1-, 1,2-, 1,3-, 2,2-, 2,3- oder 3,3-Dimethylbutyl, 1- oder 2-Ethylbutyl, Pentyl, Isopentyl, Neopentyl, tert.-Pentyl, 1-, 2-, 3- oder 4-Methylpentyl, Hexyl.

"Z" bezeichnet hierin unverzweigtes oder verzweigtes Alkenyl mit 2, 3, 4, 5, 6, 7, 8, 9 oder 10 C-Atomen, wobei unabhängig voneinander 1, 2, 3, 4, 5, 6 oder 7 H-Atome durch Hal ersetzt sein können. Alkenyle haben mindestens eine C-C Doppelbindung, können insbesondere auch zwei Doppelbindungen aufweisen (Diene). Beispiele für geeignete Alkenyle sind Vinyl, Allyl, Propenyl (-CH₂CH=CH₂; -CH=CH-CH₃; -C(=CH₂)-CH₃), 1-, 2- oder 3-Butenyl, Isobutenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl and Hexenyl, besonders bevorzugt Allyl.

Hierin ist "Het²" ein einkerniger gesättigter Heterozyklus mit 2, 3, 4, 5, 6 oder 7 C-Atomen und 1, 2, 3 oder 4 N-, O- und/oder S-Atomen, der unsubstituiert oder einfach durch A substituiert sein kann. Es ist bevorzugt, dass "Het²" einen einkernigen gesättigten Heterozyklus mit 3, 4 oder 5 C-Atomen und 1 oder 2 N- und/oder O-Atomen bedeutet. Beispiele für Het² sind Aziridinyl, Oxiranyl, Thiaranyl, Azetidinyl, Oxetanyl, Dioxetanyl, Thietanyl, Pyrrolidinyl, Tetrahydrofuranyl, Dithiolanyl, Oxazolidinyl, Piperidinyl, Tetrahydropyranyl, Piperazinyl, Dioxanyl, Thiomorpholinyl, Morpholinyl, Oxepanyl, Thiepanyl, und Azepanyl. N-haltige einkernige gesättigte Heterozyklen sind bevorzugt, z.B. Azetidinyl, Pyrrolidinyl, Piperidinyl, Piperazinyl und Morpholinyl, wobei diese entsprechend der allgemeinen Definition unsubstituiert oder einfach durch A substituiert sein können. Unsubstituiertes Azetidinyl, Pyrrolidinyl, Piperidinyl, Piperazinyl und Morpholinyl zählen zu bevorzugten Ausführungsformen, besonders bevorzugt Azetidinyl und Piperazinyl. Weiterhin ist auch Tetrahydropyranyl bevorzugt. Die Verknüpfung "-"Het² in der Gruppierung -SO₂-Het² erfolgt typischerweise über ein C-Atom. Andernfalls, also beispielsweise als Substituent von HET kann die Verknüpfung über jedes Ringmitglied der Heterozyklusgruppe erfolgen, sofern es chemisch sinnvoll erscheint, wobei auch hier eine Bindung über ein C-Atom bevorzugt ist.

"HET" steht im Rahmen der vorliegenden Erfindung für einen 5-gliedrigen aromatischen Heterozyklus, wobei dieser Heterozyklus über ein Ring-C-Atom mit dem N-Atom des Grundgerüsts verknüpft ist, wobei dieser Heterozyklus ausgewählt ist aus Pyrazolyl, Thiazolyl und Imidazolyl, und wobei dieser Heterozyklus unsubstituiert oder durch einen, zwei oder drei Substituenten substituiert sein kann, die unabhängig voneinander ausgewählt sind aus der Gruppe bestehend aus: Hal, A, Het², -(CY₂)ₚ-OY, -(CY₂)ₚ-OZ, - (CY₂)ₚ-O-Het², -(CY₂)ₚ-O-(CY₂)ₜ-Het², -(CY₂)ₚ-O-(CY₂)ₜ₋NYY, -(CY₂)ₚ-O-(CY₂)ₜ-OY, -(CY₂)ₚ-O-(CY₂)ₜ₋POAA, -(CY₂)ₚ-NYY, -(CY₂)ₚ-COOY, -(CY₂)ₚ-CO-NYY, -(CY₂)ₚ-NY-COY.

Der Begriff "Hal", oder auch "Halogen", "Halogenatom", "Halogensubstituent", bezeichnet hierin ein oder mehrere Atome von Fluor (F), Brom (Br), Chlor (Cl) oder Iod (I). Die Bezeichnungen "Dihalogen", "Trihalogen" und "Perhalogen" beziehen sich auf zwei, drei oder vier Substituenten, wobei jeder Substituent unabhängig voneinander aus der Gruppe von F, Cl, Br oder I ausgewählt werden kann. "Halogen" meint vorzugsweise F, Cl oder Br. F und Cl sind besonders bevorzugt, insbesondere wenn die Halogene Substituenten an einer Alkyl- (Haloalkyl) oder Alkoxygruppe sind (z.B. CF₃ und CF₃O).
-COOY, -CO-NYY, -COOY und -NY-COY sind übliche Schreibweisen für die folgenden Gruppen:

| | |
|---|---|
| -CO-NYY | 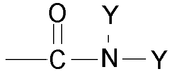 |
| -COOY | 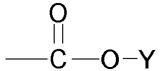 |
| -NY-COY | 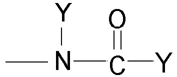 |

Der Index p bedeutet vorzugsweise 0, 1, 2 oder 3, besonders bevorzugt 0.
Der Index t bedeutet vorzugsweise 1, 2, 3 oder 4, besonders bevorzugt 2.

Beispiele für -(CY₂)ₚ-OY, -(CY₂)ₚ-OZ, -(CY₂)ₚ-O-Het², -(CY₂)ₚ-O-(CY₂)ₜ-Het², -(CY₂)ₚ-O-(CY₂)ₜ-NYY, -(CY₂)ₚ-O-(CY₂)ₜ-OY, , -(CY₂)ₚ-NYY, -(CY₂)ₚ-COOY, -(CY₂)ₚ-CO-NYY, -(CY₂)ₚ-NY-COY, sind somit: -OY, -OH, -O-A, -O-CH₃, -O-CHF₂, -O-CH₂F, -O-CH₂CH₃, -(CH₂)ₚ-OY, -CH₂-O-CH₃ -CH₂-O-CH₂CH₃, -O-CH=CH₂, -CH₂-O-CH=CH₂, -O-CH₂-CH=CH₂, -CH₂-O-CH₂-CH=CH₂, -O-Het², -O-CH₂-Het², -O-CH₂CH₂-Het², -O-CH₂-NYY, -O-CH₂CH₂-NYY, -O-CH₂-NH₂, -O-CH₂CH₂-NH₂, -O-CH₂-NHCH₃, -O-CH₂CH₂-NHCH₃, -O-CH₂-OY, -O-CH₂CH₂-OY, -O-CH₂CH₂CH₂-OY, -O-CH₂-OH, -O-CH₂CH₂-OH, -O-CH₂CH₂CH₂-OH, -O-CH₂-OA, -O-CH₂CH₂-OA, -O-CH₂CH₂CH₂-OA, -O-CH₂-OCH₃, -O-CH₂CH₂-OCH₃, -O-CH₂CH₂CH₂-OCH₃, -NYY, -NH₂, -NHA, -NAA, -NHCH₃, -N(CH₃)₂, -CH₂-NYY, -CH₂-NH₂, -CH₂-NHA, -CH₂-NAA, -CH₂-NHCH₃, -CH₂-N(CH₃)₂, -COOY, -COOA, -CH₂-COOY, -CH₂-COOA, -CH₂-COOCH₃, -CO-NYY, -CO-NAA, -CO-NHA, -CO-NH-CH₃, -CH₂-CO-NYY, -CH₂-CO-NAA, -(CH₂)ₚ-NY-COY, -(CH₂)ₚ-NY-COA.

Dementsprechend sind Gegenstand der Erfindung diejenigen Verbindungen der Formel (I), in denen mindestens eines der genannten Radikale eine der vorstehend angegebenen Bedeutungen hat. Nicht näher bezeichneten Radikale im Kontext einer der Formel (I), Teilformel davon oder irgendeines Restes daran sollen die bei der Formel (I) angegebene Bedeutung haben, wie sie hierin offenbart ist, um die Aufgabe der Erfindung zu lösen. Das heißt, dass die genannten Radikale sämtliche ihnen zugeordneten Bedeutungen annehmen können, wie sie vor- oder nachstehend beschrieben sind, einschließlich jeglicher bevorzugter Ausführungsformen, ohne auf diese beschränkt zu sein und unabhängig von ihrem Auftreten in einem anderen bestimmten Kontext. Es versteht sich insbesondere, dass jede Ausführungsform eines bestimmten Radikals mit jeder Ausführungsform eines oder mehrerer anderer Radikale kombiniert werden kann.

Erfindungsgemäß steht R1 in Formel (I) für Methyl. Entsprechend stellt die vorliegende Erfindung Verbindungen der folgenden Formeln (II) bereit: 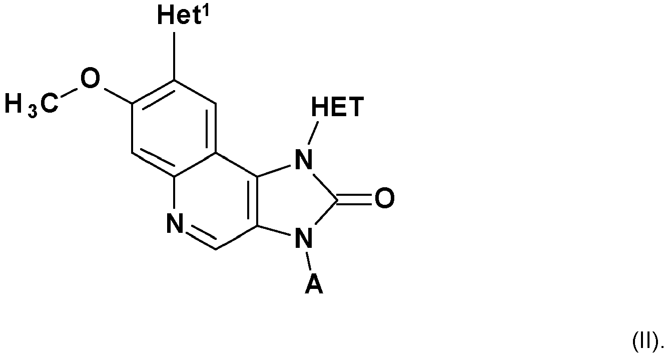 wobei A an der Position für R3 die oben für R3 in Formel (I) genannte Bedeutung hat.

Erfindungsgemäß bedeutet R3 in den Formeln (I) bzw. (II) unverzweigtes oder verzweigtes Alkyl mit 1, 2, oder 3 C-Atomen, wobei unabhängig voneinander 1, 2, 3, 4, oder 5 H-Atome durch Hal ersetzt sein können. Besonders bevorzugt bedeutet R3 Methyl. Damit stellt die vorliegende Erfindung auch Verbindungen der folgenden Formel (IV) bereit:

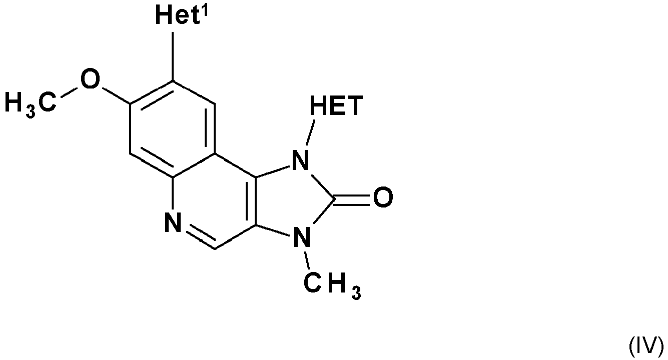

Erfindungsgemäß ist in jeder der Formeln (I), (II) und (IV) Het¹ ausgewählt aus Pyrazolyl, Triazolyl und Imidazolyl, welche unsubstituiert oder wie oben für Formel (I) definiert substituiert sein können, wobei Pyrazolyl und Triazolyl, insbesondere [1,2,3]Triazolyl, besonders bevorzugt sind.

Het¹ kann beispielsweise unsubstituiert oder mit einem oder zwei Substituenten substituiert sein, die bevorzugt unabhängig voneinander ausgewählt sind aus: Alkyl, welches unsubstituiert oder ein- oder mehrfach durch Halogen, insbesondere F substituiert sein kann; -OY, -NYY, Hal, und -Het², besonders bevorzugt Methyl, Ethyl, Amino, Methoxy, Fluormethyl, Difluormethyl, Fluor, Azetidinyl, wobei Methyl und Ethyl besonders bevorzugt sind.

Besonders bevorzugt ist Het¹ (in jeder der aufgeführten Formeln) ausgewählt aus: 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1-Difluormethyl-1H-pyrazol-4-yl, 1,3-Dimethyl-1H-pyrazol-4-yl, 1-Ethyl-1H-pyrazol-4-yl, 1-Ethyl-3-methyl-1H-pyrazolyl, 3-Fluor-1-methyl-1H-pyrazol-4-yl, 3-Amino-1H-pyrazol-5-yl, 2H-[1,2,3]Triazol-4-yl, 3H-[1,2,3]Triazol-4-yl-, 1-Methyl-1H-[1,2,3]triazol-4-yl, 2-Methyl-2H-[1,2,3]triazol-4-yl, 2-Amino-1H-imidazol-4-yl, 6-Methoxy-pyridin-3-yl, und 1-(Azetidin-3-yl)-3-methyl-1H-pyrazol-4-yl. Stark bevorzugt ist Het¹ Pyrazolyl, welches unsubstituiert oder wie oben definiert substituiert sein kann, und beispielsweise ausgewählt sein kann aus 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1-Diluormethyl-1H-pyrazol-4-yl, 1,3-Dimethyl-1H-pyrazol-4-yl, 1-Ethyl-1H-pyrazol-4-yl, 1-Ethyl-3-methyl-1H-pyrazolyl, 3-Fluor-1-methyl-1H-pyrazol-4-yl, 3-Amino-1H-pyrazol-5-yl, wobei 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1,3-Dimethyl-1H-pyrazol-4-yl, 1-Ethyl-1H-pyrazol-4-yl und besonders bevorzugt sind. Alternativ ist Het¹ bevorzugt Triazolyl, welches unsubstituiert oder wie oben definiert substituiert sein kann, und beispielsweise ausgewählt sein kann aus 2H-[1,2,3]Triazol-4-yl, 3H-[1,2,3]Triazol-4-yl-, 1-Methyl-1H-[1,2,3]triazol-4-yl, 2-Methyl-2H-[1,2,3]triazol-4-yl, besonders bevorzugt 2-Methyl-2H-[1,2,3]triazol-4-yl.

In erfindungsgemäßen Verbindungen gemäß einer jeden der der Formeln (I), (II) und (IV) ist HET aus den folgenden 5-gliedrigen einkernigen aromatischen Heterozyklen ausgewählt, welche jeweils unsubstituiert oder wie zuvor definiert substituiert sein können: Pyrazolyl, Thiazolyl, Imidazolyl.

Diese Bedeutungen für HET in Verbindungen der Formeln (I), (II), (IV), gelten insbesondere auch in Kombination mit den bevorzugten Ausführungsformen für Het¹, beispielsweise 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1-Diluormethyl-1H-pyrazol-4-yl, 1,3-Dimethyl-1H-pyrazol-4-yl, 1-Ethyl-1H-pyrazol-4-yl, 1-Ethyl-3-methyl-1H-pyrazolyl, 3-Fluor-1-methyl-1H-pyrazol-4-yl, 3-Amino-1H-pyrazol-5-yl, 2H-[1,2,3]Triazol4-yl, 3H-[1,2,3]Triazol-4-yl-, 1-Methyl-1H-[1,2,3]triazol-4-yl, 2-Methyl-2H-[1,2,3]triazol-4-yl, 2-Amino-1H-imidazol-4-yl, 6-Methoxy-pyridin-3-yl, und 1-(Azetidin-3-yl)-3-methyl-1H-pyrazol-4-yl, und gerade und insbesondere auch für die Ausführungsformen, in denen Het¹ für Pyrazolyl steht, welches unsubstituiert oder wie oben definiert substituiert sein kann und beispielsweise ausgewählt sein kann aus 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1,3-Dimethyl-1H-pyrazol-4-yl, 1-Ethyl-1H-pyrazol-4-yl.

Von den möglichen Substituenten an HET sind bevorzugt: Hal, insbesondere F, Cl; A, insbesondere Methyl, Ethyl, Propyl; Het², insbesondere Azetidinyl, Tetrahydropyranyl, Piperazinyl; -(CY₂)ₚ-OY, -(CY₂)ₚ-OA, -(CY₂)ₚ-OH, bevorzugt jeweils mit p = 0, 1, 2, 3; -(CY₂)ₚ-OZ, -(CY₂)ₚ-O-Het², -O-Het², -(CY₂)ₚ-O-(CY₂)ₜ-Het², -O-(CY₂)ₜ-Het², -O-(CH₂)ₜ-Het², -(CY₂)ₚ-O-(CY₂)ₜ-NYY, -O-(CY₂)ₜ-NYY, -(CY₂)ₚ-O-(CY₂)ₜ-OY, -O-(CY₂)ₜ-OY, -O-(CY₂)ₜ-OA, -O-(CY₂)ₜ-OH, -O-(CH₂)ₜ-OA, -O-(CH₂)ₜ-OH, -(CY₂)ₚ-CO-NYY, -(CY₂)ₚ-CO-NHA, Beispielsweise können die aromatischen Heterozyklen HET jeweils substituiert sein durch einen, zwei, drei oder mehr Substituenten, die unabhängig voneinander ausgewählt sind aus der Gruppe bestehend aus: F, Cl, Methyl, Ethyl, Propyl, Isopropyl, Methoxy, Ethoxy, Propoxy, Piperazinyl, Tetrahydropyranyl, -CN, 2-Methoxyethoxy, 2-Hydroxyethoxy, Fluormethoxy, Difluormethoxy, N-Methylcarbamoyl (-C(=O)-NH-CH₃), 2-Methylaminoethoxy, 1-Methyl-azetidin-3-ylmethoxy, Trideuteriomethoxy, Trifluormethoxy, Allyloxy, Piperazinyl, und Azetidinyloxy. Keine, einfache oder zweifache Substitution sind bevorzugt.

In anderen bevorzugten Ausführungsformen ist HET ein unsubstituiertes, bevorzugt jedoch mono- oder disubstituiertes Pyrazol. Auch diese Ausführungsform kann vorteilhaft kombiniert werden mit den bevorzugten Ausführungsformen von Het¹, insbesondere den bevorzugten Ausführungsformen, in denen Het¹ unsubstituiertes oder substituiertes Pyrazolyl oder aber unsubstituiertes oder substituiertes Triazolyl ist, und insbesondere mit den oben genannten besonders bevorzugten Ausführungsformen.

HET kann in bevorzugten Ausführungsfomen gemäß einer jeden der Formeln (I), (II), (IV) beispielsweise ausgewählt sein aus den folgenden 5-gliedrigen einkernigen aromatischen Heterozyklen: 1H-Pyrazol-4-yl, 1-Ethyl-3-methyl-1H-pyrazol-4-yl, 1,2-Dimethyl-1H-pyrazol-4-yl, 1,3-Dimethyl-1H-pyrazol-4-yl, 1-Methyl-1H-pyrazol-4-yl, 1-(Tetrahydro-pyran-4-yl)-1H-pyrazol-4-yl, 2-Methyl-2H-pyrazol-3-yl, 3-Methyl-1H-pyrazol-4-yl, 3,5-Dimethyl-1H-pyrazol-4-yl, 3-Fluor-1-methyl-pyrazol-4-yl, Thiazol-2-yl, 2-Methyl-thiazol-4-yl,und 1-Methyl-1H-imidazolyl. Besonders bevorzugt sind:, 1,3-Dimethyl-1H-pyrazol-4-yl, 1-Methyl-1H-pyrazol-4-yl, 1-(Tetrahydro-pyran-4-yl)-1H-pyrazol-4-yl, und 2-Methyl-thiazol-4-yl.

Die Präferenz für die oben genannten 5-gliedrigen einkernigen aromatischen Heterozyklen gilt insbesondere auch für Verbindungen der Formeln (I), (II), (IV) in Kombination mit den bevorzugten Ausführungsformen für Het¹, insbesondere auch für die Ausführungsformen, in denen Het¹ für Pyrazolyl steht, welches unsubstituiert oder wie oben definiert substituiert sein kann und beispielsweise ausgewählt sein kann aus den oben explizit genannten Beispielen. Sie gelten insbesondere auch für die Ausführungsformen, in denen Het¹ für Triazolyl steht, und beispielsweise ausgewählt sein kann aus den oben explizit genannten Beispielen.

Ganz besonders bevorzugt sind die in der nachfolgenden Tabelle 1 aufgeführten Verbindungen, sowie deren pharmazeutisch verwendbaren Salze, Solvate, Tautomere und/oder Stereoisomere, bzw. freien Formen einschließlich deren Mischungen in allen Verhältnissen.

**Tab. 1: Besonders bevorzugte Verbindungen**

| | |
|---|---|
| 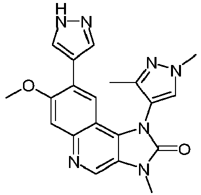 | 1-(1,3-Dimethyl-1 H-pyrazol-4-yl)-7-methoxy-3-methyl-8-(1H-pyrazol-4-yl)-1,3-dihydro-imidazo[4,5-c]chinolin-2-on |
| 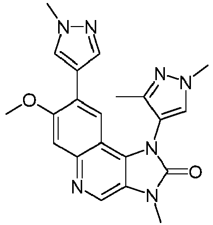 | 1-(1,3-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on |
| 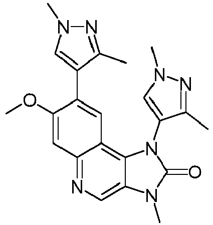 | 1,8-Bis-(1,3-dimethyl-1 H-pyrazol-4-yl)-7-methoxy-3-methyl-1,3-dihydro-imidazo[4,5-c]chinolin-2-on |
| 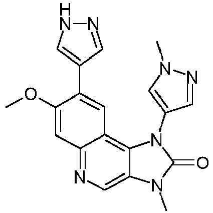 | 7-Methoxy-3-methyl-1-(1-methyl-1 H-pyrazol-4-yl)-8-(1H-pyrazol-4-yl)-1,3-dihydro-imidazo[4,5-c]chinolin-2-on |
| 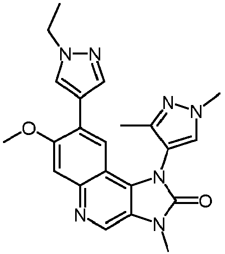 | 1-(1,3-Dimethyl-1 H-pyrazol-4-yl)-8-(1-ethyl-1H-pyrazol-4-yl)-7-methoxy-3-methyl-1,3-dihydro-imidazo[4,5-c]chinolin-2-on |
| 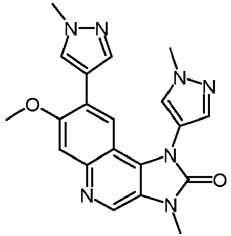 | 7-Methoxy-3-methyl-1,8-bis-(1-methyl-1H-pyrazol-4-yl)-1,3-dihydro-imidazo[4,5-c]chinolin-2-on |
| 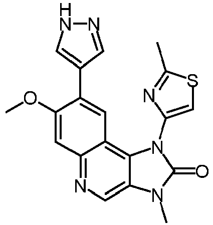 | 7-Methoxy-3-methyl-1-(2-methyl-thiazol-4-yl)-8-(1 H-pyrazol-4-yl)-1,3-dihydro-imidazo[4,5-c]chinolin-2-on |
| 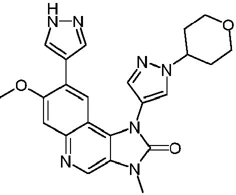 | 7-Methoxy-3-methyl-8-(1 H-pyrazol-4-yl)-1-[1-(tetrahydropyran-4-yl)-1 H-pyrazol-4-yl]-1 ,3-dihydro-imidazo[4,5-c]chinolin-2-on |
| 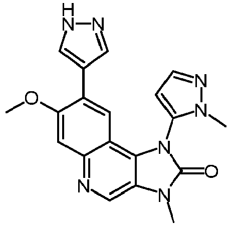 | 7-Methoxy-3-methyl-1-(2-methyl-2H-pyrazol-3-yl)-8-(1 H-pyrazol-4-yl)-1,3-dihydro-imidazo[4,5-c]chinolin-2-on |
| 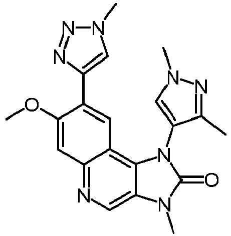 | 1-(1,3-Dimethyl-1 H-pyrazol-4-yl)-7-methoxy-3-methyl-8-(1-methyl-1 H-[1,2,3]triazol-4-yl)-1,3-dihydro-imidazo[4,5-c]chinolin-2-on |
| 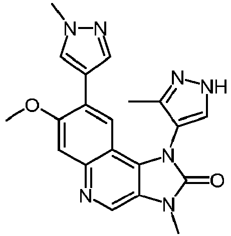 | 7-Methoxy-3-methyl-1-(3-methyl-1 H-pyrazol-4-yl)-8-(1-methyl-1 H-pyrazol-4-yl)-1,3-dihydro-imidazo[4,5-c]chinolin-2-on |
| 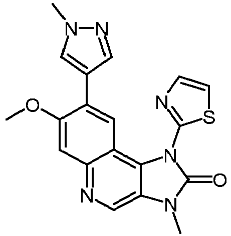 | 7-Methoxy-3-methyl-8-(1-methyl-1 H-pyrazol-4-yl)-1-thiazol-2-yl-1,3-dihydro-imidazo[4,5-c]chinolin-2-on |
| 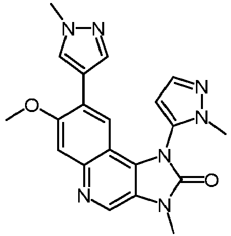 | 7-Methoxy-3-methyl-1-(2-methyl-2H-pyrazol-3-yl)-8-(1-methyl-1 H-pyrazol-4-yl)-1,3-dihydro-imidazo[4,5-c]chinolin-2-on |
| 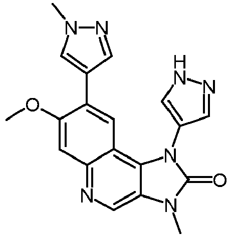 | 7-Methoxy-3-methyl-8-(1-methyl-1 H-pyrazol-4-yl)-1-(1H-pyrazol-4-yl)-1,3-dihydro-imidazo[4,5-c]chinolin-2-on |
| 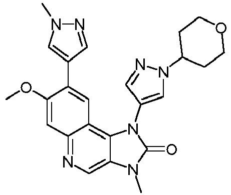 | 7-Methoxy-3-methyl-8-(1-methyl-1 H-pyrazol-4-yl)-1-[1-(tetrahydro-pyran-4-yl)-1 H-pyrazol-4-yl]-1 ,3-dihydro-imidazo[4,5-c]chinolin-2-on |
| 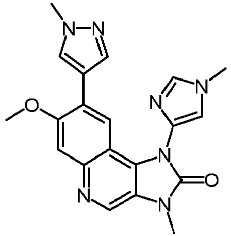 | 7-Methoxy-3-methyl-1-(1-methyl-1 H-imidazol-4-yl)-8-(1-methyl-1 H-pyrazol-4-yl)-1,3-dihydro-imidazo[4,5-c]chinolin-2-on |
| 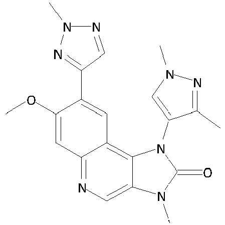 | 1-(1,3-Dimethyl-1 H-pyrazol-4-yl)-7-methoxy-3-methyl-8-(2-methyl-2H-[1,2,3]triazol-4-yl)-1,3-dihydro-imidazo[4,5-c]chinolin-2-on |
| 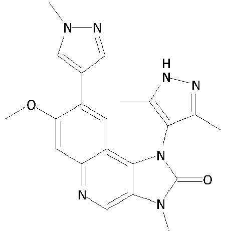 | 1-(3,5-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on |
| 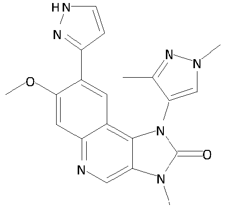 | 1-(1,3-Dimethyl-1 H-pyrazol-4-yl)-7-methoxy-3-methyl-8-(1H-pyrazol-3-yl)-1,3-dihydro-imidazo[4,5-c]chinolin-2-on |
| 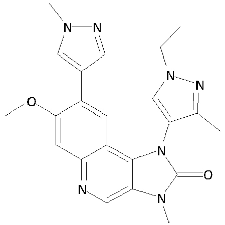 | 1-(1-Ethyl-3-methyl-1H-pyrazol-4-yl)-7-methoxy-3-methyl-8-(1-methyl-1H-pyrazol-4-yl)-1,3-dihydro-imidazo[4,5-c]chinolin-2-on |
| 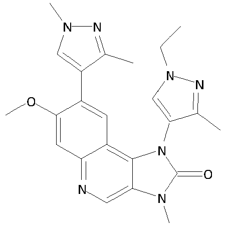 | 8-(1,3-Dimethyl-1H-pyrazol-4-yl)-1-(1-ethyl-3-methyl-1H-pyrazol-4-yl)-7-methoxy-3-methyl-1,3-dihydro-imidazo[4,5-c]chinolin-2-on |
| 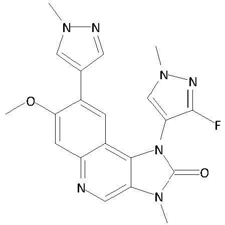 | 1-(3-Fluor-1-methyl-1 H-pyrazol-4-yl)-7-methoxy-3-methyl-8-(1-methyl-1 H-pyrazol-4-yl)-1,3-dihydro-imidazo[4,5-c]chinolin-2-on |
| 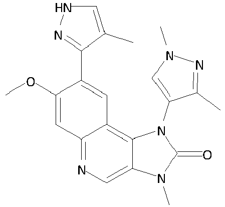 | 1-(1,3-Dimethyl-1 H-pyrazol-4-yl)-7-methoxy-3-methyl-8-(4-methyl-1H-pyrazol-3-yl)-1,3-dihydro-imidazo[4,5-c] chinolin-2-on |
| 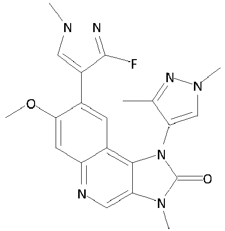 | 1-(1,3-Dimethyl-1 H-pyrazol-4-yl)-7-methoxy-3-methyl-8-(3-fluor-1-methyl-1 H-pyrazol-4-yl)-1,3-dihydro-imidazo[4,5-c]chinolin-2-on |

Jede der im Rahmen der obigen bevorzugten Verbindungen offenbarten Bedeutungen für R1, R3, HET, und Het1 ist als bevorzugte Bedeutung des entsprechenden Radikals auch unabhängig von der sonstigen Substitution der Molekülgrundstruktur anzusehen, also als mögliche Bedeutungen von R1, R3, HET, und Het1 jeweils unabhängig voneinander.

### Herstellung

Die Verbindungen der Formel (I) und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen. Die erfindungsgemäßen Verbindungen können durch geeignete Wahl bzw. Anpassung der Ausgangsmaterialien mittels bzw. analog der in den BEISPIELEN 1 bis 13 detailliert beschriebenen Synthesen hergestellt werden. Für die meisten erfindungsgemäßen Verbindungen kann die in BEISPIEL 1 dargestellte Synthese geeignet abgewandelt werden und damit analog eingesetzt werden. In den entsprechenden Synthesen einsetzbare Amin-Derivate und Boronsäureester oder Analoga sind im Schema 4 unten zusammengefasst. Ergänzend wird auch auf die Offenbarung WO 2010/139731 verwiesen, welche durch Bezugnahme vollumfänglich in die vorliegende Offenbarung aufgenommen sei.

Das Verfahren und die anschließende Aufarbeitung des Reaktionsgemisches können grundsätzlich als Batch-Reaktion oder in kontinuierlicher Reaktionsweise durchgeführt werden. Die kontinuierliche Reaktionsweise umfasst z.B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Die Ausgangsverbindungen sind in der Regel bekannt. Sind sie neu, so können sie nach an sich bekannten Methoden hergestellt werden. Falls gewünscht, können die Ausgangsstoffe in-situ gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

### Pharmazeutisch verwendbare Formen

Im Rahmen der Erfindung sind die erfindungsgemäßen Verbindungen so definiert, dass auch pharmazeutisch verwendbare Salze, Solvate einschl. Hydrate, Vorstufen der Verbindungen, Tautomere und optisch aktive Formen (wie z.B. Stereoisomere, Diastereomere, Enantiomere, Racemate) umfasst sind. Gegenstand der Erfindung ist auch die Verwendung von Mischungen der Verbindungen der Formel (I), z.B. Gemische zweier Diastereomere, z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere Mineralsäuren und ihre entsprechenden Salzen (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der erfindungsgemäßen Verbindungen verwendet werden.

Im Hinblick auf das oben Gesagte sieht man, dass unter dem Ausdruck "pharmazeutisch verwendbares Salz", oder auch "pharmazeutisch unbedenkliches" oder "pharmazeutisch akzeptables" Salz, im vorliegenden Zusammenhang eine erfindungsgemäße Verbindung zu verstehen ist, die in der Form eines ihrer Salze vorliegt, insbesondere dann, wenn diese Salzform der Verbindung im Vergleich zu ihrer freien Form verbesserte pharmakokinetische Eigenschaften verleiht. Die pharmazeutisch unbedenkliche Salzform der Verbindung kann dieser Verbindung auch erst eine gewünschte pharmakokinetische Eigenschaft verleihen und kann sogar die Pharmakodynamik der Verbindung in Bezug auf ihre therapeutische Wirksamkeit im Körper positiv beeinflussen.

Erfindungsgemäße Verbindungen können aufgrund ihrer Molekülstruktur chiral sein und dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomeren der Verbindungen der Formel (I) unterscheiden kann, kann es wünschenswert sein, die Enantiomere zu verwenden. In diesen Fällen kann das Endprodukt oder bereits das Zwischenprodukt in enantiomere Verbindungen, durch dem Fachmann bekannte chemische oder physikalische Maßnahmen, aufgetrennt oder bereits als solche bei der Synthese eingesetzt werden.

Es ist allgemein bekannt, dass Atome atomare Massen oder Massenzahlen haben können, die von den üblicherweise natürlich vorkommenden atomaren Massen oder Massenzahlen abweichen können. Beispiele für Isotope, die kommerziell verfügbar sind und die durch bekannte Methoden in eine erfindungsgemäße Verbindung inkorporiert werden können, sind Isotope von Wasserstoff, Kohlenstoff, Stickstoff, Sauerstoff, Phosphor, Fluor und Chor, bspw. ²H, ³H, ¹³C, ¹⁴C, ¹⁵N, ¹⁸O, ¹⁷O, ³¹P, ³²P, ³⁵S, ¹⁸F und ³⁶Cl. Der Einbau von schwereren Isotopen, insbesondere Deuterium (²H) in eine erfindungsgemäße Verbindung hat therapeutische Vorteile, die in der höheren metabolischen Stabilität dieser isotopenmarkierten Verbindung begründet liegen. Höhere metabolische Stabilität führt unmittelbar zu einer erhöhten *in vivo* Halbwertszeit, was eine geringere Dosierung ermöglicht.

Daher beziehen sich die Definitionen der Atome H, C, N usw., wie in den erfindungsgemäßen Verbindungen benutzt, auch auf die schwereren Isotope dieser Atome.

Erfindungsgemäß besonders bevorzugt ist die Verwendung von D (Deuterium, ²H) anstelle von Wasserstoff (¹H). Deuterium kann beispielsweise mittels eines geeigneten Substituenten an HET in den erfindungsgemäßen Verbindungen eingebaut werden, z.B.Trideuteriomethyl oder Trideuteriomethoxy.

### Verwendung

Es wurde überraschend gefunden, dass die erfindungsgemäßen Verbindungen eine spezifische Hemmung von Proteinkinasen, speziell ATM Kinase, bewirken. Entsprechend stellt die vorliegende Erfindung ATM Inhibitoren bereit, welche aus den erfindungsgemäßen Verbindungen ausgewählt sind.

Im Rahmen der hier vorgestellten Erfindung wurden erstmals neuartige 2,3-Dihydro-1H-imidazol[4,5-c]chinolin-Verbindungen bereitgestellt. Die erfindungsgemäßen Verbindungen steuern affin und/oder selektiv Serin-Threonin-Proteinkinasen, und zwar insbesondere ATM Kinase, an. Die Verbindungen aus Formel (I) zeichnen sich durch eine hohe Spezifität und Stabilität, niedrige Herstellungskosten und leichte Handhabung aus. Solche Eigenschaften bilden die Grundlage für eine reproduzierbaren Wirkungsweise und die verlässliche und sichere Wechselwirkung mit den entsprechenden Zielstrukturen. Die Erfindung beinhaltet auch die Verwendung der vorliegenden 2,3-Dihydro-*1H*-imidazol[4,5-c]chinolin-Derivate zur Hemmung, Regulation und/oder Modulation der Signalkaskade von Serin-Threonin-Proteinkinasen, insbesondere ATM Kinase, und bietet damit neuartige Werkzeuge für Forschung und/oder Diagnostik.

Ein weiterer Gegenstand der Erfindung betrifft deshalb die Verwendung von erfindungsgemäßen Verbindungen und/oder ihrer physiologisch unbedenklichen Solvate, Salze, Tautomere und/oder Stereoisomere, einschließlich deren Mischungen in allen Verhältnissen, zur Hemmung von Serin-Threonin-Proteinkinasen, insbesondere ATM Kinase. Der Begriff "Hemmung" bezieht sich auf jede Verringerung der Aktivität, die auf der Wirkung der spezifischen erfindungsgemäßen Verbindungen basiert, indem letztere in der Lage sind, mit dem Zielmolekül derart zu interagieren, dass eine Erkennung, Bindung und Blockierung ermöglicht wird. Die Verbindungen zeichnen sich durch hohe Affinität zu ATM Kinase aus, wodurch eine verlässliche Bindung und vorzugsweise vollständige Blockade der Kinaseaktivität sichergestellt wird. Die Verbindungen sind ferner sehr selektiv und ermöglichen damit eine ausschließliche und unmittelbare Erkennung der ATM Kinase. Der Begriff der "Erkennung" bezieht sich hierbei auf jede Art der Wechselwirkung zwischen der Verbindung und den genannten Zielmolekülen, insbesondere kovalente oder nichtkovalente Bindungen, wie z.B. eine kovalente Bindung, hydrophobe/ hydrophile Wechselwirkungen, van der Waals'sche Kräfte, Ionenbeziehung, Wasserstoffbrücken, Ligand-Rezeptor-Wechselwirkungen, Basenpaarungen von Nukleotiden oder Wechselwirkungen zwischen Epitop und Antikörper-Bindungsstelle.

Erfindungsgemäße Verbindungen und/oder ihre physiologisch unbedenklichen Salze, Tautomere und/oder Stereoisomere, einschließlich deren Mischungen in allen Verhältnissen, sind zur Verwendung bei der Behandlung von Erkrankungen geeignet, die durch Aktivität von Serin-Threonin-Proteinkinasen, und zwar insbesondere ATM, hervorgerufen, vermittelt und/oder verbreitet werden.

Die vorliegende Erfindung betrifft die erfindungsgemäßen Verbindungen und/oder ihrer physiologisch unbedenklichen Solvate, Salze, Tautomere und/oder Stereoisomere, einschließlich deren Mischungen in allen Verhältnissen zur Verwendung als Medikament. Erfindungsgemäß offenbart werden die erfindungsgemäßen Verbindungen und/oder ihre physiologisch unbedenklichen Salze, Tautomere und/oder Stereoisomere, einschließlich deren Mischungen in allen Verhältnissen, zur Verwendung bei der Behandlung von Erkrankungen, die durch die Aktivität von Serin-Threonin-Proteinkinasen, und zwar insbesondere ATM Kinase hervorgerufen, vermittelt und/oder verbreitet werden.

Gegenstand der vorliegenden Erfindung sind deshalb auch die erfindungsgemäßen Verbindungen und/oder ihre physiologisch unbedenklichen Solvate, Salze, Tautomere und/oder Stereoisomere, einschließlich deren Mischungen in allen Verhältnissen, zur Verwendung bei der Behandlung von Erkrankungen, die durch die Aktivität von Serin-Threonin-Proteinkinasen, und zwar insbesondere ATM Kinase, hervorgerufen, vermittelt und/oder verbreitet werden. Gegenstand der vorliegenden Erfindung ist entsprechend auch die Verwendung von erfindungsgemäßen Verbindungen und/oder ihrer physiologisch unbedenklichen Solvate, Salze, Tautomere und/oder Stereoisomere, einschließlich deren Mischungen in allen Verhältnissen, zur Herstellung eines Arzneimittels zur Behandlung von Erkrankungen, die durch die Aktivität von Serin-Threonin-Proteinkinasen, insbesondere ATM Kinase, hervorgerufen, vermittelt und/oder verbreitet werden. Anders ausgedrückt offenbart die vorliegende Erfindung auch eine erfindungsgemäße Verbindung und/oder pharmazeutisch verwendbares Salz, Solvat, Tautomer oder Stereoisomer davon, zur Verwendung bei der Behandlung von Krankheiten, die durch Inhibierung von ATM Kinase beeinflusst werden.

Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung kinaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung von Serin-Threonin-Proteinkinasen, vorzugsweise ATM Kinase, abhängig sind. Gegenstand der vorliegenden Erfindung sind auch die erfindungsgemäßen Verbindungen und/oder ihre physiologisch unbedenklichen Solvate, Salze, Tautomere und/oder Stereoisomere, einschließlich deren Mischungen in allen Verhältnissen, zur Verwendung bei der Behandlung von Krebs, Tumoren, und/oder Metastasen bzw. deren Verwendung bei der Herstellung eines Arzneimittels für ebendiese Verwendungen.

Der Tumor ist insbesondere ausgewählt aus der Gruppe von Erkrankungen von Plattenepithel, Blase, Magen, Nieren, Kopf, Hals, Ösophagus, Gebärmutterhals, Schilddrüse, Darm, Leber, Gehirn, Prostata, Urogenitaltrakts, lymphatisches System, Kehlkopf, Lunge, Haut, Blut und Immunsystem, und/oder der Krebs ist ausgewählt aus der Gruppe von Monozytenleukämie, Lungenadenokarzinom, kleinzelliges Lungenkarzinom, Bauchspeicheldrüsenkrebs, Glioblastom, Darmkarzinom, Brustkarzinom, akute myelotische Leukämie, chronische myelotische Leukämie, akute lymphatische Leukämie, chronische lymphatische Leukämie, Hodgkin-Lymphom und non-Hodgkin-Lymphom.

Gegenstand der vorliegenden Offenbarung ist darüber hinaus ein Verfahren zur Herstellung eines Medikaments, vorzugsweise zur Verwendung bei der Behandlung von Krebs und/oder Tumoren, bevorzugt der zuvor genannten Krebs- bzw. Tumorarten, umfassend:
i. Ermitteln, dass eine Konzentration, bei der eine erfindungsgemäße Verbindung und/oder pharmazeutisch verwendbares Salz, Solvat, Tautomer oder Stereoisomer davon, eine 50%ige Hemmung der Aktivität von ATM Kinase erzielt, 500 nM oder weniger beträgt, vorzugsweise 100 nM, 10 nM, 1 nM oder weniger, und
ii. Herstellen einer pharmazeutischen Zusammensetzung, die die Verbindung enthält.

Die Ermittlung der 50%igen Hemmung der Aktivität von ATM Kinase erfolgt dabei bevorzugt mit Hilfe des hierein beschriebenen Assays (IC50 ATM).

Erfindungsgemäße Verbindungen und/oder ihre physiologisch unbedenklichen Solvate, Salze, Tautomere und/oder Stereoisomere, einschließlich deren Mischungen in allen Verhältnissen, können auch zur Verwendung bei der Verlangsamung von Alterungsprozessen geeignet sein, wobei die Verlangsamung anhand des Vergleichs der Lebenspanne des behandelten Wirts oder dessen Zellen, Zellkulturen, Geweben oder Organen mit entsprechenden Positiv- oder Negativkontrollen und/oder Statistiken erfolgt.

Die Erfindung lehrt ferner ein Verfahren zur Behandlung von Krebs, Tumoren, und/oder Metastasen, wobei eine wirksame Menge mindestens einer erfindungsgemäßen Verbindung und/oder ihrer physiologisch unbedenklichen Solvate, Salze, Tautomere und/oder Stereoisomere, einschließlich deren Mischungen in allen Verhältnissen, einem zu behandelnden Probanden verabreicht wird. Bevorzugte Probanden im Sinne der Erfindung sind Mensch oder Tier, besonders bevorzugt der Mensch. Dem Fachmann ist hierbei bekannt, dass er die erfindungsgemäßen Verbindungen, die selbstverständlich auch als das erfindungsgemäße pharmazeutische Mittel verwendet werden können, in verschiedenen Dosierungen einem Organismus, insbesondere einem humanen Patienten, applizieren kann. Die wirksame Menge und die Art der Applikation können vom Fachmann durch Routineversuche bestimmt werden. Die vorherige Lehre der Erfindung und deren Ausführungsformen sind gültig und ohne Einschränkungen auf das Behandlungsverfahren anwendbar, sofern es sinnvoll erscheint.

Gegenstand der vorliegenden Offenbarung ist weiterhin die Verwendung einer erfindungsgemäßen Verbindung und/oder pharmazeutisch verwendbarem Derivat, Salz, Solvat, Tautomer oder Stereoisomer davon, zur Hemmung einer Proteinkinase, und zwar insbesondere ATM Kinase *in vitro.* Die genannte Verwendung von erfindungsgemäßen Verbindungen und/oder ihrer pharmazeutisch verwendbaren Solvate, Salze, Tautomere und/oder Stereoisomere, einschließlich deren Mischungen in allen Verhältnissen, zur Hemmung von Serin-Threonin-Proteinkinasen,und zwar insbesondere ATM Kinase, kann in *in vitro* oder *in vivo* Modellen geschehen. Die Suszeptibilität einer bestimmten Zelle gegenüber der Behandlung mit den erfindungsgemäßen Verbindungen kann durch Testen *in vitro* bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen *in vitro* können kultivierte Zellen aus einer Biopsieprobe verwendet werden. Die Menge der nach der Behandlung zurückbleibenden Zellen wird dann bestimmt. Die Verwendung *in vitro* erfolgt insbesondere an Proben von Säugerspezies, die an Krebs, Tumoren, Metastasen, Störungen der Angiogenese, retroviralen Erkrankungen, Immunerkrankungen und/oder pathogenen Alterungsprozessen leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Das Testen mehrerer spezifischer Verbindungen ermöglicht die Auswahl der Verbindung bzw. des Wirkstoffs, der am besten für die Behandlung des Patienten geeignet erscheint. Die *in vivo* Dosis der gewählten Verbindung wird vorteilhaft auf die Suszeptibilität der Kinase und/oder Schwere der Erkrankung des Patienten mit Blick auf die *in vitro* Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird.

Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird. Die Kinase wird insbesondere zu 50 % gehemmt, wenn die Konzentration der Verbindungen kleiner als 1 µM ist, vorzugsweise gleich oder kleiner als 0,5 µM, besonders bevorzugt kleiner als 0,1 µM, 0,05 µM oder 0,001 µM. Diese Konzentration wird als IC₅₀-Wert bezeichnet und bevorzugt mit Hilfe des hierin beschriebenen Assays ermittelt (IC50 ATM). "µM" steht gemäß der üblichen Nomenklatur für Micromol pro Liter, _{"}µM" für Nanomol pro Liter.

### Assays

Die erfindungsgemäßen Verbindungen zeigen eine vorteilhafte biologische Aktivität, die in den hierin beschriebenen Tests nachweisbar ist, wie z.B. auf Enzymen basierenden Assays.

Die Messung der Kinaseaktivität ist eine dem Fachmann wohlbekannte Technik. Generische Testsysteme zur Bestimmung der Kinaseaktivität mit Substraten, z.B. Histon (Alessi et al. (1996) FEBS Lett. 399(3): 333) oder dem basischen Myelinprotein sind in der Literatur beschrieben (Campos-González & Glenney (1992) JBC 267: 14535). Zur Identifikation von Kinase-Inhibitoren stehen verschiedene Assay-Systeme zur Verfügung. Beim Scintillation Proximity Assay (Sorg et al. (2002) J Biomolecular Screening 7: 11) und dem FlashPlate Assay werden die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al. (2002) J Biomolecular Screening 191). Andere nicht-radioaktive ELISA-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszens nachweisbar.

Im Rahmen der vorliegenden Erfindung wird die Hemmung (Inhibition) der Aktivität von ATM bevorzugt mit Hilfe des folgenden Assays bestimmt:

### ATM-Kinase Assay - Bestimmung der ATM Inhibition (IC50 ATM):

Der IC₅₀-Wert wurde mit Hilfe eines biochemischen ATM-Kinaseassays bestimmt. Der Assay besteht aus zwei Schritten: der enzymatischen Reaktion und dem Detektionsschritt. Zunächst werden ATM Protein *(Ataxia Telangiectasia* Mutated) und die Testsubstanz in unterschiedlichen Konzentrationen zzgl. Substratprotein p53 und ATP inkubiert. ATM vermittelt die Phosphorylierung von p53 an mehreren Positionen, u.a. an der Aminosäure S15. Die Menge an phosphoryliertem p53 wird mit Hilfe von spezifischen Antikörpern und der TR-FRET-Technik nachgewiesen. Der enzymatische ATM Assay wird als TR-FRET-(HTRF™, Cisbio Bioassays) basierter 384-Well-Assay durchgeführt. Im ersten Schritt wird aufgereinigtes humanes rekombinantes ATM (humane ATM, volle Länge, GenBank-ID NM_000051, exprimiert in einer Säugerzelllinie) in Assaypuffer 15 Minuten lang mit dem ATM Inhibitor in verschiedenen Konzentrationen sowie ohne Testsubstanz als Negativ- bzw. Neutralkontrolle inkubiert. Der Assaypuffer enthält 25 mM HEPES pH 8,0, 10 mM Mg(CH₃COO)₂, 1 mM MnCl₂, 0,1% BSA, 0,01% Brij® 35, 5mM Dithiothreitol (DTT). Die Testsubstanzlösungen wurden mit einem ECHO 555 (Labcyte) in die Mikrotiterplatten dispensiert. Im zweiten Schritt werden aufgereinigtes humanes rekombinantes cmycmarkiertes p53 (humanes p53, volle Länge, GenBank-ID BC003596, exprimiert in Sf21 Insektenzellen) und ATP hinzugefügt und das Reaktionsgemisch 30 - 35 Minuten bei 22°C inkubiert. Das pharmakologisch relevante Assayvolumen beträgt 5 µl. Die finalen Konzentrationen im Assay während der Inkubation des Reaktionsgemisches sind 0,3 - 0,4 nM ATM, 50 - 75 nM p53 und 10 µM ATP. Die enzymatische Reaktion wird durch die Zugabe von EDTA gestoppt. Die Bildung von phosphoryliertem p53 als Resultat der ATMvermittelten Reaktion in Gegenwart von ATP wird über spezifische Antikörper [markiert mit den Fluorophoren Europium (Eu) als Donor und d2 als Akzeptor (Cisbio Bioassays)] nachgewiesen, welche FRET ermöglichen. Hierzu werden 2 µl Antikörper-haltige Stopplösung (12,5 mM HEPES pH 8,0, 125 mM EDTA, 30 mM Natriumchlorid, 300mM Kaliumfluorid, 0,006 % Tween-20, 0,005 % Brij® 35, 0,21 nM anti-phospho-p53(ser15)-Eu Antikörper, 15 nM anti-cmyc-d2 Antikörper) zum Reaktionsgemisch zugegeben. Die Platten werden nach gewöhnlich 2 Stunden Inkubation (zwischen 1,5 und 15h) zur Signalentwicklung in einem Plattenlesegerät (EnVision, PerkinElmer) unter Verwendung des TRF-Modus (sowie mit Laser-Anregung) analysiert. Nach Anregung des Donors Europium bei einer Wellenlänge von 340 nm werden das emittierte Fluoreszenzlicht sowohl des Akzeptors d2 bei 665 nm als auch des Donors Eu bei 615 nm gemessen. Die Menge an phosphoryliertem p53 ist direkt proportional zum Quotienten aus den emittierten Lichtmengen, d.h. den relativen Fluoreszenzeinheiten (RFU) bei 665 nm und 615 nm. Die Messdaten wurden mittels der Genedata Screener Software verarbeitet. IC₅₀ Bestimmungen werden insbesondere durch Anpassen einer Dosiswirkungskurve an die Datenpunkte mittels nichtlinearer Regressionsanalyse bestimmt.
IC₅₀ = halbmaximale inhibitorische Konzentration
ATP = Adenosintriphosphat
TR-FRET = Time-Resolved Fluorescence Resonance Energy Transfer
HTRF® = Homogeneous Time Resolved Fluorescence
HEPES = 2-(4-(2-Hydroxyethyl)-1-piperazinyl)-ethansulfonsäure
Mg(CH₃COO)₂ = Magnesiumacetat
MnCl₂ = Mangan(II)-chlorid
BSA = Bovines Serum Albumin
EDTA = Ethylendiamintetraacetat
TRF = Time Resolved Fluorescence

Die Aktivität der erfindungsgemäßen Substanzen in zellulärem Umfeld kann mit Hilfe des folgenden Assays bestimmt werden:

### Zellulärer pCHK2 Assay:

Zur Identifizierung von Substanzen, welche die Phosphorylierung der Proteinkinase CHK2 an der Aminosäure Threonin68 hemmen, wurde in HCT116 Zellen ein Immunfluoreszenzbasierter "high content" Analyse Assay verwendet.

*In vitro* zellbasierter Immunfluoreszenz-Assay zur Identifizierung von Inhibitoren der Bleomycin-induzierten Phosphorylierung von CHK2 (phospho-Thr68) in der humanen Kolonkarzinomzelllinie HCT116:
HCT116 Zellen werden in definierter Zelldichte in 384er Multiwell-Platten in Kulturmedium (DMEM high glucose, 2mM GlutaMax, 1mM Na-Pyruvat, 10% FCS) ausgesät und über Nacht bei 37°C und 10% CO₂ inkubiert. Am Folgetag werden die Testsubstanzen in definiertem Konzentrationsbereich (1nM bis 30µM) in Kombination mit 10µM Bleomycin zugegeben, wobei die Konzentration des Lösungsmittels DMSO konstant bei 0.5% gehalten wird. Nach vier Stunden Inkubation bei 37°C und 10% CO₂ werden die Zellen fixiert (15min 4% Formaldehyd in PBS), permeabilisiert (10min 0.2% Triton X-100 in PBS) und nach Blockierung unspezifischer Bindungsstellen (10% Ziegenserum, 1% BSA in PBS) über Nacht bei 4°C mit einem spezifischen anti-pCHK2 Antikörper inkubiert (Cell Signaling #2661). Der Nachweis von pCHK2 (Thr68) erfolgt mit einem Alexa488-markierten sekundären anti-Kaninchen-IgG-Antikörper. Paralleles Anfärben von DNA mit Propidiumjodid ermöglicht die Zellzahlbestimmung. Die Detektion des pCHK2-Signals erfolgt mit einem high-content-Imager (IMX Ultra von Molecular Devices) und automatische Imageanalyse mit der zum Gerät gehörenden MetaXpress Software. Es wird die Anzahl an Zellkernen bestimmt, die ein pCHK2 Signal über einem definierten Hintergrund aufweisen.

Ferner kann die Wirkung, insbesondere Hemmung, anderer Kinasen und damit die Selektivität der erfindungsgemäßen Verbindungen mit Hilfe der folgenden Assays bestimmt werden:

### mTOR (human)

mTOR (human) wurde mit 50 mM HEPES pH 7.5, 1 mM EGTA, 0.01% Tween 20, 2 mg/mL des Substrates, 3 mM MnCl₂ und [γ-³³P-ATP] (spezifische Aktivität ungefähr 500 cpm/pmol, Konzentration wie erforderlich) inkubiert. Die Reaktion wurde durch Zugabe einer MgATP-Lösung initiiert. Nach einer 40 minütigen Inkubation bei Raumtemperatur wurde die Reaktion durch Zugabe von 3% Phosphorsäure gestoppt. 10 µL der Reaktionslösung wurden dann auf ein P30 Filtermat getropft und drei Mal für 5 min in 75 mM Phosphorsäure und einmal in Methanol gewaschen, getrocknet und mittels Flüssigszintillationszählung ausgewertet.

### PI3K p110α/p85α (human), nicht-radioaktiver Assay

PI3K p110α/p85α (human) wurde in einem Assaypuffer aus 10 µM Phosphatidylinositol-4,5-bisphosphat und MgATP (Konzentration wie erforderlich) inkubiert. Die Reaktion wurde durch Zugabe von ATP-Lösung initiiert. Nach einer 40 minütigen Inkubation bei Raumtemperatur wurde die Reaktion durch Zugabe einer Lösung bestehend aus EDTA und biotinyliertem Phosphatidylinositol-3,4,5-trisphosphat zum Stillstand gebracht. Schließlich wurde der Detektionspuffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale mit Hilfe der Formel HTRF = 10000 x (Em665nm/Em620nm) ausgewertet.

### PI3K p110β/p85α (human), nicht-radioaktiver Assay

PI3K p110β/p85α (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.

### PI3K p110δ/p85α (human), nicht-radioaktiver Assay

PI3K p110δ/p85α (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.

### PI3K (p120γ) (human), nicht-radioaktiver Assay

PI3K (p120γ) (human) wurde in einem Assay Puffer aus 10 µM Phosphatidylinositol-4,5-bisphosphat und MgATP (Konzentration wie erforderlich) inkubiert. Die Reaktion wurde durch Zugabe von MgATP-Lösung initiiert. Nach einer Inkubationszeit von 30 min bei Raumtemperatur wurde die Reaktion durch Zugabe einer Lösung bestehend aus EDTA und biotinyliertem Phosphatidylinositol-3,4,5-trisphosphat zum Stillstand gebracht. Letztendlich wurde der Detektion Puffer, bestehend aus einem Europium-gelabelten anti-GST monoklonalen Antikörper, einer GST-markierten GRP1 PH Domäne und Streptavidin Allophycocyanin, zugefügt. Die Platte wurde über homogene zeitaufgelöste Fluoreszenz (HTRF) ausgelesen und die entsprechenden Signale über die Formel HTRF = 10000 x (Em665nm/Em620nm) ausgewertet.
MgATP = Magnesium 5'-O-[hydroxy({[(hydroxyphosphinato)oxy]phosphinato}oxy)-phosphoryl]adenosine
MgCl₂ = Magnesiumdichlorid
EGTA = Ethylenglycol-bis(aminoethylether)-N,N,N',N'-tetraessigsäure
Tween 20 = Polysorbat 20

### Arzneimittel/Pharmazeutische Zusammensetzung

Gegenstand der Erfindung ist auch ein Arzneimittel bzw. Medikament umfassend mindestens eine erfindungsgemäße Verbindung und/oder ihre physiologisch unbedenklichen Solvate, Salze, Tautomere und/oder Stereoisomere, einschließlich deren Mischungen in allen Verhältnissen.

Gegenstand der Erfindung ist ferner eine pharmazeutische Zusammensetzung umfassend eine wirksame Menge von mindestens einer erfindungsgemäßen Verbindung und/oder eines ihrer physiologisch unbedenklichen Salze, Solvate, Tautomere und/oder Stereoisomere, einschließlich deren Mischungen in allen Verhältnissen, optional zusammen mit mindestens einem pharmazeutisch verträglichen Hilfsstoff bzw. optional einem Träger- und/oder Hilfsstoff.

Ein "Arzneimittel", "Medikament" sowie eine "pharmazeutische Zusammensetzung" oder "pharmazeutische Formulierung" ist zu verstehen jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Krebs, Tumoren und/oder Metastasen.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden.

Das Einbringen des pharmazeutischen Mittels in eine Zelle respektive einen Organismus kann erfindungsgemäß auf jede Art und Weise geschehen, die es ermöglicht, dass die Kinasen mit den in der Zusammensetzung enthaltenen Verbindungen in Kontakt gebracht werden, infolgedessen eine Antwort induziert wird. Das pharmazeutische Mittel der vorliegenden Erfindung kann oral, transdermal, transmucosal, transurethal, vaginal, rektal, pulmonal, enteral und/oder parenteral angewendet werden. Die gewählte Art der Verabreichung richtet sich nach der Indikation, der zu verabreichenden Dosis, Individuumsspezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Kolliphor, Glycerintriacetat, Gelatine, Kohlenhydrate, wie z.B. Laktose oder Stärke, Hydroxypropylmethylcellulose (HPMC), Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel als Injektionslösung vor. Für die Herstellung der Injektionslösung können wässrige Medien, wie z.B. destilliertes Wasser oder physiologische Salzlösungen verwendet werden, wobei letztere saure und basische Additionssalze einschließen. Das pharmazeutische Mittel kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Verbindung in der Formulierung kann 0,1 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge", "wirksame Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit oder sogar die Infektion mit einem Pathogen nach dem Eindringen einzelner Vertreter derart, dass deren nachfolgende Ausbreitung stark verringert wird oder sie sogar vollständig inaktiviert werden. Eine "prophylaktische Wirkung" umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit. Eine "therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die erfindungsgemäßen Verbindungen anwendbar, sofern es sinnvoll erscheint.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 100 mg. Die tägliche Dosierung liegt insbesondere zwischen 0,02 und 100 mg/kg Körpergewicht.

Aufgrund ihrer überraschend starken und/oder selektiven Kinase-Hemmung, insbesondere Hemmung von ATM-Kinase, die über die Reparatur von Doppelstrang-DNA zelluläre Prozesse regeln, können die Verbindungen der Erfindung in vorteilhaft niedriger Dosierung verabreicht werden, während sie im Vergleich mit weniger potenten bzw. weniger selektiven Inhibitoren eine ähnliche oder sogar überlegene biologische Wirksamkeit erzielen. Eine reduzierte Dosis geht typischerweise mit verringerten medizinischen Nebenwirkungen einher. Darüber hinaus wird eine hoch selektive Hemmung generell auch durch eine Verringerung unerwünschter Nebenwirkungen reflektiert.

Sämtliche genannte sowie weitere Bestandteile bzw. Komponenten eines Medikaments bzw. einer pharmazeutischen Formulierung sind dem Fachmann geläufig und können in Routineversuchen eine spezielle Ausgestaltung für die erfindungsgemäße Lehre erfahren.

### Kombinationstherapie

Arzneimittel und pharmazeutische Zusammensetzungen, die die erfindungsgemäßen Verbindungen enthalten, und der Einsatz dieser Verbindungen zur Behandlung Kinasevermittelter Störungen sind ein vielversprechenden Ansatz für ein breites Spektrum von Therapien, wodurch sich bei Mensch und Tier eine direkte und unmittelbare Linderung von Symptomen erreichen lässt. Dies ist besonders für die wirksame Bekämpfung schwerer Krankheiten wie Krebs von Vorteil, entweder als Monotherapie, wie oben geschildert, oder in Kombination mit anderen Therapien, wie beispielsweise Chemo- oder Radiotherapie. Die Schlüsselbeteiligung von ATM an DNA-Reparaturprozessen und der Nachweis, dass ATM-Kinase-Mangel Säugerzellen strahlungsempfindlicher werden lässt, ermöglicht eine therapeutische Anwendung der ATM spezifischen Inhibitoren im Rahmen der Behandlung von z.B. soliden Krebstumoren durch Bestrahlungs- und/oder einer vorzugswiese auf DNA-Doppelstrang-Schäden abzielenden Chemotherapie.

Zur Unterstützung der medizinischen Wirkung kann die pharmazeutische Zusammensetzung entsprechend in einer Ausgestaltung der Erfindung auch einen oder mehrere weitere Wirkstoffe umfassen, beispielsweise ein Antikrebsmittel, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Kinasehemmung bestimmte Antikrebsmittel besser wirken oder durch die Verminderung der Dosis die Anzahl der Nebenwirkungen dieser Medikamente reduziert wird. Entsprechend können die erfindungsgemäßen Verbindungen in Kombination mit anderen Wirkstoffen, einschließlich Antikrebsmitteln, verabreicht werden.

In einer bevorzugten Ausführungsform der Erfindung wird die erfindungsgemäße pharmazeutische Zusammensetzung mit einem Antikrebsmittel kombiniert bzw. umfasst dieses. Entsprechend ist Gegenstand der vorliegenden Erfindung auch eine erfindungsgemäße Verbindung und/oder pharmazeutisch verwendbares Salz, Solvat, Tautomer oder Stereoisomer davon zur Verwendung bei der Behandlung von Krebs, Tumoren und/oder Metastasen in Kombination mit mindestens einem Antikrebsmittel.

Wie hier verwendet, betrifft der Begriff "Antikrebsmittel" jedes Mittel, das einem Patienten mit Krebs, Tumoren und/oder Metastasen zum Zweck der Behandlung des Krebses verabreicht wird.

Das Antikrebsmittel ist besonders bevorzugt ausgewählt aus der Gruppe umfassend Cytokine, Chemokine, pro-apoptotische Mittel, Interferone, radioaktive Verbindungen, Östrogenrezeptor-Modulatoren, Androgenrezeptor-Modulatoren, Retinoidrezeptor-Modulatoren, Zytotoxika, Zytostatika, Prenyl-Proteintransferase-Hemmer und Angiogenese-Hemmer oder Kombinationen davon. Es ist bevorzugt, dass das Antikrebsmittel den Nukleinsäure- und/oder Proteinstoffwechsel, die Zellteilung, DNA-Replikation, Purin-, Pyrimidin- und/oder Aminosäure-Biosynthese, Genexpression, mRNA-Prozessierung, Proteinsynthese, Apoptose oder Kombinationen davon verändert, insbesondere abschwächt.

Erfindungsgemäß bevorzugte Antikrebsmittel sind solche, die die DNA von Tumorzellen schädigen und damit in DNA-Replikation, DNA-Transkription oder die Genexpression eingreifen. Hierfür kommen insbesondere in Frage:
- Alkylierungsagentien, wie Altretamin, Bendamustin, Busulfan, Carmustin, Chlorambucil, Chlormethine, Cyclophosphamid, Dacarbazin, Ifosfamid, Improsulfantosylat, Lomustin, Melphalan, Mitobronitol, Mitolactol, Nimustin, Ranimustin, Temozolomid, Thiotepa, Treosulfan, Mechloretamin, Carboquon, Apaziquon, Fotemustin, Glufosfamid, Palifosfamid, Pipobroman, Trofosfamid, Uramustin,
- Platinverbindungen, wie Carboplatin, Cisplatin, Eptaplatin, Miriplatinhydrat, Oxaliplatin, Lobaplatin, Nedaplatin, Picoplatin, Satraplatin;
- DDR (DNA damage response) Inhibitoren, wie Topoisomerase-Inhibitoren, z.B. Etoposid, Irinotecan, Razoxan, Sobuzoxan, Topotecan, Camptothecin, Doxorubicin, Amsacrin; Poly-(ADP-ribose)-polymerase (PARP) Inhibitoren, z.B. Talazoparib, Olaparib, Veliparib, Rucaparib, CEP 9722, MK4827, BGB-290; ATR *(Ataxia*telangiectasia and Rad3 related) Inhibitoren, z.B. VE-822, AZ20, AZD6738;
- DNA-verändernde Agentien, wie Amrubicin, Bisantren, Decitabin, Mitoxantron, Procarbazin, Trabectedin, Clofarabin, Amsacrin, Brostallicin, Pixantron, Laromustine;
- Antikrebs-Antibiotica, wie Bleomycin, Dactinomycin, Doxorubicin, Epirubicin, Idarubicin, Levamisol, Miltefosin, Mitomycin C,Romidepsin, Streptozocin, Valrubicin, Zinostatin, Zorubicin, Daunurobicin, Plicamycin, Aclarubicin, Peplomycin, Pirarubicin;
- Alpha-Emitter, wie Alpharadin (²²³Ra Dichlorid, Xofgio), ²¹¹At, ²¹³Bi, ²²⁵Ac, ²²⁷Th.

Besonders bevorzugt sind Bleomycin, Alpharadin, und DDR Inhibitoren, z.B. Etoposid, Irinotecan, Razoxan, Sobuzoxan, Topotecan, Camptothecin, Doxorubicin, Amsacrin; Talazoparib, Olaparib, Veliparib, Rucaparib, CEP 9722, MK4827, BGB-290; VE-822, AZ20, AZD6738.

Die Erfindung kann auch als Kit praktiziert werden, das die erfindungsgemäßen Verbindungen enthält. Das Kit besteht aus getrennten Packungen (a) einer wirksamen Menge einer erfindungsgemäßen Verbindung und/oder ein physiologisch unbedenklichen Salzes, Solvats, Tautomers und/oder Stereoisomers davon, einschließlich Mischungen in allen Verhältnissen, und (b) einer wirksamen Menge eines weiteren Wirkstoffs. Der weitere Wirkstoff ist bevorzugt ein Antikrebsmittel.

Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge einer erfindungsgemäßen Verbindung und/oder ihrer pharmazeutisch verwendbaren Solvate, Salze, Tautomere und/oder Stereoisomere, einschließlich deren Mischungen in allen Verhältnissen, und einer wirksamen Menge eines weiteren Arzneimittelwirkstoffs gelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Eine weitere Ausgestaltung der vorliegenden Erfindung betrifft die erfindungsgemäßen Verbindungen in Kombination mit Radiotherapie und/oder mit mindestens einem weiteren Wirkstoff, vorzugsweise in Kombination mit Radiotherapie und/oder einem Antikrebsmittel. Anders ausgedrückt betrifft eine weitere Ausgestaltung der vorliegenden Erfindung die erfindungsgemäßen Verbindungen zur Verwendung bei der Behandlung von Krebs, Tumoren und/oder Metastasen in Kombination mit Radiotherapie. Ferner ist Gegenstand der vorliegenden Erfindung eine erfindungsgemäße Verbindung und/oder pharmazeutisch verwendbares Salz, Solvat, Tautomer oder Stereoisomer davon, zur Verwendung bei der Sensibilisierung von Krebszellen gegenüber einem Antikrebsmittel und/oder ionisierender Strahlung.

Technische Bestrahlungsmethoden, die klinisch Verwendung finden, umfassen vorzugsweise Photonenbestrahlung (klassische, elektromagnetische Röntgen-/GammaStrahlung), Protonenbestrahlung, Schwerionenbestrahlung (ionisierter Kohlenstoff) sowie Neutronenbestrahlung, ohne hierauf beschränkt zu sein. Dem Fachmann sind diese Radiotherapien und weitere geeignete Bestrahlungstherapien im Sinne der Erfindung bekannt, wie z.B. aus Herrmann et al. (2006) Klinische Strahlenbiologie, Elsevier München, 4. Auflage, 67-68; Bhide & Nutting (2010) BMC Medicine 8: 25; Choi & Hung (2010) Current Urology Reports 11(3): 172. Als häufigste Anwendung ist die Photonenbestrahlung technisch durch die Verfahren IMRT (Intensitäts-modulierte Radiotherapie) sowie durch bildgebende Verfahren (dreidimensional konforme Radiotherapie) bei der Bestrahlungsplanung und -durchführung zur möglichst exakten Fokussierung verfeinert worden. Die erfindungsgemäßen Verbindungen erzielen bei existierenden Therapien und Bestrahlungen synergistische Effekte und/oder stellen die Wirksamkeit existierender Therapien und Bestrahlungen wieder her. Noch eine weitere Ausgestaltung der Erfindung bezieht sich auf die Verwendung von mindestens einer Verbindung und/oder ihrer pharmazeutisch verwendbaren Salze, Solvate, Tautomere und/oder Stereoisomere, einschließlich deren Mischungen in allen Verhältnissen, zur Sensibilisierung von Krebszellen gegenüber einem Antikrebsmittel und/oder ionisierender Strahlung (Radiotherapie) unter der Maßgabe, dass die Sensibilisierung nicht *in vivo* am menschlichen oder tierischen Körper erfolgt.

Die Sensibilisierung erfolgt vorzugsweise *ex vivo* oder *in vitro,* indem die Verbindungen Zellen, Zellkulturen, Geweben oder Organen verabreicht werden, die Serin-Threonin-Proteinkinasen umfassen. Die *ex vivo*-Verwendung wird insbesondere bei tierischen Zellen angewandt, die aus einem tierischen Organismus stammen, der von einer Krankheit betroffen ist, die ausgewählt ist aus der Gruppe von Krebs, Tumoren, Metastasen und/oder Störungen der Angiogenese. Die *ex vivo*-behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße *ex vivo-*Sensibilisierung ist insbesondere von Vorteil, um den spezifische Wirkung der Verbindungen zu testen, so dass unter Auswertung dieser *ex vivo*-Daten die *in vivo*-Dosis entsprechend voreingestellt werden kann. Im Ergebnis dessen wird der therapeutische Effekt signifikant erhöht. Alternativ ist die Erfindung auch für die Anwendung *in vivo* ausgestaltet und betrifft mindestens eine erfindungsgemäße Verbindung und/oder ihre physiologisch unbedenklichen Salze, Tautomere und/oder Stereoisomere, einschließlich deren Mischungen in allen Verhältnissen, zur Verwendung zur Sensibilisierung von Krebszellen gegenüber einem Antikrebsmittel und/oder ionisierender Strahlung.

Zusammenfassend ist fest zu halten, dass die erfindungsgemäßen Verbindungen einzeln und/oder in Kombination mit anderen Behandlungsmaßnahmen verwendet werden können, wie z.B. chirurgischen Eingriffen, Immun-, Strahlen- und/oder Chemotherapie. Letztere beziehen sich auf eine zielgerichtete Therapie mit einem beliebigen NME (d.h. NCE und/oder NBE) als Mono- und/oder On-Target-/Off-Target-Kombinationstherapie.

Wie vorliegend in der Spezifikation einschließlich der anhängigen Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "eine Verbindung" ein einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

### BEISPIELE

Im Folgenden wird die Erfindung anhand nicht limitierender Beispiele für konkrete Ausführungsformen und Vergleichsbeispielen näher erläutert. Die Beispiele (insbesondere Verbindungsbeispiele) sind insbesondere dahingehend zu interpretieren, dass sie nicht auf die konkret veranschaulichten Merkmalskombinationen beschränkt sind, sondern die beispielhaften Merkmale wiederum frei kombiniert werden können, solange die Aufgabe der Erfindung gelöst wird.

### Analytische Methoden

NMR (¹H) wurde mit den folgenden Parametern durchgeführt.
Geräte: Bruker Avance DRX 500, Bruker Avance 400, Bruker DPX 300 Standartbedingungen (im Einzelfall abweichend)
Referenz: TMS
TD (Time Domaine = Anzahl der Datenpunkte oder digitale Auflösung): 65536 Lösungsmittel: DMSO-d6
NS (Number of Scans = Häufigkeit der Abtastung): 32
SF (Spectrometer Frequence = Sendefrequenz): siehe oben
TE (Temperatur): 297 K
Kopplungskonstanten (J) werden in Hertz (Hz) angegeben

HPLC-MS wurde mit den folgenden Parametern durchgeführt.

Geräte:
- Shimadzu LCMS-2020,
- Shimadzu SP-M20A 2010EV
- Shimadzu UFLC-MS 2010EV

Verwendete Säulen:
- Shim-pack VP-ODS,
- Shim-pack XR-ODS,
- Kinetex XB-C18 100A,
- Xbridge BEH C18,
- Gemini-NX 3u C18 110A
- ACE UltraCore 2.5 SuperC18

Methoden: Lösungsmittel-Gradienten mit
- A: Wasser + 0.1% Ameisensäure, B: Acetonitril + 0.1% Ameisensäure;
- A :Wasser + 0.05% Trifluoressigsäure, B : Acetonitril + 0.05% Trifluoressigsäure
- A: Wasser + 5mM Ammoniumcarbonat, B: Acetonitrile

Detektionswellenlänge: 220 nm
MS-Typ: API-ES

### Beschreibung der Synthesen

### BEISPIEL 1: Synthese von 1-(3-Fluor-2-pyridyl)-7-methoxy-3-methyl-8-(1-methylpyrazol-4-yl)imidazo[4,5-c]chinolin-2-on (Vergleichsbeispiel 14)

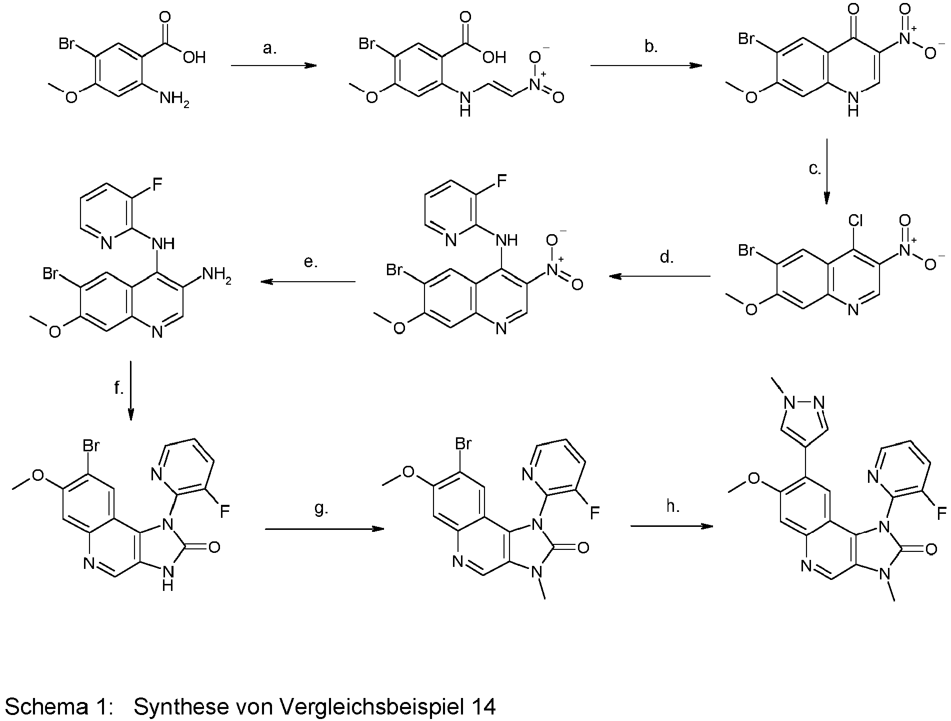

### a. Synthese von 5-Brom-4-methoxy-2-[[(E)-2-nitrovinyl]amino]benzoesäure

Einer Lösung von Natriumhydroxid (14,7 g, 368 mmol) in Wasser (33 mL) wurde unter Rühren Nitromethan (0,63 mL, 11,7 mmol) bei Raumtemperatur zugetropft. Anschließend wurde langsam für 5 Minuten auf 45°C unter Rühren erwärmt. Die Reaktionslösung wurde danach auf Raumtemperatur abgekühlt und erneut Nitromethan (0,63 mL, 11,7 mmol) zugetropft. Die Reaktionsmischung wurde anschließend für 10 Minuten weitergerührt, wobei eine klare, rötliche Lösung entstand. Nach kurzem Erwärmen (5 Minuten) auf 50°C wurde auf Raumtemperatur abgekühlt und auf Eis (11 g) abdekantiert. Die wässrige Lösung wurde mit konz. Salzsäure vorsichtig auf pH < 2 angesäuert und anschließend sofort einer Lösung aus 2-Amino-5-brom-4-methoxy-benzoesäure (8,00 g, 32,5 mmol) in Wasser (259 mL), angesäuert mit konz. Salzsäure (126 mL, 2,75 mol), unter Rühren zugefügt. Die erhaltene Suspension wurde über Nacht gerührt und anschließend filtriert. Der Rückstand wurde bei 50° getrocknet, wobei 9,60 g (93 %) 5-Brom-4-methoxy-2-[[(E)-2-nitrovinyl]amino]benzoesäure als farbloser Feststoff erhalten wurde.

### b. Synthese von 6-Brom-7-methoxy-3-nitro-1H-chinolin-4-on

5-Brom-4-methoxy-2-((E)-2-nitro-vinylamino)-benzoesäure (4,0 g, 12,6 mmol) wurde in N,N-Dimethylformamid (150 mL) gelöst. Anschließend wurde 1,1'-Carbonyldiimidazol (3,07 g, 18,9 mmol) bei Raumtemperatur unter Rühren zugefügt. Die Reaktionslösung wurde über Nacht bei Raumtemperatur gerührt. Anschließend wurde Acetonitril (120 mL) zugefügt. Die erhaltene Suspension wurde gekühlt und danach abfiltriert. Der gelbe Rückstand wurde mit Diethylether gewaschen und bei 40°C über Nacht getrocknet, wobei 3,0 g (80 %) 6-Brom-7-methoxy-3-nitro-1H-chinolin-4-on als farbloser Feststoff erhalten wurde.

### c. Synthese von 6-Brom-4-chlor-7-methoxy-3-nitro-chinolin

Unter einer trockenen Stickstoffatmosphäre wurde 6-Brom-7-methoxy-3-nitro-1H-chinolin-4-on (2,50 g, 8,36 mmol) vorgelegt. Anschließend wurden Phosphorylchlorid (20 mL, 215 mmol) und N,N-Dimethylformamid (0,13 mL, 1,68 mmol) zugefügt. Die Reaktionslösung wurde 12 Stunden bei 115°C unter Rühren erhitzt. Anschließend wurde im Vakuum eingeengt und der erhaltene Rückstand an Kieselgel chromatographisch (Petrolether/Essigsäureethylester = 87:13, Volumenanteile) aufgereinigt, wobei 2,40 g (90 %) 6-Brom-4-chlor-7-methoxy-3-nitro-chinolin als farbloser Feststoff erhalten wurden.

### d. Synthese von 6-Brom-N-(3-fluor-2-pyridyl)-7-methoxy-3-nitro-chinolin-4-amin

Unter einer trockenen Stickstoffatmosphäre wurden 3-Fluorpyridin-2-amin (390 mg, 3,48 mmol), gelöst in N,N-Dimethylformamid (10 mL), vorgelegt. Anschließend wurde der Lösung Natriumhydrid (630 mg, 26,3 mmol) zugefügt und weitere 5 Minuten bei Raumtemperatur gerührt. Danach wurde 6-Brom-4-chlor-7-methoxy-3-nitro-chinolin (1,00 g, 3,15 mmol) zur Reaktionsmischung gegeben, 30 Minuten bei Raumtemperatur gerührt und anschließend durch Zugabe von Eiswasser (100 mL) abgebrochen. Die wässrige Lösung wurde mit Essigsäureethylester dreimal mit je 100 mL extrahiert. Die vereinten organischen Phasen wurden über wasserfreiem Natriumsulfat getrocknet, abfiltriert und im Vakuum zur Trockne eingeengt, wobei 1,0 g (81 %) 6-Brom-N-(3-fluor-2-pyridyl)-7-methoxy-3-nitrochinolin-4-amin als gelber Feststoff erhalten wurden.

### e. Synthese von 6-Brom-N-4-(3-fluor-2-pyridyl)-7-methoxy-chinolin-3,4-diamin

6-Brom-N-(3-fluor-2-pyridyl)-7-methoxy-3-nitrochinolin-4-amin (1,0 g, 2,54 mmol) wurde unter einer Stickstoffschutzgasatmosphäre in Methanol (50 mL) gelöst vorgelegt. Anschließend wurde der Lösung Raney-Ni (100 mg, 1,17 mmol) zugefügt und die Reaktionsmischung unter einer Wasserstoffatmosphäre 30 Minuten bei Normaldruck gerührt. Nach Belüftung mit Stickstoff, wurde die Suspension filtriert und das Filtrat im Vakuum zur Trockne eingeengt, wobei 0,8 g (87 %) 6-Brom-N-4-(3-fluor-2-pyridyl)-7-methoxy-chinolin-3,4-diamin als gelber Feststoff erhalten wurden.

### f. Synthese von 8-Brom-1-(3-fluor-2-pyridyl)-7-methoxy-3H-imidazo[4,5-c]chinolin-2-on

6-Brom-N-4-(3-fluor-2-pyridyl)-7-methoxy-chinolin-3,4-diamin (0,8 g, 2,20 mmol) wurden in Tetrahydrofuran (25 mL) gelöst vorgelegt. Anschließend wurden 1,1'-Carbonyldiimidazol (1,78 g, 11,0 mmol) und Hünig-Base (1,42 g, 11,0 mmol) zugefügt. Die Reaktionslösung wurde 2 Stunden bei 40°C unter Rühren erwärmt. Danach wurde durch Zugabe von Eiswasser (200 mL) abgebrochen. Die wässrige Phase wurde mit Essigsäureethylester dreimal mit je 50 mL extrahiert. Die vereinten organischen Phasen wurden über wasserfreiem Natriumsulfat getrocknet, abfiltriert und im Vakuum zur Trockne eingeengt, wobei 0,8 g (93 %) 8-Brom-1-(3-fluor-2-pyridyl)-7-methoxy-3H-imidazo[4,5-c]chinolin-2-on als hellgelber Feststoff erhalten wurden.

### g. Synthese von 8-Brom-1-(3-fluor-2-pyridyl)-7-methoxy-3-methyl-imidazo[4,5-c]chinolin-2-on

Unter einer trockenen Stickstoffschutzatmosphäre wurde 8-Brom-1-(3-fluor-2-pyridyl)-7-methoxy-3H-imidazo[4,5-c]chinolin-2-on (0,8 g, 2,06 mmol), gelöst in N,N-Dimethylformamid (10 mL), vorgelegt. Anschließend wurden Natriumhydrid (412 mg, 17,2 mmol) und Methyliodid (1,46 g, 10,3 mmol) zugefügt. Die Reaktionsmischung wurde eine Stunde lang bei Raumtemperatur gerührt. Anschließend wurde durch Zugabe von Eiswasser (100 mL) abgebrochen. Der erhaltene Niederschlag wurde abfiltriert und unter Vakuum getrocknet, wobei 0,6 g (72%) 8-Brom-1-(3-fluor-2-pyridyl)-7-methoxy-3-methyl-imidazo[4,5-c]chinolin-2-on als hellgelber Feststoff erhalten wurden.

### h. Synthese von 1-(3-Fluor-2-pyridyl)-7-methoxy-3-methyl-8-(1-methylpyrazol-4-yl)imidazo[4,5-c]chinolin-2-on (Verbindungsbeispiel 14)

Unter einer Argon-Inertgasatmosphäre wurden in einer geschlossenen Apparatur 8-Brom-1-(3-fluor-2-pyridyl)-7-methoxy-3-methyl-imidazo[4,5-c]chinolin-2-on (150 mg, 0,37 mmol), 1-Methyl-4-(tetramethyl-1,3,2-dioxaborolan-2-yl)-1H-pyrazol (93 mg, 0,45 mmol), Pd(PPh₃)₄ (43 mg, 0,04 mmol) und Kaliumcarbonat (103 mg, 0,75 mmol) in 1,4-Dioxan (10 mL) und Wasser (3 mL) vorgelegt. Die Reaktionsmischung wurde 2 Stunden lang unter Rühren auf 80°C erhitzt. Anschließend wurde auf Raumtemperatur abgekühlt und die Reaktionsmischung im Vakuum zur Trockne eingeengt. Der Rückstand wurde an Kieselgel chromatographisch (Essigsäureethylester/Methanol = 10:1, Volumenanteile) vorgereinigt. Das Eluat wurde zur Trockne eingeengt und das erhaltene Rohprodukt mittels präparativer RP-HPLC (Wasser/Acetonitril) endgereinigt. Nach Einengen der Produktfraktionen wurde 1-(3-Fluor-2-pyridyl)-7-methoxy-3-methyl-8-(1-methylpyrazol-4-yl)imidazo[4,5-c]chinolin-2-on (60 mg, 40%, Verbindungsbeispiel 14) als farbloser Feststoff erhalten.

### BEISPIEL 2: Synthese von 7-Methoxy-3-methyl-8-(1-methylpyrazol-4-yl)-1-thiazol-2-yl-imidazo[4,5-c]chinolin-2-on (Verbindungsbeispiel 47)

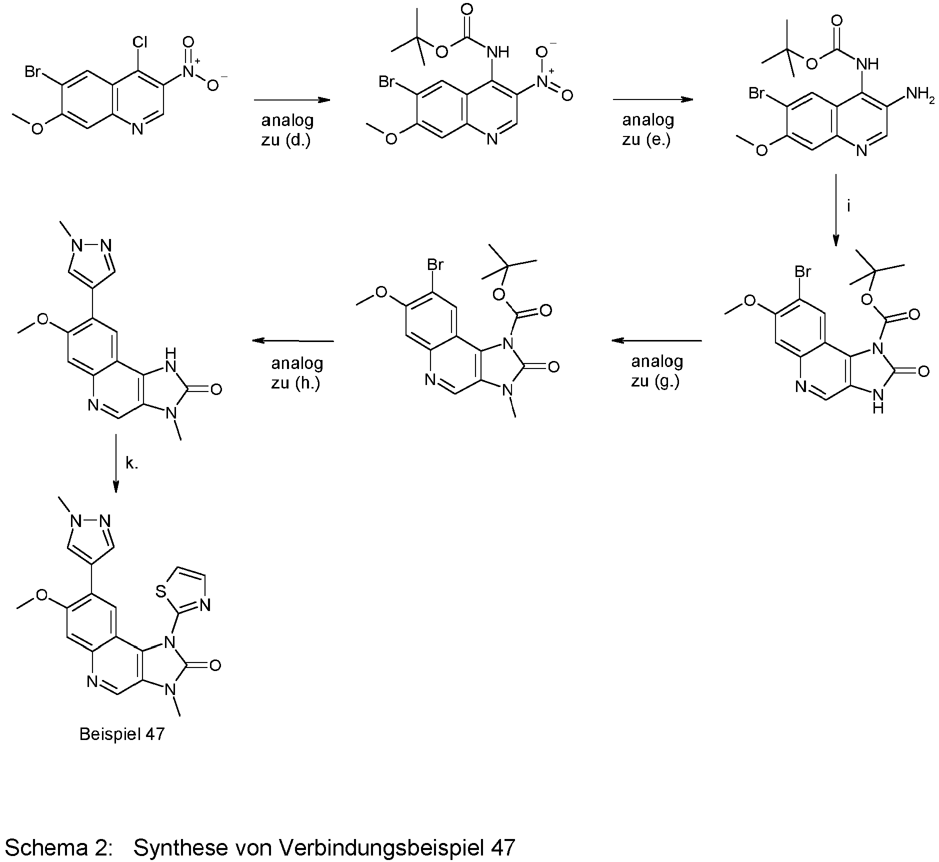

### i. Synthese von tert-Butyl 8-brom-7-methoxy-2-oxo-3H-imidazo[4,5-c]chinolin-1-carboxylat

Tert-Butyl N-(3-amino-6-brom-7-methoxychinolin-4-yl)carbamat (50,0 mg, 0,14 mmol), Ditrichlormethylcarbonat (119 mg, 0,40 mmol) und Triethylamin (0,18 mL, 1,29 mmol) wurden in Dichlormethan (3 mL) gelöst und 2 Stunden lang bei 25°C gerührt. Die Mischung wurde unter Vakuum zur Trockne eingeengt und der Rückstand an Kieselgel chromatographisch aufgereinigt (Essigsäureethylester:Methanol = 10:1, Volumenanteile). Man erhielt 50,0 mg (93 %) tert-Butyl 8-brom-7-methoxy-2-oxo-3H-imidazo[4,5-c]chinolin-1-carboxylat als farblosen Feststoff.

### k. Synthese von 7-Methoxy-3-methyl-8-(1-methylpyrazol-4-yl)-1-thiazol-2-yl-imidazo[4,5-c]chinolin-2-on

Unter Argon wurden in einem verschließbaren Reaktionsgefäß 7-Methoxy-3-methyl-8-(1-methylpyrazol-4-yl)-1H-imidazo[4,5-c]chinolin-2-on (20 mg, 0,06 mmol), 2-Brom-1,3-thiazol (20 mg, 0,12 mmol), Cul (20,00 mg, 0,11 mmol), K₃PO₄ (50 mg, 0,24 mmol) in Toluol (4 mL) aufgenommen. Anschließend wurde bei Raumtemperatur (1S,2S)-1-N,2-N-Dimethylcyclohexan-1,2-diamin (30 mg, 0,21 mmol) zugefügt. Die Mischung wurde unter Rühren 18 Stunden lang auf 100°C erhitzt. Die abgekühlte Lösung wurde unter Vakuum zur Trockne eingeengt und der Rückstand an Kieselgel chromatographisch aufgereinigt (Wasser/0,05% NH₄HCO₃ : Acetonitril = 10:1). Man erhielt 10 mg (43 %) 7-Methoxy-3-methyl-8-(1-methylpyrazol-4-yl)-1-thiazol-2-yl-imidazo[4,5-c]chinolin-2-on als farblosen Feststoff.

### BEISPIEL 3: Synthese von 1-[5-(Azetidin-3-ylmethoxy)-3-fluor-pyridin-2-yl]-7-methoxy-3-methyl-8-(1-methyl-1H-pyrazol-4-yl)-1,3-dihydro-imidazo[4,5-c]chinolin-2-on (Vergleichsbeispiel)

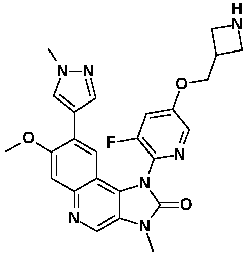

i. In einem 500 mL Rundhalskolben wurden 1-(5-Benzyloxy-3-fluor-2-pyridyl)-7-methoxy-3-methyl-8-(1-methylpyrazol-4-yl)imidazo[4,5-c]chinolin-2-on (1,1 g, 2,15 mmol), MeOH (200 mL), und Pd/C (229 mg, 2,15 mmol) vorgelegt, es wurde mit H₂ gespült und eine H₂-Atmosphäre aufrechterhalten. Die Lösung wurde 8 Stunden lang bei Raumtemperatur gerührt. Feststoff wurde abfiltriert und das Gemisch im Vakuum eingeengt. Man erhielt 740 mg (80%) 1-(3-Fluor-5-hydroxy-2-pyridyl)-7-methoxy-3-methyl-8-(1-methylpyrazol-4-yl)imidazo[4,5-c]chinolin-2-on als weißen Feststoff.
ii. In einem 30 mL Reaktionsgefäß wurden 1-(3-Fluor-5-hydroxy-2-pyridyl)-7-methoxy-3-methyl-8-(1-methylpyrazol-4-yl)imidazo[4,5-c]chinolin-2-on (100 mg, 0,23 mmol, 98%), 3-(Brom-methyl)azetidin-1-*tert*-butylcarbamat (80 mg, 0,32 mmol), Kaliumcarbonat (66,2 mg, 0,48 mmol) and *N,N*-Dimethylformamid (10 mL) vorgelegt. Die Lösung wurde bei 50°C über Nacht gerührt. Die Reaktion wurde dann durch Zugabe von 10 mL Wasser beendet. Die Lösung wurde dann dreimal mit je 10 mL Ethylacetat extrahiert, die vereinten organischen Phasen über wasserfreiem Natriumsulfat getrocknet, abfiltriert und im Vakuum eingeengt. Man erhielt 160 mg (>100%) 3-[[5-fluor-6-[7-methoxy-3-methyl-8-(1-methylpyrazol-4-yl)-2-oxo-imidazo[4,5-c]chinolin-1-yl]-3-pyridyl]oxymethyl]azetidin-1-tert-butylcarbamat als gelbes Öl.
iii. In einem 50 mL Rundkolben wurden 3-[[5-Fluor-6-[7-methoxy-3-methyl-8-(1-methylpyrazol-4-yl)-2-oxo-imidazo[4,5-c]chinolin-1-yl]-3-pyridyl]oxymethyl]azetidin-1-*tert-*butylcarbamat (160 mg, 0,27 mmol, 99%), Dichlormethan (5 mL) und Trifluoressigsäure (1 mL) vorgelegt. Die Lösung wurde bei 25°C über Nacht gerührt. Das erhaltene Rohprodukt wurde mittels präparativer HPLC aufgereinigt. Man erhielt 30 mg (21%) 1-[5-(Azetidin-3-ylmethoxy)-3-fluor-pyridin-2-yl]-7-methoxy-3-methyl-8-(1-methyl-1H-pyrazol-4-yl)-1,3-dihydro-imidazo[4,5-c]chinolin-2-on als gelben Feststoff.

### BEISPIEL 8: Synthese von 1-[3-Fluor-5-(trideuteriomethoxy)-2-pyridyl]-7-methoxy-3-methyl-8-(1-methylpyrazol-4-yl)imidazo[4,5-c]chinolin-2-on (Vergleichsbeispiel 155)

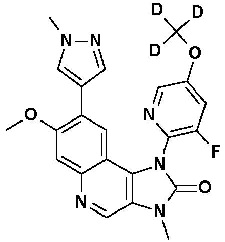

In einem 25 mL Rundkolben wurden 1-(3-Fluor-5-hydroxy-2-pyridyl)-7-methoxy-3-methyl-8-(1-methylpyrazol-4-yl)imidazo[4,5-c]chinolin-2-on (80 mg, 0,19 mmol, 98%), N,N-Dimethylformamid (5,00 mL), Kaliumcarbonat (51,7 mg, 0,37 mmol) und lod(D3)methan (57,1 mg, 0,37 mmol, 95%) vorgelegt. Die Lösung wurde 30 Minuten lang bei Raumtemperatur gerührt. Der Reaktionsfortschritt wurde mittels LCMS verfolgt (Dichlormethan/MeOH = 10:1). Die Reaktion wurde dann durch die Zugabe von 50 mL Eiswasser abgebrochen. Die Lösung wurde dreimal mit je 20 mL Ethylacetat extrahiert und die organischen Phasen vereint. Das Gemisch wurde unter Vakuum eingeengt. Man erhielt 38,5 mg (47%) 1-[3-Fluor-5-(trideuteriomethoxy)-2-pyridyl]-7-methoxy-3-methyl-8-(1-methylpyrazol-4-yl)imidazo[4,5-c]chinolin-2-on als weißen Feststoff.

### BEISPIEL 9: Synthese von 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on (Vergleichsbeispiel 123)

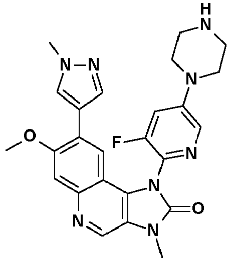

In einem verschlossenen 30 mL Reaktionsgefäß, welches mit Argon gespült und unter Argon-Inertgasatmosphäre gehalten wurde, wurden 1-(5-Chlor-3-fluor-2-pyridyl)-7-methoxy-3-methyl-8-(1-methylpyrazol-4-yl)imidazo[4,5-c]chinolin-2-on (100 mg, 0,22 mmol, 95%), Piperazin-1-*tert*-butylcarbamat (128 mg, 0,65 mmol, 95%), Pd₂(dba)₃ (21 mg, 0,02 mmol), Xantphos (27 mg, 0,05 mmol), Cs₂CO₃ (150 mg, 0,46 mmol) und Toluol (10 mL) vorgelegt. Das Gemisch wurde bei 90°C über Nacht gerührt und anschließend unter Vakuum eingeengt. Der Rückstand wurde an Kieselgel mit EtOAc/MeOH (95:5, Volumeneinheiten) gereinigt. Man erhielt 120 mg (94%) 4-[5-Fluor-6-[7-methoxy-3-methyl-8-(1-methylpyrazol-4-yl)-2-oxo-imidazo[4,5-c]chinolin-1-yl]-3-pyridyl]piperazin-1-*tert-*butylcarbamat als gelben Feststoff.

ii. In einem 25 mL Rundkolben wurden 4-[5-Fluor-6-[7-methoxy-3-methyl-8-(1-methylpyrazol-4-yl)-2-oxo-imidazo[4,5-c]chinolin-1-yl]-3-pyridyl]piperazin-1-*tert-*butylcarbamat (115 mg, 0,18 mmol, 92%), Dichlormethan (10 mL) und Trifluoressigsäure (4 mL) vorgelegt. Das Gemisch wurde 30 Minuten lang bei 0°C in einem Wasser/Eis-Bad gerührt und unter Vakuum eingeengt. Das Rohprodukt wurde mittels präparativer HPLC aufgereinigt. Man erhielt 50 mg (55%) 1-(3-Fluor-5-piperazin-1-yl-pyridin-2-yl)-7-methoxy-3-methyl-8-(1-methyl-1H-pyrazol-4-yl)-1,3-dihydro-imidazo[4,5-c]chinolin-2-on als weißen Feststoff.

### BEISPIEL 10: Synthese von 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid (Vergleichsbeispiel 138)

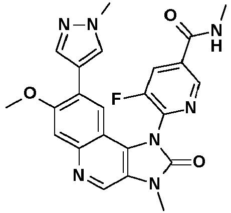

i. In einem 8 mL Reaktionsgefäß wurden eine Lösung von Schwefelsäure (2 mL, 98%) in Wasser (2 mL) und 5-Fluor-6-[7-methoxy-3-methyl-8-(1-methylpyrazol-4-yl)-2-oxo-imidazo[4,5-c]chinolin-1-yl]pyridin-3-nitril (110 mg, 0,26 mmol) vorgelegt. Das Gemisch wurde bei 80°C über Nacht gerührt. Natriumhydroxid (4 mol/L) wurde verwendet, um den pH-Wert auf 8 einzustellen. Das Gemisch wurde unter Vakuum eingeengt und der Rückstand an Kieselgel mit einer 0,5% wäßrigen NH₄HCO₃-Lösung und CH₃CN (82/18) gereinigt. Man erhielt 70 mg (61%) 5-Fluor-6-[7-methoxy-3-methyl-8-(1-methylpyrazol-4-yl)-2-oxo-imidazo[4,5-c]chinolin-1-yl]pyridin-3-carbonsäure als weißen Feststoff.
ii. In einem verschlossenen 30 mL Reaktionsgefäß wurden 5-Fluor-6-[7-methoxy-3-methyl-8-(1-methylpyrazol-4-yl)-2-oxo-imidazo[4,5-c]chinolin-1-yl]pyridin-3-carbonsäure (65,0 mg, 0,14 mmol), eine Lösung von Methylamin (10 mg, 0,32 mmol) in Tetrahydrofuran (0,5 mL), Triethylamin (44 mg, 0,43 mmol), BOP (64,9 mg, 0,15 mmol), und Tetrahydrofuran (5 mL) vorgelegt. Die Lösung wurde 18 Stunden lang bei Raumtemperatur gerührt und unter Vakuum eingeengt, der Rückstand mit 10 mL EtOAc verdünnt. Das Gemisch wurde über wasserfreiem Natriumsulfat getrocknet und unter Vakuum eingeengt. Das Rohprodukt wurde mittels präparativer HPLC aufgereinigt. Man erhielt 20 mg (30%) 5-Fluor-6-[7-methoxy-3-methyl-8-(1-methyl-1H-pyrazol-4-yl)-2-oxo-2,3-dihydro-imidazo[4,5-c]chinolin-1-yl]-N-methyl-nicotinamid als weißen Feststoff.

### BEISPIEL 11: Synthese von 1-[3-Fluor-5-(trideuteriomethoxy)-4-pyridyl]-7-methoxy-3-methyl-8-(1-methylpyrazol-4-yl)imidazo[4,5-c]chinolin-2-on (Vergleichsbeispiel 145)

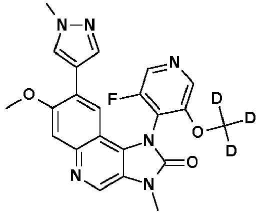

In einem verschlossenen 8 mL Reaktionsgefäß wurden 1-(3-Fluor-5-hydroxy-4-pyridyl)-7-methoxy-3-methyl-8-(1-methylpyrazol-4-yl)imidazo[4,5-c]chinolin-2-on (145 mg, 0,33 mmol, 97%), CD₃OD (0,3 mL), Kaliumcarbonat (143 mg, 1,03 mmol) und *N,N*-Dimethylformamid (3 mL) vorgelegt. Das Gemisch wurde bei 100°C über Nacht gerührt. Die Reaktion wurde dann durch Zugabe von 10 mL Wasser abgebrochen. Die Lösung wurde dreimal mit je 10 mL Ethylacetat extrahiert. Die organischen Phasen wurden vereint, über wasserfreiem Natriumsulfat getrocknet und unter Vakuum eingeengt. Das Rohprodukt wurde mittels präparativer HPLC aufgereinigt. Man erhielt 20 mg (14%) 1-[3-Fluor-5-(trideuteriomethoxy)-4-pyridyl]-7-methoxy-3-methyl-8-(1-methylpyrazol-4-yl)imidazo[4,5-c]chinolin-2-on als weißen Feststoff.

### BEISPIEL 12: Synthese von 1-(3-Fluor-5-methoxy-pyridin-4-yl)-8-(1-fluormethyl-1H-pyrazol-4-yl)-7-methoxy-3-methyl-1,3-dihydro-imidazo[4,5-c]chinolin-2-on (Vergleichsbeispiel 140)

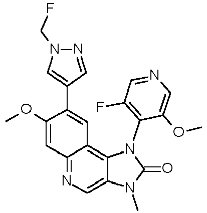

In einem verschlossenen 30 mL Reaktionsgefäß wurden 1-(3-Fluor-5-methoxy-4-pyridyl)-7-methoxy-3-methyl-8-(1H-pyrazol-4-yl)imidazo[4,5-c]chinolin-2-on (60 mg, 0,14 mmol, 97%), Kaliumcarbonat (57,6 mg, 0,42 mmol) und *N,N*-Dimethylformamid (5 mL) vorgelegt. Das Gemisch wurde 5 Minuten lang bei Raumtemperatur gerührt und auf 0°C gekühlt. Bromfluormethan (165 mg, 1.39 mmol, 95%) wurde zugegeben, das Gemisch auf Raumtemperatur erwärmt und eine Stunde lang weiter gerührt. Die Reaktion wurde dann durch die Zugabe von 50 mL Wasser abgebrochen. Die Lösung wurde dreimal mit je 30 mL Ethylacetat extrahiert, die organischen Phasen vereint und unter Vakuum eingeengt. Das Rohprodukt wurde mittels präparativer HPLC aufgereinigt. Man erhielt 5 mg (8%) 1-(3-Fluor-5-methoxy-pyridin-4-yl)-8-(1-fluormethyl-1H-pyrazol-4-yl)-7-methoxy-3-methyl-1,3-dihydro-imidazo[4,5-c]chinolin-2-on als weißen Feststoff.

Die vorstehend benutzten, im Fachbereich üblichen Abkürzungen haben die folgenden Bedeutungen: MeOH: Methanol; Pd(PPh₃)_{4:} Tetrakis(triphenylphosphin)palladium; EtOAc: Ethylacetat; BOP: Benzotriazolyloxytris(dimethylamino)-phosphonium hexafluorophosphat; Pd₂(dba)₃: Tris(dibenzylideneacetone)dipalladium

### BEISPIEL 14: Verbindungsbeispiele, die entsprechend bzw. analog zu den vorherigen Beispielen hergestellt wurden, finden sich in der nachfolgenden Tabelle 2. Die verwendeten Amin-Derivate, Boronsäureester oder Analoga sind im nachstehenden Schema 4 zusammengefasst.

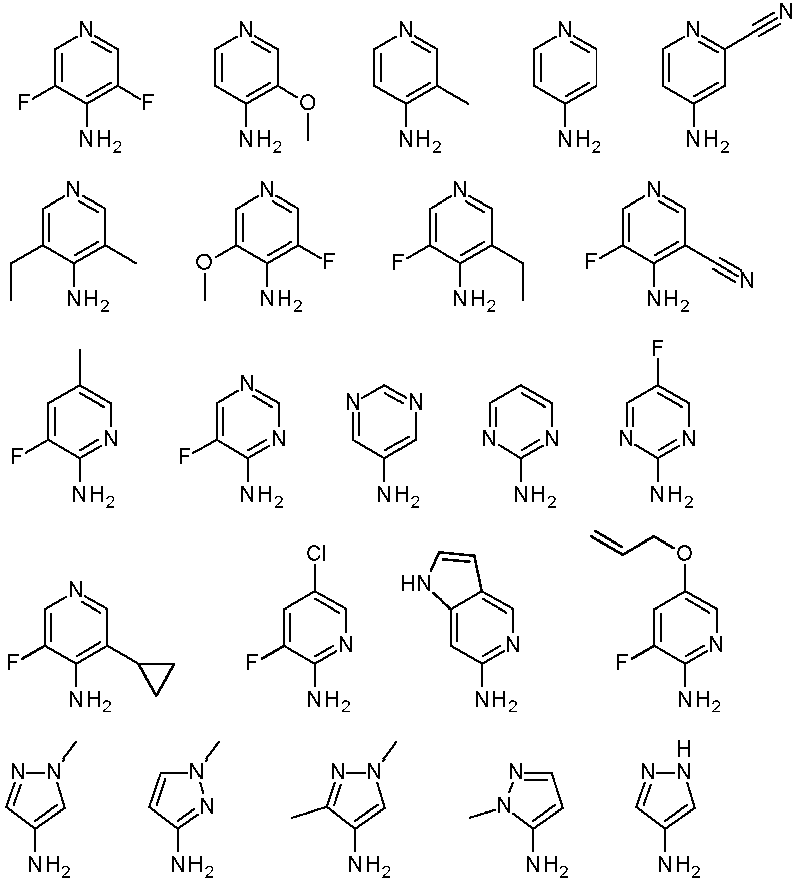

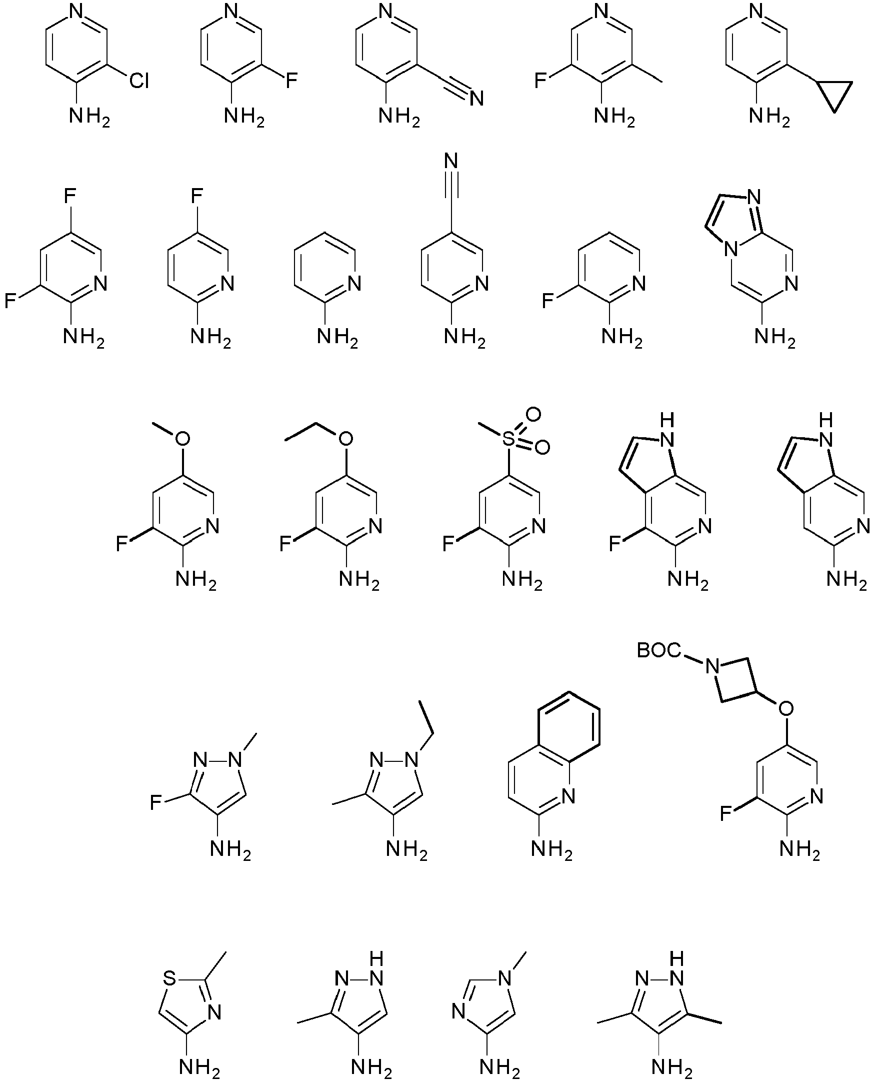

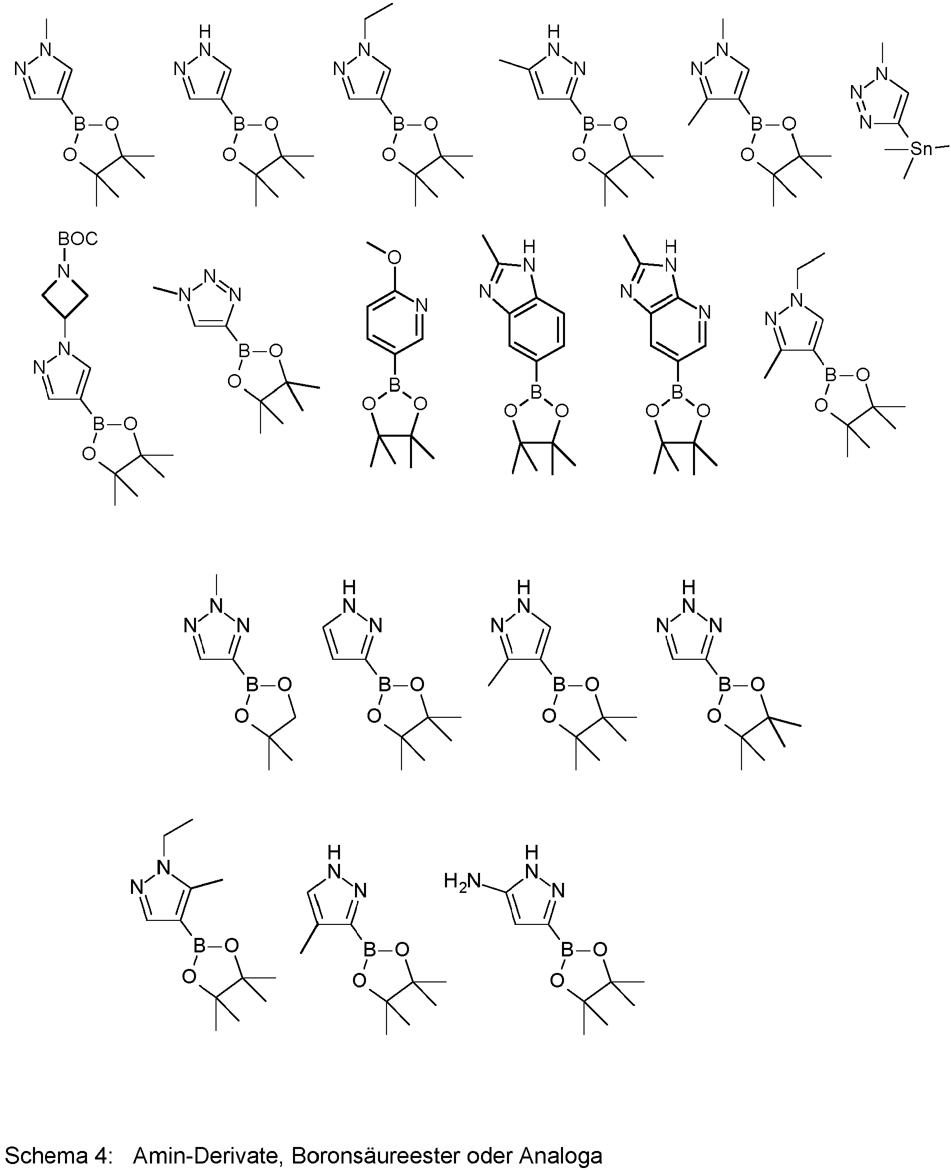

**Tabelle 2**

| Bsp. | Strukturformel | Name | IC₅₀ (pCHK2) | IC₅₀ (ATM) [µM] |
|---|---|---|---|---|
| 3 | 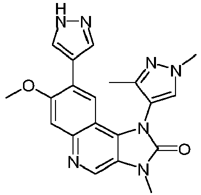 | 1-(1,3-Dimethyl-1 H-pyrazol-4-yl)-7-methoxy-3-methyl-8-(1 H-pyrazol-4-yl)-1,3-dihydro-imidazo[4,5-c] chinolin-2-on | +++ | < 0,001 |
| | MS: 390 (M+H⁺) | 1H NMR (400MHz,DMSO,ppm): 13.03(s,1H), 8.80 (s,1H), 8.13 (s,1H), 7.96 (br,1H), 7.45 (d,3H), 3.97 (m,6H), 3.53 (s,3H), 1.94 (s,3H) | | |
| 5 | 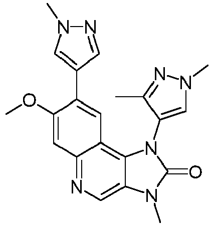 | 1-(1,3-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on | +++ | < 0,001 |
| | MS: 404 (M+H⁺) | 1H NMR (400MHz,DMSO,ppm): 8.80 (s,1H), 8.13 (s,1H), 8.00 (s,1H), 7.41(m,3H), 3.97 (m,6H), 3.87 (d,3H), 3.54 (s,3H), 1.94 (s,3H) | | |
| 10 | 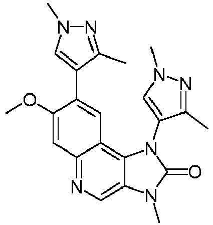 | 1,8-Bis-(1,3-dimethyl-1H-pyrazol-4-yl)-7-methoxy-3-methyl-1,3-dihydro-imidazo[4,5-c]chinolin-2-on | +++ | < 0,001 |
| | MS: 418 (M+H⁺) | 1H NMR (400MHz, DMSO, ppm): 8.82 (s,1H), 8.08 (s, 1H), 7.86 (s,1H), 7.48 (s,1H), 7.35 (s,1H), 3.92 (d,3H), 3.87 (s,3H), 3.78 (s,3H), 3.54 (s,3H), 1.90 (d,6H) | | |
| 17 | 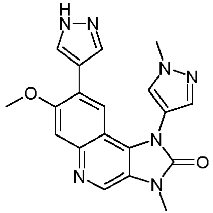 | 7-Methoxy-3-methyl-1-(1-methyl-1 H-pyrazol-4-yl)-8-(1 H-pyrazol-4-yl)-1,3-dihydro-imidazo[4,5-c] chinolin-2-on | +++ | < 0,001 |
| | MS: 375 (M+H⁺) | 1H NMR (300MHz,DMSO,ppm): 13.009 (s,1H), 8.802 (s,1H), 8.239 (s,1H), 7.818 (s,1H), 7.729 (s,2H), 7.476 (m,2H), 4.034 (s,3H), 3.987 (s,3H), 3.532 (s,3H) | | |
| 21 | 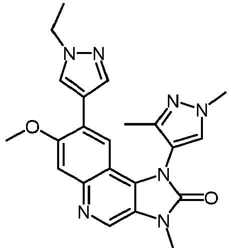 | 1-(1,3-Dimethyl-1H-pyrazol-4-yl)-8-(1-ethyl-1H-pyrazol-4-yl)-7-methoxy-3-methyl-1,3-dihydro-imidazo[4,5-c] chinolin-2-on | +++ | < 0,001 |
| | MS: 418 (M+H⁺) | 1H NMR (400MHz,DMSO,ppm): 8.81 (s,1H), 8.14 (s, 1H), 8.03 (s, 1H), 7.78-7.38 (m,3H), 4.16 (m,2H), 3.99 (d,6H), 3.55 (s,3H), 1.95 (s,3H), 1.40 (m,3H) | | |
| 23 | 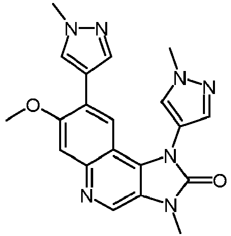 | 7-Methoxy-3-methyl-1,8-bis-(1-methyl-1 H-pyrazol-4-yl)-1,3-dihydro-imidazo[4,5-c] chinolin-2-on | +++ | < 0,01 |
| | MS: 390 (M+H⁺) | 1H NMR (300MHz,DMSO,ppm): 8.798 (s,1H), 8.241 (s,1H), 8.005 (s,1H), 7.811 (s,1H), 7.471-7.462 (d, J=2.7Hz,2H), 7.348 (s,1H), 4.037 (s,3H), 3.984 (s,3H), 3.874 (s,3H), 3.525 (s,3H) | | |
| 30 | 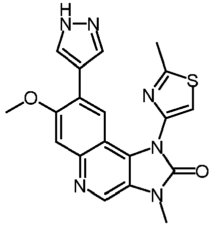 | 7-Methoxy-3-methyl-1-(2-methyl-thiazol-4-yl)-8-(1 H-pyrazol-4-yl)-1,3-dihydro-imidazo[4,5-c]chinolin-2-on | +++ | < 0,01 |
| | MS: 393 (M+H⁺) | 1H NMR (400MHz,DMSO,ppm): 12.96 (br,1H), 8.84 (s,1H), 8.00 (s,1H), 7.73 (s,2H), 7.48 (s,1H), 7.15 (s,1H), 3.99 (s,3H), 3.55 (s,3H), 2.79 (s,3H) | | |
| 37 | 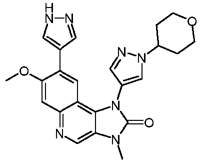 | 7-Methoxy-3-methyl-8-(1 H-pyrazol-4-yl)-1-[1-(tetrahydro-pyran-4-yl)-1 H-pyrazol-4-yl]-1,3-dihydro-imidazo[4,5-c] chinolin-2-on | +++ | < 0,01 |
| | MS: 446 (M+H⁺) | 1H NMR (400MHz,DMSO,ppm): 12.98 (br,1H), 8.79 (s,1H), 8.35 (s,1H), 7.84 (s,1H), 7.69 (s,2H), 7.44 (d,2H), 4.58 (m,1H), 4.00 (m,5H), 3.52 (m,5H), 2.07 (m,4H) | | |
| 41 | 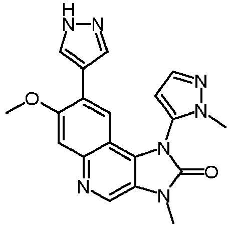 | 7-Methoxy-3-methyl-1-(2-methyl-2 H-pyrazol-3-yl)-8-(1 H-pyrazol-4-yl)-1,3-dihydro-imidazo[4,5-c] chinolin-2-on | ++ | < 0,01 |
| | MS: 376 (M+H⁺) | 1H NMR (400MHz,DMSO,ppm): 8.77 (s,1H), 7.85 (s,1H), 7.73 (s,2H), 7.50 (s,1H), 6.92 (s,1H), 6.71 (s,1H), 6.04 (s,1H), 3.99 (s,3H), 3.73 (s,3H), 3.62 (s,3H) | | |
| 42 | 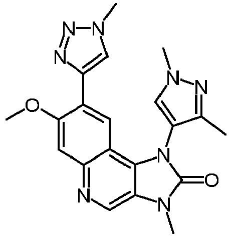 | 1-(1,3-Dimethyl-1H-pyrazol-4-yl)-7-methoxy-3-methyl-8-(1-methyl-1H-[1,2,3]triazol-4-yl)-1,3-dihydro-imidazo[4,5-c]chinolin-2-on | ++ | < 0,01 |
| | MS: 405 (M+H⁺) | 1H NMR (400MHz,DMSO,ppm): 8.85 (s,1H), 8.40 (s, 1H), 8.28 (s, 1H), 8.08 (s,1H), 7.52 (s,1H), 4.07 (s,3H), 4.01 (s,3H), 3.91 (s,3H), 3.54 (s,3H), 1.93 (s,3H) | | |
| 43 | 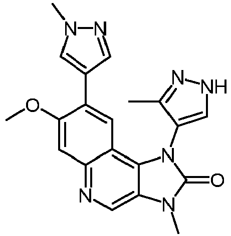 | 7-Methoxy-3-methyl-1-(3-methyl-1 H-pyrazol-4-yl)-8-(1-methyl-1 H-pyrazol-4-yl)-1,3-dihydro-imidazo[4,5-c]chinolin-2-on | ++ | < 0,001 |
| | MS: 390 (M+H⁺) | 1H NMR 1H NMR (400 MHz, DMSO): 13.21 (s, 1H), 8.80 (s, 1H), 7.98 (d, J = 25.3 Hz, 2H), 7.47 (s, 1H), 7.36 (s, 1H), 7.31 (s, 1H), 3.98 (s, 3H), 3.87 (s, 3H), 3.54 (s, 3H), 2.05 (s, 3H). | | |
| 47 | 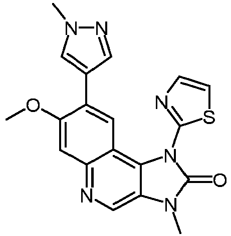 | 7-Methoxy-3-methyl-8-(1-methyl-1 H-pyrazol-4-yl)-1-thiazol-2-yl-1,3-dihydro-imidazo[4,5-c]chinolin-2-on | ++ | < 0,01 |
| | MS: 393 (M+H⁺) | 1H NMR (400HMz,DMSO,ppm): 8.82 (s,1H), 8.04 (s,2H), 7.98 (s,1H), 7.60 (s, 1H), 7.40 (d,2H), 3.94 (s,3H), 3.81 (s,3H), 3.50 (s,3H) | | |
| 49 | 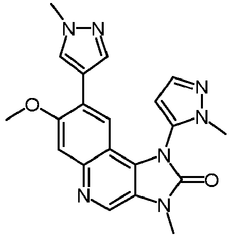 | 7-Methoxy-3-methyl-1-(2-methyl-2 H-pyrazol-3-yl)-8-(1-methyl-1 H-pyrazol-4-yl)-1,3-dihydro-imidazo[4,5-c]chinolin-2-on | ++ | < 0,01 |
| | MS: 390 (M+H⁺) | 1H NMR (300MHz,MeOD,ppm): 8.79 (s,1H), 7.90 (m,2H), 7.51 (s,2H), 7.04 (s, 1H), 6.72 (s, 1H), 4.06 (s,3H), 3.92 (s,3H), 3.73 (s,3H), 3.66 (s,3H) | | |
| 53 | 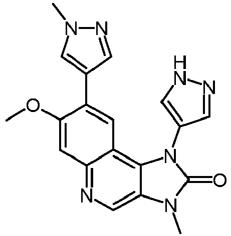 | 7-Methoxy-3-methyl-8-(1-methyl-1 H-pyrazol-4-yl)-1-(1 H-pyrazol-4-yl)-1,3-dihydro-imidazo[4,5-c]chinolin-2-on | ++ | < 0,01 |
| | MS: 376 (M+H⁺) | 1H NMR (400 MHz, DMSO): 8.80 (s, 1H), 8.03 (d, J = 20.4 Hz, 2H), 7.46 (s, 1H), 7.39 (s, 1H), 7.29 (s,1H), 3.97 (s,3H), 3.85 (s,3H), 3.53 (s,3H). | | |
| 55 | 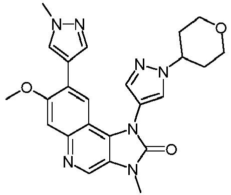 | 7-Methoxy-3-methyl-8-(1-methyl-1H-pyrazol-4-yl)-1-[1-(tetrahydro-pyran-4-yl)-1H-pyrazol-4-yl]-1,3-dihydro-imidazo[4,5-c]chinolin-2-on | ++ | < 0,01 |
| | MS: 460 (M+H⁺) | 1H NMR (300MHz,DMSO,ppm): 8.80 (s,1H), 8.35 (s, 1H), 7.96 (s, 1H), 7.83 (s, 1H), 7.40 (m,3H), 4.60 (m,1H), 3.97 (m,5H), 3.84 (s,3H), 3.53 (m,5H), 2.07 (m,4H) | | |
| 59 | 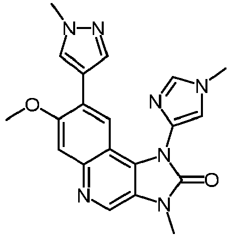 | 7-Methoxy-3-methyl-1-(1-methyl-1 H-imidazol-4-yl)-8-(1-methyl-1 H-pyrazol-4-yl)-1,3-dihydro-imidazo[4,5-c] chinolin-2-on | + | < 0,01 |
| | MS: 390 (M+H⁺) | 1H NMR (400MHz,DMSO,ppm): 8.79 (s,1H), 7.96 (1d,2H), 7.58 (s, 1H), 7.45 (s, 1H), 7.39 (d,2H), 4.00 (s,3H), 3.88 (m,6H), 3.53 (s,3H) | | |
| 102 | 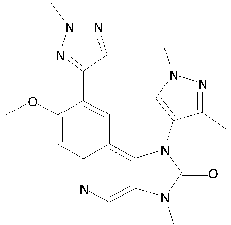 | 1-(1,3-Dimethyl-1 H-pyrazol-4-yl)-7-methoxy-3-methyl-8-(2-methyl-2H-[1,2,3]triazol-4-yl)-1,3-dihydro-imidazo[4,5-c]chinolin-2-on | +++ | < 0,05 |
| | MS: 405 (M+H⁺) | 1H NMR (400 MHz, DMSO-d6) ppm = 8.88 (s, 1H), 8.14-8.11 (s,2H), 8.04 (s, 1H), 7.54 (s, 1H), 4.19 (s,3H), 4.03-3.99 (d,6H), 3.56 (s,3H), 1.56 (s,3H) | | |
| 118 | 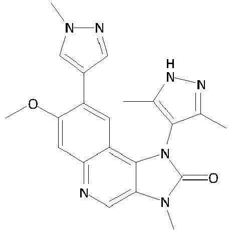 | 1-(3,5-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on | ++ | < 0,01 |
| | MS: 404 (M+H⁺) | 1H NMR (400MHz,DMSO) ppm = 12.9 (s, 1H), 8.80 (s, 1H), 8.00 (s, 1H), 7.47 (s, 1H), 7.33 (s, 1H), 7.30 (d, J=0.8Hz, 1H), 3.97 (s,3H), 3.86 (s,3H), 3.55 (s,3H), 1.98 (s,6H). | | |
| 121 | 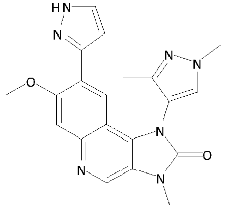 | 1-(1,3-Dimethyl-1 H-pyrazol-4-yl)-7-methoxy-3-methyl-8-(1 H-pyrazol-3-yl)-1,3-dihydro-imidazo[4,5-c]chinolin-2-on | +++ | < 0,01 |
| | MS: 390 (M+H⁺) | 1H NMR (400MHz, DMSO) ppm = 12.97 (s,1H), 8.84 (d, J=40, 1H), 8.07-7.72 (m,2H), 7.54-6.72 (m,2H), 6.15 (s, 1H), 4.00-3.92 (m,6H), 3.55 (s,3H), 1.95 (s,3H) | | |
| 130 | 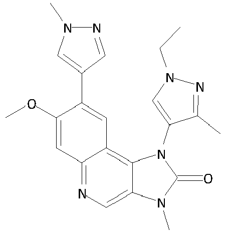 | 1-(1-Ethyl-3-methyl-1 H-pyrazol-4-yl)-7-methoxy-3-methyl-8-(1-methyl-1 H-pyrazol-4-yl)-1,3-dihydro-imidazo[4,5-c]chinolin-2-on | ++ | < 0,001 |
| | MS: 418 (M+H⁺) | 1H NMR (400MHz,DMSO) ppm = 8.83 (s, 1H), 8.19 (s, 1H), 8.01 (s, 1H), 7.49 (s, 1H), 7.43 (s, 1H), 7.36 (s, 1H), 4.30-4.20 (m,2H), 4.00 (s,3H), 3.88 (s,3H), 3.56 (s,3H), 1.96 (s,3H), 1.50 (m,3H) | | |
| 135 | 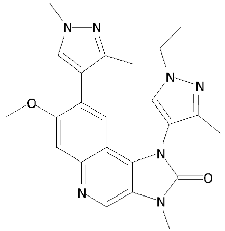 | 8-(1,3-Dimethyl-1 H-pyrazol-4-yl)-1-(1-ethyl-3-methyl-1 H-pyrazol-4-yl)-7-methoxy-3-methyl-1,3-dihydro-imidazo[4,5-c]chinolin-2-on | ++ | < 0,001 |
| | MS: 432 (M+H⁺) | 1H NMR (400 MHz, DMSO) ppm = 8.84 (s,1H), 8.13 (s, 1H), 7.82 (s, 1H), 7.49 (s, 1H), 7.32 (s, 1H), 4.15 (q, J = 7.2 Hz, 2H), 3.92 (s,3H), 3.78 (s,3H), 3.55 (s,3H), 1.91 (d, J = 15.3 Hz, 6H), 1.40 (t, J = 7.2 Hz, 3H). | | |
| 141 | 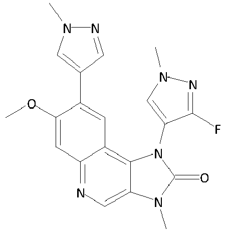 | 1-(3-Fluor-1-methyl-1 H-pyrazol-4-yl)-7-methoxy-3-methyl-8-(1-methyl-1 H-pyrazol-4-yl)-1,3-dihydro-imidazo[4,5-c]chinolin-2-on | ++ | < 0,01 |
| | MS: 408 (M+H⁺) | 1H NMR (400 MHz, DMSO) ppm = 8.82 (s,1H), 8.03 (s,1H), 7.89 (d, J = 2.4 Hz, 1H), 7.60 (s,1H), 7.49 (s,1H), 7.46 (s,1H), 3.98 (s,3H), 3.91 (s,3H), 3.86 (s,3H), 3.53 (s,3H). | | |
| 151 | 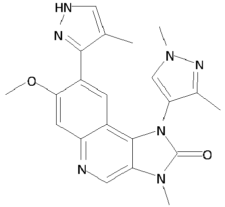 | 1-(1,3-Dimethyl-1 H-pyrazol-4-yl)-7-methoxy-3-methyl-8-(4-methyl-1 H-pyrazol-3-yl)-1,3-dihydro-imidazo[4,5-c] chinolin-2-on | +++ | < 0,05 |
| | MS: 404 (M+H⁺) | | | |

| | | | | |
|---|---|---|---|---|
| +++: 0,2 µM oder weniger ++: > 0,2 µM bis 1 µM +: > 1 µM bis 2 µM | | | | |

Erfindungsgemäße Verbindungen weisen nicht nur eine sehr gute Hemmung von ATM Kinase auf, sie sind auch zusätzlich noch sehr selektiv gegenüber anderen Kinasen, wie beispielsweise mTOR, PIK3 alpha, PI3K beta, PI3K delta und PI3K gamma, was anhand der in der nachfolgenden Tabelle 3 aufgeführten experimentellen Daten belegt wird:

**Tabelle 3**

| Bsp. | IC₅₀ (ATM) [µM] | IC50 (PI3Kalpha) [µM] | IC50 (PI3Kbeta) [µM] | IC50 (PI3Kdelta) [µM] | IC50 (PI3Kgamm a) [µM] | IC50 (mTOR) [µM] |
|---|---|---|---|---|---|---|
| 3 | < 0,001 | > 30 | > 30 | > 30 | > 30 | > 5 |
| 5 | < 0,001 | > 30 | > 30 | > 30 | > 30 | > 30 |
| 10 | < 0,001 | > 30 | > 30 | > 30 | > 30 | > 30 |
| 17 | < 0,01 | > 30 | > 30 | > 30 | > 30 | > 10 |
| 21 | < 0,001 | > 20 | > 30 | > 30 | > 300 | > 5 |
| 23 | < 0,01 | > 20 | > 30 | > 30 | > 30 | > 20 |
| 30 | < 0,01 | > 30 | > 30 | > 30 | > 30 | > 30 |
| 37 | < 0,01 | > 30 | > 30 | > 30 | > 30 | > 10 |
| 41 | < 0,01 | > 30 | > 30 | > 30 | > 30 | > 10 |
| 43 | < 0,001 | > 30 | > 30 | > 30 | > 30 | > 30 |
| 47 | < 0,01 | > 30 | > 30 | > 30 | > 30 | > 30 |
| 49 | < 0,01 | > 30 | > 30 | > 30 | > 30 | > 30 |
| 53 | < 0,01 | > 30 | > 30 | > 30 | > 30 | > 30 |
| 55 | < 0,01 | > 30 | > 30 | > 30 | > 30 | > 30 |
| 59 | < 0,01 | > 30 | > 30 | > 30 | > 30 | > 30 |

Für die übrigen Verbindungsbeispiele konnten bereits folgende vorteilhaften Werte festgestellt werden:
Bezüglich mTOR weisen die folgenden Verbindungsbeispiele einen mindestens 2000-fach höhren IC50 (ATM) auf (in Bezug auf IC50 (mTOR)), wobei bei einigen Verbindungsbeispielen das Verhältnis IC50 (ATM) : IC50(mTOR) sogar 100.000 übersteigt: 118, 121, 141.

Bezüglich PI3Kbeta weist folgendes Verbindungsbeispiel einen mindestens 2000-fach höhren IC50 (ATM) auf (in Bezug auf IC50 (PI3Kbeta)): 118.

### BEISPIEL 15: Eine weitere Verbindung, die entsprechend bzw. analog hergestellt werden kann, findet sich in der nachfolgenden Tabelle 4.

**Tabelle 4**

| | | |
|---|---|---|
| 301 | 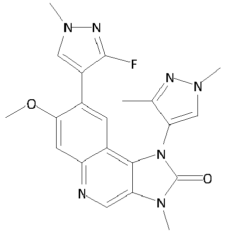 | 1-(1,3-Dimethyl-1 H-pyrazol-4-yl)-7-methoxy-3-methyl-8-(3-fluor-1-methyl-1 H-pyrazol-4-yl)-1,3-dihydro-imidazo[4,5-c]chinolin-2-on |

### BEISPIEL 16: Pharmazeutische Zusammensetzungen

### Beispiel A: Injektionsgläser

Eine Lösung von 100 g erfindungsgemäßem Wirkstoff und 5 g Dinatriumhydrogenphosphat wird in 3 I zweifach destilliertem Wasser mit 2 N Salzsäure auf pH 6,8 eingestellt, steril filtriert, in Injektionsgläser abgefüllt, unter sterilen Bedingungen lyophilisiert und steril verschlossen. Jedes Injektionsglas enthält 5 mg erfindungsgemäßen Wirkstoff.

### Beispiel B: Suppositorien

Man schmilzt ein Gemisch von 20 g erfindungsgemäßem Wirkstoff mit 100 g Sojalecithin und 1400 g Kakaobutter, gießt es in Formen und lässt es erkalten. Jedes Suppositorium enthält 20 mg erfindungsgemäßen Wirkstoff.

### Beispiel C: Lösung

Man bereitet eine Lösung aus 1 g erfindungsgemäßem Wirkstoff, 9,38 g NaH₂PO₄*2 H₂O, 28,48 g Na₂HPO₄*12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg erfindungsgemäßen Wirkstoff mit 99,5 g Vaseline unter aseptischen Bedingungen.

### Beispiel E: Tabletten

Ein Gemisch von 1 kg erfindungsgemäßem Wirkstoff, 4 kg Laktose, 1,2 kg Kartoffelstärke, 0,2 kg Talk und 0,1 kg Magnesiumstearat wird in üblicher Weise zu Tabletten verpresst, derart, dass jede Tablette 10 mg erfindungsgemäßen Wirkstoff enthält

### Beispiel F: Dragees

Analog Beispiel E werden Tabletten gepresst, die danach in üblicher Weise mit einem Überzug aus Saccharose, Kartoffelstärke, Talk, Tragant und Farbstoff überzogen werden.

### Beispiel G: Kapseln

2 kg erfindungsgemäßer Wirkstoff werden in üblicher Weise in Hartgelatinekapseln gefüllt, so dass jede Kapsel 20 mg erfindungsgemäßen Wirkstoff enthält.

### Beispiel H: Ampullen

Eine Lösung von 1 kg erfindungsgemäßem Wirkstoff in 60 I zweifach destilliertem Wasser wird steril filtriert, in Ampullen abgefüllt, unter sterilen Bedingungen lyophilisiert und steril verschlossen. Jede Ampulle enthält10 mg erfindungsgemäßen Wirkstoff.

### Beispiel I: Inhalations-Spray

Man löst 14 g erfindungsgemäßen Wirkstoff in 10 l isotonischer NaCI-Lösung und füllt die Lösung in handelsübliche Sprühgefäße mit Pump-Mechanismus. Die Lösung kann in Mund oder Nase gesprüht werden. Ein Sprühstoß (ca. 0,1 ml) entspricht einer Dosis von ca. 0,14 mg.

## Patentansprüche

1. Verbindung der Formel (I) 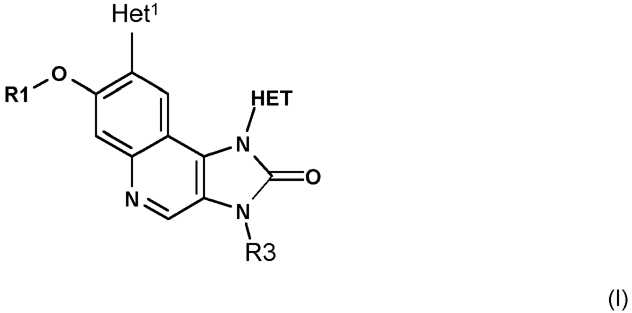 wobei
R1 Methyl ist,
R3 unverzweigtes oder verzweigtes Alkyl mit 1, 2, oder 3 C-Atomen bedeutet, wobei unabhängig voneinander 1, 2, 3, 4, oder 5 H-Atome durch Hal ersetzt sein können,
Het¹ ausgewählt ist aus Pyrazolyl, Triazolyl und Imidazolyl, wobei Het¹ unsubstituiert oder mit einem oder zwei Substituenten substituiert ist, die unabhängig voneinander ausgewählt sind aus Alkyl, welches unsubstituiert oder ein- oder mehrfach durch Halogen substituiert sein kann, -OY, -NYY, Hal, und -Het²,
HET einen 5-gliedrigen einkernigen aromatischen Heterozyklus bedeutet, wobei dieser Heterozyklus über ein Ring-C-Atom mit dem N-Atom des Grundgerüsts verknüpft ist, wobei dieser Heterozyklus ausgewählt ist aus Pyrazolyl, Thiazolyl, und Imidazolyl, und unsubstituiert oder durch einen, zwei oder drei Substituenten substituiert sein kann, die unabhängig voneinander ausgewählt sind aus der Gruppe bestehend aus: Hal, A, Het², -(CY₂)ₚ-OY, -(CY₂)ₚ-OZ, -(CY₂)ₚ-O-Het² -(CY₂)ₚ-O-(CY₂)ₜ-Het²,-(CY₂)ₚ-O-(CY₂)ₜ-NYY, -(CY₂)ₚ-O-(CY₂)ₜ-OY, -(CY₂)ₚ-NYY, -(CY₂)ₚ-COOY,-(CY₂)ₚ-CO-NYY, und -(CY₂)ₚ-NY-COY,
A jeweils unabhängig unverzweigtes oder verzweigtes Alkyl mit 1, 2, 3, 4, 5, 6, 7, 8, 9 oder 10 C-Atomen bedeutet, wobei unabhängig voneinander 1, 2, 3, 4, 5, 6 oder 7 H-Atome durch Hal ersetzt sein können,
Het² einen einkernigen gesättigten Heterozyklus mit 2, 3, 4, 5, 6 oder 7 C-Atomen und 1, 2, 3 oder 4 N-, O- und/oder S-Atomen bedeutet, der unsubstituiert oder einfach durch A substituiert sein kann,
Y H oder A bedeutet,
Z unverzweigtes oder verzweigtes Alkenyl mit 2, 3, 4, 5, 6, 7, 8, 9 oder 10 C-Atomen bedeutet, wobei unabhängig voneinander 1, 2, 3, 4, 5, 6 oder 7 H-Atome durch Hal ersetzt sein können,
Hal F, Cl, Br oder I bedeutet, und
p, 0, 1, 2, 3, 4, 5 oder 6
t 1, 2, 3, 4, 5 oder 6 bedeuten,
und/oder pharmazeutisch verwendbares Salz, Solvat, Tautomer, Stereoisomer davon.

2. Verbindung der Formel (I) nach Anspruch 1, wobei HET substituiert sein kann durch einen, zwei, oder drei Substituenten, die unabhängig voneinander ausgewählt sind aus der Gruppe bestehend aus: F, Cl, Methyl, Ethyl, Propyl, Isopropyl, Methoxy, Ethoxy, Propoxy, Piperazinyl, Tetrahydropyranyl, 2-Methoxyethoxy, 2-Hydroxyethoxy, Fluormethoxy, Difluormethoxy, N-Methylcarbamoyl (-C(=O)-NH-CH₃), 2-Methylamino-ethoxy, 1-Methyl-azetidin-3-ylmethoxy, Trideuteriomethoxy, Trifluormethoxy, Allyloxy, und Azetidinyloxy, und/oder pharmazeutisch verwendbares Salz, Solvat, Tautomer, Stereoisomer davon.

3. Verbindung der Formel (I) nach Anspruch 1 oder 2, wobei HET ausgewählt ist aus: 1H-Pyrazol-4-yl, 1-Ethyl-3-methyl-1H-pyrazol-4-yl, 1,2-Dimethyl-1H-pyrazol-4-yl, 1,3-Dimethyl-1H-pyrazol-4-yl, 1-Methyl-1H-pyrazol-4-yl, 1-(Tetrahydropyran-4-yl)-1H-pyrazol-4-yl, 2-Methyl-2H-pyrazol-3-yl, 3-Methyl-1H-pyrazol-4-yl, 2-Methyl-thiazol-4-yl, 3,5-Dimethyl-1H-pyrazol-4-yl, 3-Fluor-1-methyl-pyrazol-4-yl, Thiazol-2-yl, und 1-Methyl-1H-imidazolyl, und/oder pharmazeutisch verwendbares Salz, Solvat, Tautomer, Stereoisomer davon.

4. Verbindung der Formel (I) nach Anspruch 1, 2 oder 3, wobei Het¹ Pyrazolyl ist, welches unsubstituiert oder wie in Anspruch 1 definiert substituiert sein kann, und/oder pharmazeutisch verwendbares Salz, Solvat, Tautomer, Stereoisomer davon.

5. Verbindung der Formel (I) nach Anspruch 1, 2 oder 3, wobei Het¹ Triazolyl ist, bevorzugt [1,2,3]Triazolyl, welches unsubstituiert oder wie in Anspruch 1 oder 2 definiert substituiert sein kann, und/oder pharmazeutisch verwendbares Salz, Solvat, Tautomer, Stereoisomer davon.

6. Verbindung der Formel (I) nach einem der Ansprüche 1 bis 4, wobei Het¹ unsubstituiert oder mit einem oder zwei Substituenten substituiert ist, die unabhängig voneinander ausgewählt sind Methyl, Ethyl, Amino, Methoxy, Fluormethyl, Difluormethyl, Fluor, Azetidinyl, und/oder pharmazeutisch verwendbares Salz, Solvat, Tautomer, Stereoisomer davon.

7. Verbindung der Formel (I) nach einem der Ansprüche 1 bis 4, wobei Het¹ ausgewählt ist aus: 1H-Pyrazol-4-yl, 2H-Pyrazol-3-yl, 1H-Pyrazol-3-yl, 1-Methyl-1H-pyrazol-4-yl, 3-Methyl-1H-pyrazol-4-yl, 5-Methyl-1H-pyrazol-3-yl, 4-Methyl-1H-pyrazol-3-yl, 1-Fluormethyl-1H-pyrazol-4-yl, 1-Difluormethyl-1H-pyrazol-4-yl, 1,3-Dimethyl-1H-pyrazol-4-yl, 1-Ethyl-1H-pyrazol-4-yl, 1-Ethyl-3-methyl-1H-pyrazolyl, 3-Fluor-1-methyl-1H-pyrazol-4-yl, 3-Amino-1H-pyrazol-5-yl, 2H-[1,2,3]Triazol-4-yl, 3H-[1,2,3]Triazol-4-yl-, 1-Methyl-1H-[1,2,3]triazol-4-yl, 2-Methyl-2H-[1,2,3]triazol-4-yl, 2-Amino-1H-imidazol-4-yl, und 1-(Azetidin-3-yl)-3-methyl-1H-pyrazol-4-yl, und/oder pharmazeutisch verwendbares Salz, Solvat, Tautomer, Stereoisomer davon.

8. Verbindung der Formel (I) nach einem der Ansprüche 1 bis 7, wobei R3 Methyl bedeutet, und/oder pharmazeutisch verwendbares Salz, Solvat, Tautomer, Stereoisomer davon.

9. Verbindung nach Anspruch 1, ausgewählt aus:
| | |
|---|---|
| 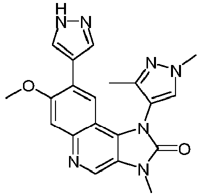 | 1-(1,3-Dimethyl-1 H-pyrazol-4-yl)-7-methoxy-3-methyl-8-(1 H-pyrazol-4-yl)-1,3-dihydro-imidazo[4,5-c]chinolin-2-on |
| 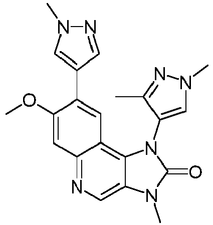 | 1-(1,3-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on |
| 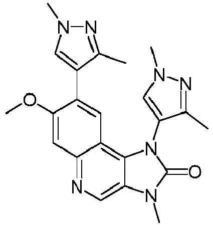 | 1,8-Bis-(1,3-dimethyl-1 H-pyrazol-4-yl)-7-methoxy-3-methyl-1,3-dihydro-imidazo[4,5-c]chinolin-2-on |
| 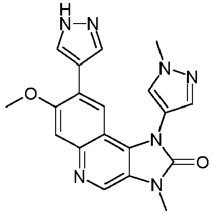 | 7-Methoxy-3-methyl-1-(1-methyl-1 H-pyrazol-4-yl)-8-(1 H-pyrazol-4-yl)-1,3-dihydro-imidazo[4,5-c]chinolin-2-on |
| 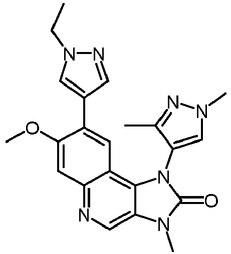 | 1-(1,3-Dimethyl-1 H-pyrazol-4-yl)-8-(1-ethyl-1 H-pyrazol-4-yl)-7-methoxy-3-methyl-1,3-dihydro-imidazo[4,5-c]chinolin-2-on |
| 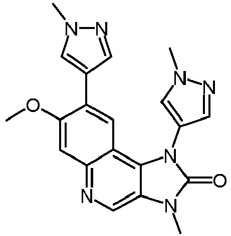 | 7 -Methoxy-3-methyl-1 , 8-bis-(1-methyl-1 H-pyrazol-4-yl)-1,3-dihydro-imidazo[4,5-c]chinolin-2-on |
| 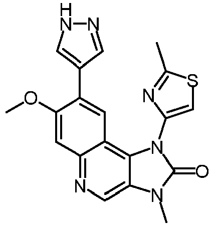 | 7-Methoxy-3-methyl-1-(2-methyl-thiazol-4-yl)-8-(1 H-pyrazol-4-yl)-1 ,3-dihydro-imidazo[4,5-c]chinolin-2-on |
| 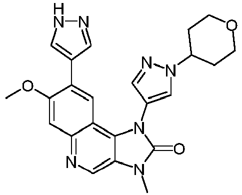 | 7-Methoxy-3-methyl-8-(1 H-pyrazol-4-yl)-1-[1-(tetrahydro-pyran-4-yl)-1 H-pyrazol-4-yl]-1,3-dihydro-imidazo[4,5-c]chinolin-2-on |
| 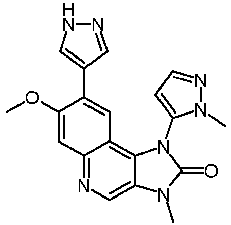 | 7-Methoxy-3-methyl-1-(2-methyl-2H-pyrazol-3-yl)-8-(1 H-pyrazol-4-yl)-1,3-dihydro-imidazo[4,5-c]chinolin-2-on |
| 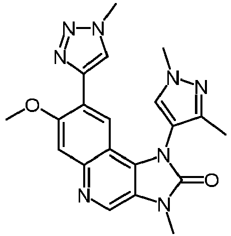 | 1-(1,3-Dimethyl-1 H-pyrazol-4-yl)-7-methoxy-3-methyl-8-(1-methyl-1 H-[1,2,3]triazol-4-yl)-1,3-dihydro-imidazo[4,5-c]chinolin-2-on |
| 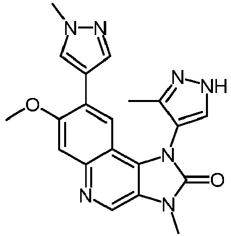 | 7-Methoxy-3-methyl-1-(3-methyl-1 H-pyrazol-4-yl)-8-(1-methyl-1 H-pyrazol-4-yl)-1,3-dihydro-imidazo[4,5-c]chinolin-2-on |
| 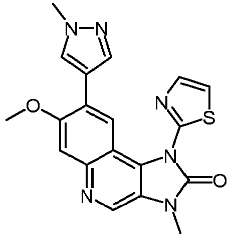 | 7-Methoxy-3-methyl-8-(1-methyl-1 H-pyrazol-4-yl)-1-thiazol-2-yl-1,3-dihydro-imidazo[4,5-c]chinolin-2-on |
| 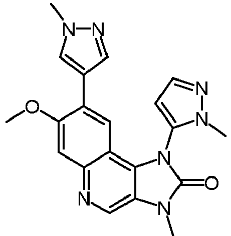 | 7-Methoxy-3-methyl-1-(2-methyl-2H-pyrazol-3-yl)-8-(1-methyl-1H-pyrazol-4-yl)-1,3-dihydro-imidazo[4,5-c]chinolin-2-on |
| 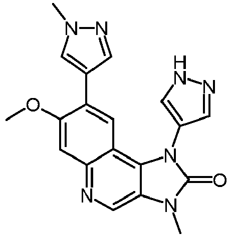 | 7-Methoxy-3-methyl-8-(1-methyl-1 H-pyrazol-4-yl)-1-( 1 H-pyrazol-4-yl)-1 ,3-dihydro-imidazo[4,5-c]chinolin-2-on |
| 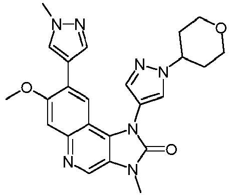 | 7-Methoxy-3-methyl-8-(1-methyl-1 H-pyrazol-4-yl)-1-[1-(tetrahydro-pyran-4-yl)-1 H-pyrazol-4-yl]-1,3-dihydro-imidazo[4,5-c]chinolin-2-on |
| 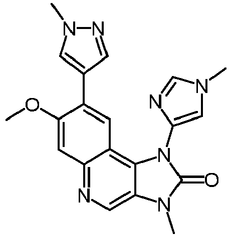 | 7-Methoxy-3-methyl-1-(1-methyl-1 H-imidazol-4-yl)-8-(1-methyl-1 H-pyrazol-4-yl)-1,3-dihydro-imidazo[4,5-c]chinolin-2-on |
| 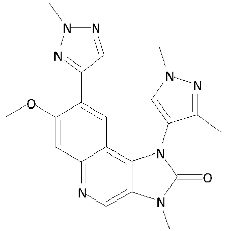 | 1-(1,3-Dimethyl-1 H-pyrazol-4-yl)-7-methoxy-3-methyl-8-(2-methyl-2H-[1,2,3]triazol-4-yl)-1,3-dihydro-imidazo[4,5-c]chinolin-2-on |
| 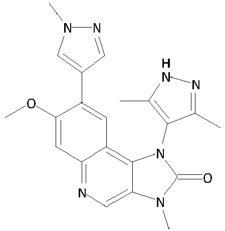 | 1-(3,5-Dimethyl-1 H-pyrazol-4-yl)-7-methoxy-3-methyl-8-(1-methyl-1 H-pyrazol-4-yl)-1,3-dihydro-imidazo[4,5-c]chinolin-2-on |
| 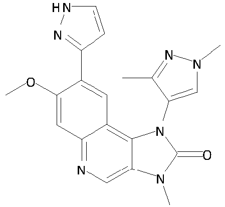 | 1-(1,3-Dimethyl-1 H-pyrazol-4-yl)-7-methoxy-3-methyl-8-(1 H-pyrazol-3-yl)-1,3-dihydro-imidazo[4,5-c]chinolin-2-on |
| 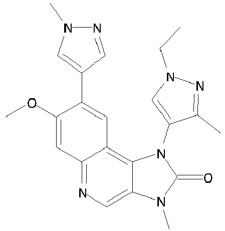 | 1-(1-Ethyl-3-methyl-1 H-pyrazol-4-yl)-7-methoxy-3-methyl-8-(1-methyl-1 H-pyrazol-4-yl)-1,3-dihydro-imidazo[4,5-c]chinolin-2-on |
| 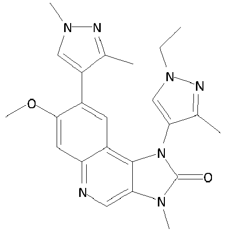 | 8-(1,3-Dimethyl-1 H-pyrazol-4-yl)-1-(1-ethyl-3-methyl-1 H-pyrazol-4-yl)-7-methoxy-3-methyl-1,3-dihydro-imidazo[4,5-c]chinolin-2-on |
| 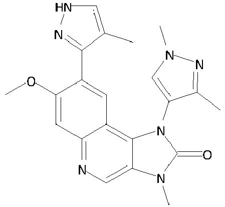 | 1-(1,3-Dimethyl-1 H-pyrazol-4-yl)-7-methoxy-3-methyl-8-(4-methyl-1 H-pyrazol-3-yl)-1,3-dihydro-imidazo[4,5-c] chinolin-2-on |
| 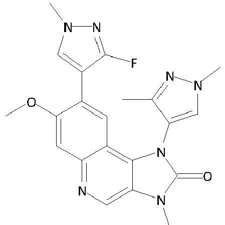 | 1-(1,3-Dimethyl-1 H-pyrazol-4-yl)-7-methoxy-3-methyl-8-(3-fluor-1-methyl-1 H-pyrazol-4-yl)-1,3-dihydro-imidazo[4,5-c]chinolin-2-on |
und/oder pharmazeutisch verwendbares Salz, Solvat, Tautomer, Stereoisomer davon.

10. Verbindung nach einem der Ansprüche 1 bis 9 und/oder pharmazeutisch verwendbares Salz, Solvat, Tautomer, oder Stereoisomer davon, zur Verwendung als Medikament.

11. Verbindung nach einem der Ansprüche 1 bis 9 und/oder pharmazeutisch verwendbares Salz, Solvat, Tautomer, oder Stereoisomer davon, zur Verwendung bei der Behandlung von Krebs, Tumoren und/oder Metastasen, optional in Kombination mit Radiotherapie oder mindestens einem Antikrebsmittel.

12. Verbindung nach einem der Ansprüche 1 bis 9 und/oder pharmazeutisch verwendbares Salz, Solvat, Tautomer, oder Stereoisomer davon, zur Verwendung in der Sensibilisierung von Krebszellen gegenüber einem Antikrebsmittel und/oder ionisierender Strahlung.

13. Pharmazeutische Zusammensetzung, enthaltend eine wirksame Menge mindestens einer Verbindung nach einem der Ansprüche 1 bis 9 und/oder einem pharmazeutisch verwendbaren Salz, Solvat, Tautomer, oder Stereoisomer davon, optional zusammen mit mindestens einem pharmazeutisch verträglichen Hilfsstoff.

14. Kit, bestehend aus getrennten Packungen von
(a) einer wirksamen Menge einer Verbindung nach einem der Ansprüche 1 bis 9 und/oder pharmazeutisch verwendbares Salz, Solvat, Tautomer, oder Stereoisomer davon, und
(b) einer wirksamen Menge eines Antikrebsmittels.

## Claims

1. Compound of the formula (I) 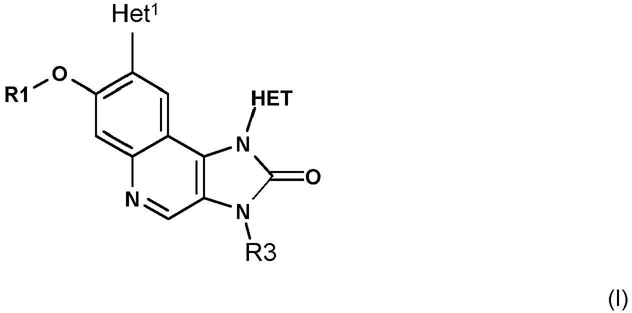 where
R1 is methyl,
R3 denotes unbranched or branched alkyl having 1, 2 or 3 C atoms, where, independently of one another, 1, 2, 3, 4 or 5 H atoms may be replaced by Hal,
Het¹ is selected from pyrazolyl, triazolyl and imidazolyl, wherein Het¹ may be unsubstituted or mono- or disubstituted, independently of one another, by alkyl, which may be unsubstituted or mono- or polysubstituted by Hal, -OY, -NYY, Hal and -Het²,
HET denotes a 5-membered monocyclic aromatic heterocycle, where this heterocycle is linked to the N atom of the skeleton via the ring C atom and where this heterocycle is selected from pyrazolyl, thiazolyl and imidazolyl and may be unsubstituted or substituted by one, two or three substituents, which are selected, independently of one another, from the group consisting of: Hal, A, Het², -(CY₂)ₚ-OY, -(CY₂)ₚ-OZ, -(CY₂)ₚ-O-Het², -(CY₂)ₚ-O-(CY₂)ₜ-Het², -(CY₂)ₚ-O-(CY₂)ₜ-NYY, -(CY₂)ₚ-O-(CY₂)ₜ-OY, -(CY₂)ₚ-NYY, -(CY₂)ₚ-COOY, -(CY₂)ₚ-CO-NYY, -(CY₂)ₚ-NY-COY,
A in each case independently denotes unbranched or branched alkyl having 1, 2, 3, 4, 5, 6, 7, 8, 9 or 10 C atoms, where, independently of one another, 1, 2, 3, 4, 5, 6 or 7 H atoms may be replaced by Hal,
Het² denotes a monocyclic saturated heterocycle having 2, 3, 4, 5, 6 or 7 C atoms and 1, 2, 3 or 4 N, O and/or S atoms, which may be unsubstituted or monosubstituted by A,
Y denotes H or A,
Z denotes unbranched or branched alkenyl having 2, 3, 4, 5, 6, 7, 8, 9 or 10 C atoms, where, independently of one another, 1, 2, 3, 4, 5, 6 or 7 H atoms may be replaced by Hal,
Hal denotes F, Cl, Br or I, and
p denotes 0, 1, 2, 3, 4, 5 or 6
t denotes 1, 2, 3, 4, 5 or 6,
and/or pharmaceutically usable salt, solvate, tautomer or stereoisomer thereof.

2. Compound of the formula (I) according to Claim 1, where HET may be substituted by one, two or three substituents which are selected, independently of one another, from the group consisting of: F, Cl, methyl, ethyl, propyl, isopropyl, methoxy, ethoxy, propoxy, piperazinyl, tetrahydropyranyl, 2-methoxyethoxy, 2-hydroxyethoxy, fluoromethoxy, difluoromethoxy, N-methylcarbamoyl (-C(=O)-NH-CH₃), 2-methylaminoethoxy, 1-methyl-azetidin-3-ylmethoxy, trideuteriomethoxy, trifluoromethoxy, allyloxy and azetidinyloxy, and/or pharmaceutically usable salt, solvate, tautomer or stereoisomer thereof.

3. Compound of the formula (I) according to Claim 1 or 2, where HET is selected from: 1H-pyrazol-4-yl, 1-ethyl-3-methyl-1H-pyrazol-4-yl, 1,2-dimethyl-1H-pyrazol-4-yl, 1,3-dimethyl-1H-pyrazol-4-yl, 1-methyl-1 H-pyrazol-4-yl, 1-(tetrahydropyran-4-yl)-1H-pyrazol-4-yl, 2-methyl-2H-pyrazol-3-yl, 3-methyl-1H-pyrazol-4-yl, 2-methylthiazol-4-yl, 3,5-dimethyl-1H-pyrazol-4-yl, 3-fluoro-1-methylpyrazol-4-yl, thiazol-2-yl, and 1-methyl-1H-imidazolyl, and/or pharmaceutically usable salt, solvate, tautomer or stereoisomer thereof.

4. Compound of the formula (I) according to Claim 1, 2 or 3, where Het¹ is pyrazolyl, which may be unsubstituted or substituted as defined in Claim 1, and/or pharmaceutically usable salt, solvate, tautomer or stereoisomer thereof.

5. Compound of the formula (I) according to Claim 1, 2 or 3, where Het¹ is triazolyl, preferably 1,2,3-triazolyl, which may be unsubstituted or substituted as defined in Claim 1, and/or pharmaceutically usable salt, solvate, tautomer or stereoisomer thereof.

6. Compound of the formula (I) according to one of Claims 1 to 4, where Het¹ is unsubstituted or substituted by one or two substituents selected, independently of one another, from methyl, ethyl, amino, methoxy, fluoromethyl, difluoromethyl, fluorine, azetidinyl, and/or pharmaceutically usable salt, solvate, tautomer or stereoisomer thereof.

7. Compound of the formula (I) according to any of Claims 1 to 4, where Het¹ is selected from: 1H-pyrazol-4-yl, 2H-pyrazol-3-yl, 1H-pyrazol-3-yl, 1-methyl-1H-pyrazol-4-yl, 3-methyl-1H-pyrazol-4-yl, 5-methyl-1H-pyrazol-3-yl, 4-methyl-1H-pyrazol-3-yl, 1-fluoromethyl-1H-pyrazol-4-yl, 1-difluoromethyl-1H-pyrazol-4-yl, 1,3-dimethyl-1H-pyrazol-4-yl, 1-ethyl-1H-pyrazol-4-yl, 1-ethyl-3-methyl-1H-pyrazolyl, 3-fluoro-1-methyl-1H-pyrazol-4-yl, 3-amino-1H-pyrazol-5-yl, 2H-1,2,3-triazol-4-yl, 3H-1,2,3-triazol-4-yl-, 1-methyl-1H-1,2,3-triazol-4-yl, 2-methyl-2H-1,2,3-triazol-4-yl, 2-amino-1H-imidazol-4-yl, and 1-(azetidin-3-yl)-3-methyl-1H-pyrazol-4-yl, and/or pharmaceutically usable salt, solvate, tautomer or stereoisomer thereof.

8. Compound of the formula (I) according to any of Claims 1 to 7, where R3 denotes methyl, and/or pharmaceutically usable salt, solvate, tautomer or stereoisomer thereof.

9. Compound according to Claim 1, selected from:
| | |
|---|---|
| 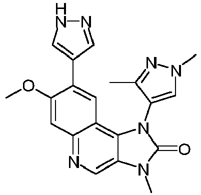 | 1-(1,3-Dimethyl-1 H-pyrazol-4-yl)-7-methoxy-3-methyl-8-(1 H-pyrazol-4-yl)-1,3-dihydro-imidazo[4,5-c]quinolin-2-one |
| 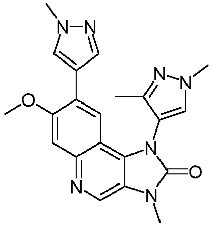 | 1-(1,3-Dimethyl-1 H-pyrazol-4-yl)-7-methoxy-3-methyl-8-(1-methyl-1 H-pyrazol-4-yl)-1 ,3-dihydroimidazo[4,5-c]quinolin-2-one |
| 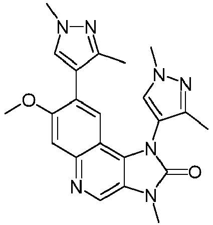 | 1,8-Bis-(1,3-dimethyl-1 H-pyrazol-4-yl)-7-methoxy-3-methyl-1,3-dihydroimidazo-[4,5-c]quinolin-2-one |
| 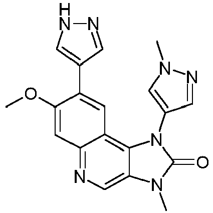 | 7-Methoxy-3-methyl-1-(1-methyl-1H-pyrazol-4-yl)-8-(1 H-pyrazol-4-yl)-1,3-dihydroimidazo[4,5-c]quinolin-2-one |
| 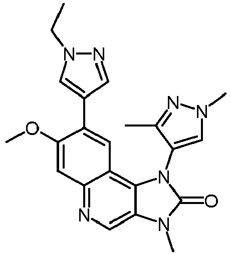 | 1-(1,3-Dimethyl-1 H-pyrazol-4-yl)-8-(1-ethyl-1 H-pyrazol-4-yl)-7 -methoxy-3-methyl-1 ,3-dihydroimidazo[4,5-c]quinolin-2-one |
| 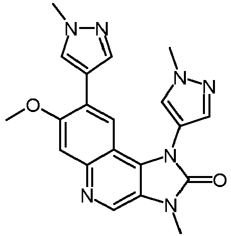 | 7-Methoxy-3-methyl-1,8-bis-(1-methyl-1 H-pyrazol-4-yl)-1,3-dihydroimidazo[4,5-c]-quinolin-2-one |
| 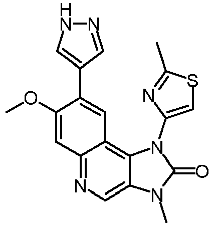 | 7-Methoxy-3-methyl-1-(2-methylthiazol-4-yl)-8-(1 H-pyrazol-4-yl)-1,3-dihydroimidazo-[4,5-c]quinolin-2-one |
| 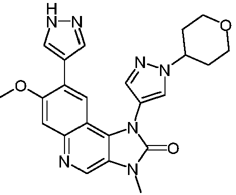 | 7-Methoxy-3-methyl-8-(1H-pyrazol-4-yl)-1-[1-(tetrahydropyran-4-yl)-1H-pyrazol-4-yl]-1,3-dihydroimidazo[4,5-c]quinolin-2-one |
| 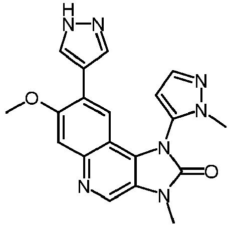 | 7-Methoxy-3-methyl-1-(2-methyl-2H-pyrazol-3-yl)-8-(1H-pyrazol-4-yl)-1,3-dihydroimidazo[4,5-c]quinolin-2-one |
| 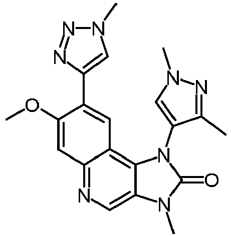 | 1-(1,3-Dimethyl-1 H-pyrazol-4-yl)-7-methoxy-3-methyl-8-(1-methyl-1 H-1,2, 3-triazol-4-yl)-1,3-dihydroimidazo[4,5-c]quinolin-2-one |
| 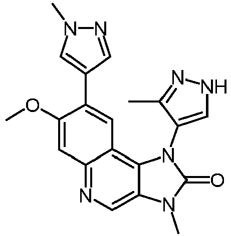 | 7-Methoxy-3-methyl-1-(3-methyl-1 H-pyrazol-4-yl)-8-(1-methyl-1 H-pyrazol-4-yl)-1,3-dihydroimidazo[4,5-c]quinolin-2-one |
| 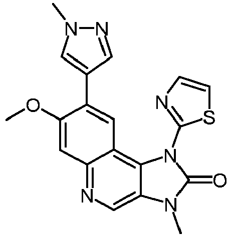 | 7-Methoxy-3-methyl-8-(1-methyl-1H-pyrazol-4-yl)-1-thiazol-2-yl-1,3-dihydro-imidazo[4,5-c]quinolin-2-one |
| 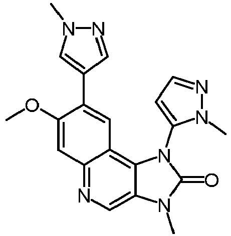 | 7-Methoxy-3-methyl-1-(2-methyl-2H-pyrazol-3-yl)-8-(1-methyl-1H-pyrazol-4-yl)-1,3-dihydroimidazo[4,5-c]quinolin-2-one |
| 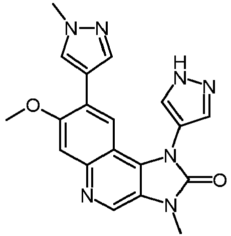 | 7-Methoxy-3-methyl-8-(1-methyl-1 H-pyrazol-4-yl)-1-( 1 H-pyrazol-4-yl)-1 ,3-dihydroimidazo[4,5-c]quinolin-2-one |
| 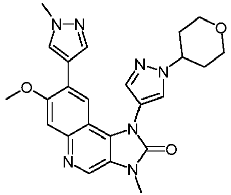 | 7-Methoxy-3-methyl-8-(1-methyl-1 H-pyrazol-4-yl)-1-[1-(tetrahydropyran-4-yl)-1 H-pyrazol-4-yl]-1,3-dihydroimidazo[4,5-c]-quinolin-2-one |
| 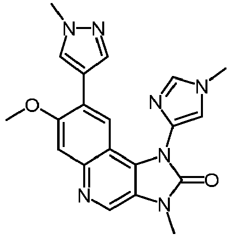 | 7-Methoxy-3-methyl-1-(1-methyl-1H-imidazol-4-yl)-8-(1-methyl-1 H-pyrazol-4-yl)-1,3-dihydroimidazo[4,5-c]quinolin-2-one |
| 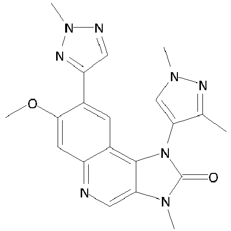 | 1-(1,3-Dimethyl-1 H-pyrazol-4-yl)-7-methoxy-3-methyl-8-(2-methyl-2 H-1,2, 3-triazol-4-yl)-1,3-dihydroimidazo[4,5-c]quinolin-2-one |
| 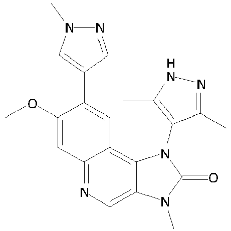 | 1-(3,5-Dimethyl-1 H-pyrazol-4-yl)-7-methoxy-3-methyl-8-(1-methyl-1 H-pyrazol-4-yl)-1 ,3-dihydroimidazo[4,5-c]quinolin-2-one |
| 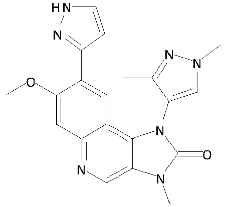 | 1-(1,3-Dimethyl-1 H-pyrazol-4-yl)-7-methoxy-3-methyl-8-(1 H-pyrazol-3-yl)-1,3-dihydro-imidazo[4,5-c]quinolin-2-one |
| 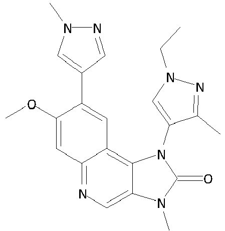 | 1-(1-Ethyl-3-methyl-1 H-pyrazol-4-yl)-7-methoxy-3-methyl-8-(1-methyl-1 H-pyrazol-4-yl)-1,3-dihydroimidazo[4,5-c]quinolin-2-one |
| 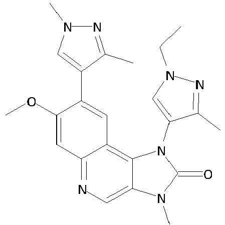 | 8-(1,3-Dimethyl-1 H-pyrazol-4-yl)-1-(1-ethyl-3-methyl-1 H-pyrazol-4-yl)-7-methoxy-3-methyl-1,3-dihydroimidazo[4,5-c]quinolin-2-one |
| 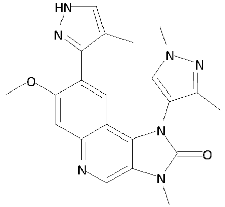 | 1-(1,3-Dimethyl-1 H-pyrazol-4-yl)-7-methoxy-3-methyl-8-(4-methyl-1 H-pyrazol-3-yl)-1,3-dihydroimidazo[4,5-c] quinolin-2-one |
| 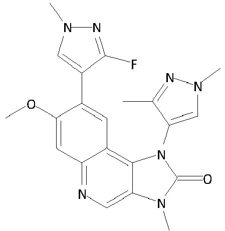 | 1-(1,3-Dimethyl-1 H-pyrazol-4-yl)-7-methoxy-3-methyl-8-(3-fluoro-1-methyl-1 H-pyrazol-4-yl)-1,3-dihydroimidazo[4,5-c]quinolin-2-one |
and/or pharmaceutically usable salt, solvate, tautomer, or stereoisomer thereof.

10. Compound according to any one of Claims 1 to 9 and/or pharmaceutically usable salt, solvate, tautomer, or stereoisomer thereof, for use as medicament.

11. Compound according to any one of Claims 1 to 9 and/or pharmaceutically usable, salt, solvate, tautomer, or stereoisomer thereof, for use in the treatment of cancer, tumours and/or metastases, optionally in combination with radiotherapy or at least one anticancer agent.

12. Compound according to one of Claims 1 to 9 and/or pharmaceutically usable salt, solvate, tautomer, or stereoisomer thereof, for use in the sensitisation of cancer cells to an anticancer agent and/or ionising radiation.

13. Pharmaceutical composition comprising an effective amount of at least one compound according to one of Claims 1 to 9 and/or pharmaceutically usable salt, solvate, tautomer, or stereoisomer thereof, optionally together with at least one pharmaceutically tolerated excipient.

14. Kit consisting of separate packs of
(a) an effective amount of a compound according to one of Claims 1 to 9 and/or pharmaceutically usable salt, solvate, tautomer, or stereoisomer thereof, and
(b) an effective amount of an anticancer agent.

## Revendications

1. Composé de formule (I) 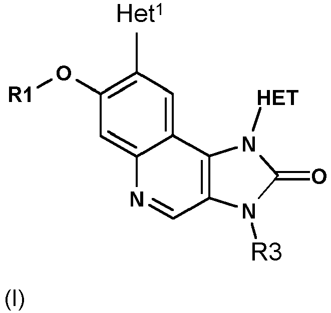 dans lequel
R1 est méthyle,
R3 désigne alkyle non ramifié ou ramifié ayant 1, 2 ou 3 atomes de C, où, indépendamment les uns des autres, 1, 2, 3, 4 ou 5 atomes de H peuvent être remplacés par Hal,
Het¹ est choisi parmi pyrazolyle, triazolyle et imidazolyle, où Het¹ est non substitué ou substitué par un ou deux substituants choisis, indépendamment les uns des autres, parmi alkyle, qui peut être non substitué ou mono- ou polysubstitué par halogène, -OY, -NYY, Hal et -Het²,
HET désigne un hétérocycle monocyclique aromatique de 5 chaînons, où cet hétérocycle est lié à l'atome de N du squelette via un atome de C annulaire, où cet hétérocycle est choisi parmi pyrazolyle, thiazolyle et imidazolyle, et peut être non substitué ou substitué par un, deux ou trois substituants choisis, indépendamment les uns des autres, dans le groupe constitué par: Hal, A, Het², -(CY₂)ₚ-OY, -(CY₂)ₚ-OZ, -(CY ₂)ₚ-O-Het², -(CY₂)ₚ-O-(CY₂)ₜ-Het², -(CY₂)ₚ-O-(CY₂)ₜ-NYY, -(CY₂)ₚ-O-(CY₂)ₜ-OY, -(CY₂)ₚ-NYY, -(CY₂)ₚ-COOY, -(CY₂)ₚ-CO-NYY et -(CY₂)ₚ-NY-COY,
A dans chaque cas indépendamment, désigne alkyle non ramifié ou ramifié ayant 1, 2, 3, 4, 5, 6, 7, 8, 9 ou 10 atomes de C, où, indépendamment les uns des autres, 1, 2, 3, 4, 5, 6 ou 7 atomes de H peuvent être remplacés par Hal,
Het² désigne un hétérocycle monocyclique saturé ayant 2, 3, 4, 5, 6 ou 7 atomes de C et 1, 2, 3 ou 4 atomes de N, O et/ou S, qui peut être non substitué ou monosubstitué par A,
Y désigne H ou A,
Z désigne alcényle non ramifié ou ramifié ayant 2, 3, 4, 5, 6, 7, 8, 9 ou 10 atomes de C, où, indépendamment les uns des autres, 1, 2, 3, 4, 5, 6 ou 7 atomes de H peuvent être remplacés par Hal,
Hal désigne F, Cl, Br ou I, et
p désigne 0, 1, 2, 3, 4, 5 ou 6,
t désigne 1, 2, 3, 4, 5 ou 6,
et/ou un sel, solvate, tautomère ou stéréoisomère pharmaceutiquement utilisable de celui-ci.

2. Composé de formule (I) selon la revendication 1, dans lequel HET peut être substitué par un, deux ou trois substituants choisis, indépendamment les uns des autres, dans le groupe constitué par : F, Cl, méthyle, éthyle, propyle, isopropyle, méthoxy, éthoxy, propoxy, pipérazinyle, tétrahydropyranyle, 2-méthoxyéthoxy, 2-hydroxyéthoxy, fluoro-méthoxy, difluorométhoxy, N-méthylcarbamoyle (-C(=O)-NH-CH₃), 2-méthylaminoéthoxy, 1-méthylazétidin-3-ylméthoxy, trideutériométhoxy, trifluorométhoxy, allyloxy et azétidinyloxy, et/ou un sel, solvate, tautomère ou stéréoisomère pharmaceutiquement utilisable de celui-ci.

3. Composé de formule (I) selon la revendication 1 ou 2, dans lequel HET est choisi parmi: 1H-pyrazol-4-yle, 1-éthyl-3-méthyl-1H-pyrazol-4-yle, 1,2-diméthyl-1H-pyrazol-4-yle, 1,3-diméthyl-1H-pyrazol-4-yle, 1-méthyl-1H-pyrazol-4-yle, 1-(tétrahydropyran-4-yl)-1H-pyrazol-4-yle, 2-méthyl-2H-pyrazol-3-yle, 3-méthyl-1H-pyrazol-4-yle, 2-méthyl-thiazol-4-yle, 3,5-diméthyl-1H-pyrazol-4-yle, 3-fluoro-1-méthyl-pyrazol-4-yle, thiazol-2-yle et 1-méthyl-1H-imidazolyle, et/ou un sel, solvate, tautomère ou stéréoisomère pharmaceutiquement utilisable de celui-ci.

4. Composé de formule (I) selon la revendication 1, 2 ou 3, dans lequel Het¹ est pyrazolyle, qui peut être non substitué ou substitué tel que défini selon la revendication 1, et/ou un sel, solvate, tautomère ou stéréoisomère pharmaceutiquement utilisable de celui-ci.

5. Composé de formule (I) selon la revendication 1, 2 ou 3, dans lequel Het¹ est triazolyle, de préférence 1,2,3-triazolyle, qui peut être non substitué ou substitué tel que défini selon la revendication 1 ou 2, et/ou un sel, solvate, tautomère ou stéréoisomère pharmaceutiquement utilisable de celui-ci.

6. Composé de formule (I) selon l'une des revendications 1 à 4, dans lequel Het¹ est non substitué ou substitué par un ou deux substituants choisis, indépendamment les uns des autres, parmi méthyle, éthyle, amino, méthoxy, fluorométhyle, difluoro-méthyle, fluor et azétidinyle, et/ou un sel, solvate, tautomère ou stéréoisomère pharmaceutiquement utilisable de celui-ci.

7. Composé de formule (I) selon l'une des revendications 1 à 4, dans lequel Het¹ est choisi parmi: 1H-pyrazol-4-yle, 2H-pyrazol-3-yle, 1H-pyrazol-3-yle, 1-méthyl-1H-pyrazol-4-yle, 3-méthyl-1H-pyrazol-4-yle, 5-méthyl-1H-pyrazol-3-yle, 4-méthyl-1H-pyrazol-3-yle, 1-fluorométhyl-1H-pyrazol-4-yle, 1-difluorométhyl-1H-pyrazol-4-yle, 1,3-diméthyl-1H-pyrazol-4-yle, 1-éthyl-1H-pyrazol-4-yle, 1-éthyl-3-méthyl-1H-pyrazolyle, 3-fluoro-1-méthyl-1H-pyrazol-4-yle, 3-amino-1H-pyrazol-5-yle, 2H-1,2,3-triazol-4-yle, 3H-1,2,3-triazol-4-yl-, 1-méthyl-1H-1,2,3-triazol-4-yle, 2-méthyl-2H-1,2,3-triazol-4-yle, 2-amino-1H-imidazol-4-yle et 1-(azétidin-3-yl)-3-méthyl-1H-pyrazol-4-yle, et/ou un sel, solvate, tautomère ou stéréoisomère pharmaceutiquement utilisable de celui-ci.

8. Composé de formule (I) selon l'une des revendications 1 à 7, dans lequel R3 désigne méthyle, et/ou un sel, solvate, tautomère ou stéréoisomère pharmaceutiquement utilisable de celui-ci.

9. Composé selon la revendication 1, choisi parmi:
| | |
|---|---|
| 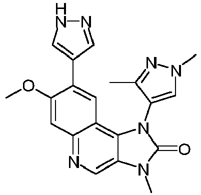 | 1-(1,3-Diméthyl-1 H-pyrazol-4-yl)-7-méthoxy-3-méthyl-8-(1 H-pyrazol-4-yl)-1,3-dihydro-imidazo[4,5-c]quinoléin-2-one |
| 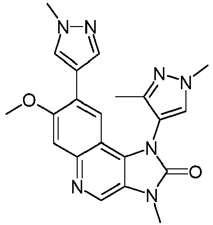 | 1-(1,3-Diméthyl-1 H-pyrazol-4-yl)-7-méthoxy-3-méthyl-8-(1-méthyl-1 H-pyrazol-4-yl)-1 ,3-dihydroimidazo[4,5-c]quinoléin-2-one |
| 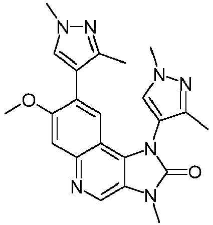 | 1 ,8-Bis(1 ,3-diméthyl-1 H-pyrazol-4-yl)-7-méthoxy-3-méthyl-1,3-di hyd roimidazo-[4,5-c]quinoléin-2-one |
| 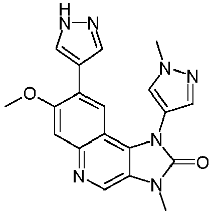 | 7-Méthoxy-3-méthyl-1-(1-méthyl-1 H-pyrazol-4-yl)-8-(1 H-pyrazol-4-yl)-1,3-dihydroimidazo[4,5-c]quinoléin-2-one |
| 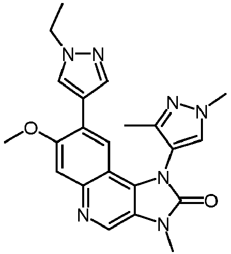 | 1-(1,3-Diméthyl-1 H-pyrazol-4-yl)-8-(1-éthyl-1 H-pyrazol-4-yl)-7-méthoxy-3-méthyl-1,3-dihydroimidazo[4,5-c]quinoléin-2-one |
| 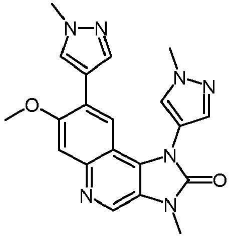 | 7 -Méthoxy-3-méthyl-1 , 8-bis(1-méthyl-1 H-pyrazol-4-yl)-1, 3-di hydroimidazo[4,5-c]-quinoléin-2-one |
| 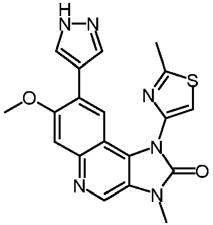 | 7-Méthoxy-3-méthyl-1-(2-méthyl-thiazol-4-yl)-8-(1 H-pyrazol-4-yl)-1 ,3-dihydroimidazo-[4,5-c]quinoléin-2-one |
| 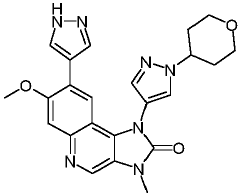 | 7-Méthoxy-3-méthyl-8-(1 H-pyrazol-4-yl)-1-[1-(tétrahydropyran-4-yl)-1 H-pyrazol-4-yl]-1,3-dihydroimidazo[4,5-c]quinoléin-2-one |
| 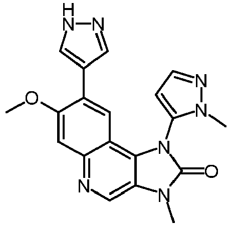 | 7-Méthoxy-3-méthyl-1-(2-méthyl-2H-pyrazol-3-yl)-8-(1 H-pyrazol-4-yl)-1,3-dihydroimidazo[4,5-c]quinoléin-2-one |
| 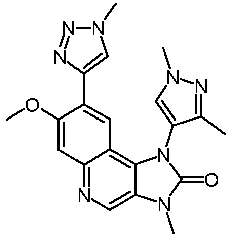 | 1-(1,3-Diméthyl-1 H-pyrazol-4-yl)-7-méthoxy-3-méthyl-8-(1-méthyl-1 H-1,2, 3-triazol-4-yl)-1,3-dihydroimidazo[4,5-c]quinoléin-2-one |
| 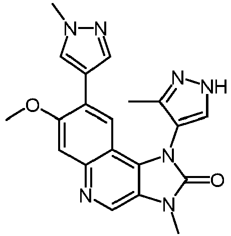 | 7-Méthoxy-3-méthyl-1-(3-méthyl-1 H-pyrazol-4-yl)-8-(1-méthyl-1 H-pyrazol-4-yl)-1,3-dihydroimidazo[4,5-c]quinoléin-2-one |
| 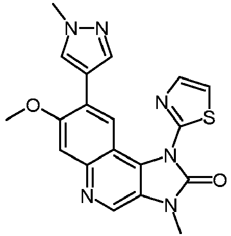 | 7-Méthoxy-3-méthyl-8-(1-méthyl-1 H-pyrazol-4-yl)-1-thiazol-2-yl-1,3-dihydro-imidazo[4,5-c]quinoléin-2-one |
| 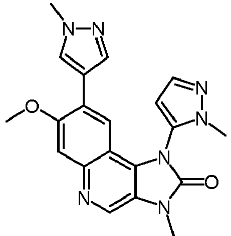 | 7-Méthoxy-3-méthyl-1-(2-méthyl-2H-pyrazol-3-yl)-8-(1-méthyl-1 H-pyrazol-4-yl)-1,3-dihydroimidazo[4,5-c]quinoléin-2-one |
| 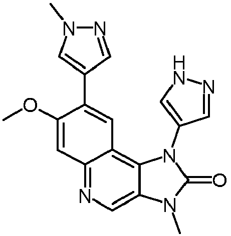 | 7-Méthoxy-3-méthyl-8-(1-méthyl-1 H-pyrazol-4-yl)-1-( 1 H-pyrazol-4-yl)-1 ,3-dihydroimidazo[4,5-c]quinoléin-2-one |
| 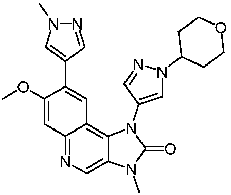 | 7-Méthoxy-3-méthyl-8-(1-méthyl-1 H-pyrazol-4-yl)-1-[1-(tétrahydropyran-4-yl)-1 H-pyrazol-4-yl]-1, 3-di hydroimidazo[4,5-c]-quinoléin-2-one |
| 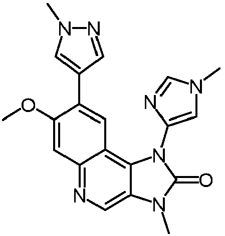 | 7-Méthoxy-3-méthyl-1-(1-méthyl-1 H-imidazol-4-yl)-8-(1-méthyl-1 H-pyrazol-4-yl)-1,3-dihydroimidazo[4,5-c]quinoléin-2-one |
| 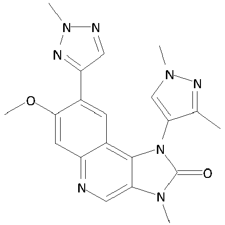 | 1-(1,3-Diméthyl-1 H-pyrazol-4-yl)-7-méthoxy-3-méthyl-8-(2-méthyl-2 H-1,2, 3-triazol-4-yl)-1,3-dihydroimidazo[4,5-c]quinoléin-2-one |
| 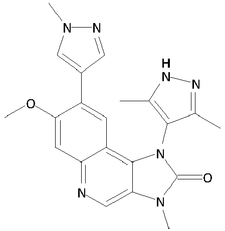 | 1-(3,5-Diméthyl-1 H-pyrazol-4-yl)-7-méthoxy-3-méthyl-8-(1-méthyl-1 H-pyrazol-4-yl)-1 ,3-dihydroimidazo[4,5-c]quinoléin-2-one |
| 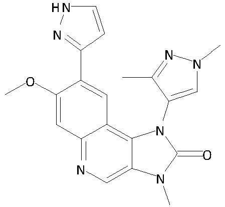 | 1-(1,3-Diméthyl-1 H-pyrazol-4-yl)-7-méthoxy-3-méthyl-8-(1 H-pyrazol-3-yl)-1,3-dihydro-imidazo[4,5-c]quinoléin-2-one |
| 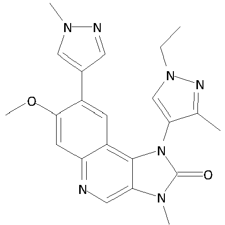 | 1-(1-Éthyl-3-méthyl-1 H-pyrazol-4-yl)-7-méthoxy-3-méthyl-8-(1-méthyl-1 H-pyrazol-4-yl)-1,3-dihydroimidazo[4,5-c]quinoléin-2-one |
| 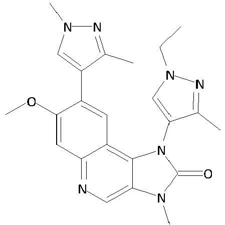 | 8-(1,3-Diméthyl-1 H-pyrazol-4-yl)-1-(1-éthyl-3-méthyl-1 H-pyrazol-4-yl)-7-méthoxy-3-méthyl-1,3-dihydroimidazo[4,5-c]quinoléin-2-one |
| 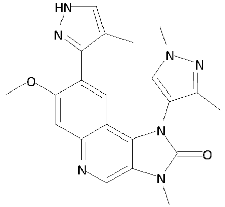 | 1-(1 ,3-Diméthyl-1 H-pyrazol-4-yl)-7-méthoxy-3-méthyl-8-(4-méthyl-1 H-pyrazol-3-yl)-1,3-dihydroimidazo[4,5-c]quinoléin-2-one |
| 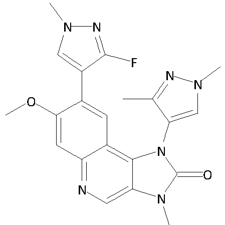 | 1-(1,3-Diméthyl-1 H-pyrazol-4-yl)-7-méthoxy-3-méthyl-8-(3-fluoro-1-méthyl-1 H-pyrazol-4-yl)-1,3-dihydroimidazo[4,5-c]quinoléin-2-one |
et/ou un sel, solvate, tautomère ou stéréoisomère pharmaceutiquement utilisable de celui-ci.

10. Composé selon l'une des revendications 1 à 9 et/ou un sel, solvate, tautomère ou stéréoisomère pharmaceutiquement utilisable de celui-ci, pour une utilisation comme médicament.

11. Composé selon l'une des revendications 1 à 9 et/ou un sel, solvate, tautomère ou stéréoisomère pharmaceutiquement utilisable de celui-ci, pour une utilisation dans le traitement du cancer, de tumeurs et/ou de métastases, éventuellement en combinaison avec une radiothérapie ou au moins un agent anticancéreux.

12. Composé selon l'une des revendications 1 à 9 et/ou un sel, solvate, tautomère ou stéréoisomère pharmaceutiquement utilisable de celui-ci, pour une utilisation dans la sensibilisation de cellules cancéreuses vis-à-vis d'un agent anticancéreux et/ou d'un rayonnement ionisant.

13. Composition pharmaceutique, comprenant une quantité efficace d'au moins un composé selon l'une des revendications 1 à 9 et/ou d'un sel, solvate, tautomère ou stéréoisomère pharmaceutiquement utilisable de celui-ci, éventuellement conjointement avec au moins un excipient pharmaceutiquement toléré.

14. Kit, constitué de conditionnements séparés
(a) d'une quantité efficace d'un composé selon l'une des revendications 1 à 9 et/ou d'un sel, solvate, tautomère ou stéréoisomère pharmaceutiquement utilisable de celui-ci, et
(b) d'une quantité efficace d'un agent anticancéreux.
